# EUROPÄISCHE PATENTSCHRIFT

(11) **EP 2 523 958 B1**
(45) Veröffentlichungstag und Bekanntmachung des Hinweises auf die Patenterteilung: **25.12.2013**
(21) Anmeldenummer: 11700098.4
(22) Anmeldetag: 12.01.2011
(51) Int. Cl.: C07D 498/04, A61K 31/519, A61P 3/10, A61P 7/02, A61P 9/00, A61P 11/00, A61P 13/12, A61P 25/28, A61P 29/00, A61P 31/02, A61P 35/00

(54) **CARBONSÄUREDERIVATE MIT EINEM 2,5,7-SUBSTITUIERTEN OXAZOLOPYRIMIDINRING**
CARBOXYLIC ACID DERIVATIVES HAVING A 2,5,7-SUBSTITUTED OXAZOLOPYRIMIDINE RING
DÉRIVÉS D'ACIDE CARBOXYLIQUE À COMPOSÉ CYCLIQUE OXAZOLOPYRIMIDINE SUBSTITUÉ EN 2,5,7

(30) Priorität: 13.01.2010 EP 10305036
(43) Veröffentlichungstag der Anmeldung: 21.11.2012
(73) Patentinhaber: SANOFI, 75008 Paris (FR)
(72) Erfinder: KADEREIT, Dieter, 65926 Frankfurt am Main (DE); SCHAEFER, Matthias, 65926 Frankfurt am Main (DE); HACHTEL, Stephanie, 65926 Frankfurt am Main (DE); DIETRICH, Axel, 65926 Frankfurt am Main (DE); HUEBSCHLE, Thomas, 65926 Frankfurt am Main (DE); GILLE, Andreas, 65926 Frankfurt am Main (DE); HISS, Katrin, 65926 Frankfurt am Main (DE)
(74) Vertreter: Then, Johann
(86) Internationale Anmeldenummer: PCT/EP2011/050298
(87) Internationale Veröffentlichungsnummer: WO 2011/086077

(56) Entgegenhaltungen:
- WO-A1-2005/000833
- WO-A1-2009/154775
- WO-A2-2007/061458
- WO-A2-2007/109334

## Beschreibung

Die vorliegende Erfindung betrifft Carbonsäurederivate mit einem 2,5,7-substituierten Oxazolopyrimidinring, sowie deren physiologisch akzeptable Salze.

Es sind bereits strukturähnliche Verbindungen im Stand der Technik beschrieben (siehe WO2009/154775), die zur Behandlung multipler Sklerose geeignet sind. Die Wirkungsweise dieser Verbindungen besteht darin, durch Aktivierung des EDG-1 Rezeptors eine Desensitisierung des EDG-1 Signalweges zu verursachen (sog. Superagonismus), der dann einem funktionellen Antagonismus des EDG-1-Signalweges gleichkommt. Systemisch bedeutet das, daß vor allem auf Lymphozyten der EDG-1 Signalweg dauerhaft unterdrückt wird, wodurch diese Zellen nicht mehr chemotaktisch dem S1 P Gradienten zwischen Blut und Lymphflüssigkeit folgen können. Dies bedingt, dass die betroffenen Lymphozyten nicht mehr das sekundäre lymphatische Gewebe verlassen können (verstärktes Homeing) und die Zahl der frei zirkulierenden Lymphozyten im Plasma stark gesenkt wird. Dieser Mangel an Lymphozyten im Plasma (Lymphopenie) bewirkt eine Immunsuppression, die zwingend für den Wirkmechanismus der in WO 2009/154775 beschriebenen EDG-1-Rezeptor-Modulatoren erforderlich ist.

WO 2005/ 000833 offenbart immunsuppressive Verbindungen, deren Wirkung in Verbindung mit dem EDG-Signalweg steht.

Der vorliegenden Erfindung lag die Aufgabe zugrunde, Verbindungen zur Verfügung zustellen, die spezifisch zur Wundheilung und insbesondere zur Behandlung von Wundheilungsstörungen von Diabetes Patienten geeignet sind. Weiter war es wünschenswert Verbindungen zur Verfügung zustellen, die zur Behandlung des Diabetische Fußsyndroms (DFS) geeignet sind.

Weiter war es wünschenswert eine wiederholbare Aktivierung des EDG-1-Rezeptor Signalweges zu erreichen, was damit pharmakologisch eine persistente Aktivierung des EDG-1 Signalweges ermöglicht.

Die vorliegende Erfindung betrifft Oxazolopyrimidinverbindungen der Formel I. 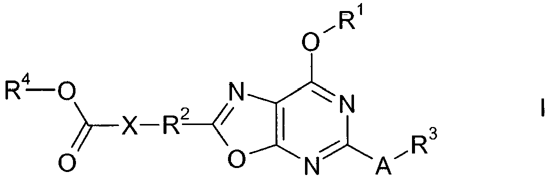 worin A, R¹, R², R³, R⁴ und X wie nachstehend definiert sind. Der Wirkmechanismus der Verbindungen der Formel I beruht also nicht auf Desensitisierung des EDG-1-Signalweges und steht somit dem in WO 2009/154775 beschriebenen Wirkmechanismus diametral gegenüber. Die Erfindung betrifft ferner Verfahren zur Herstellung von Verbindungen der Formel I, ihre Verwendung, insbesondere als Wirkstoff in Pharmazeutika, und pharmazeutische Zusammensetzungen, die sie enthalten.

Patienten mit Diabetes haben gegenüber gesunden Menschen eine verzögerte Wundheilung und eine erhöhte Infektionsrate, vor allem bei länger währender Hyperglykämie, zum Beispiel hervorgerufen durch eine schlechte Blutzuckereinstellung. Zu den Ursachen gehören Durchblutungsstörungen, vor allem im Bereich der kleinen Gefäße, die zu einer verschlechterten Sauerstoff- und Nährstoffversorgung des Gewebes führen. Außerdem besteht eine verminderte Zellteilungs- und Zellmigrationsrate von Keratinozyten, Fibroblasten und dermalen Endothelzellen. Zusätzlich ist die Aktivität verschiedener Abwehrzellen (Granulozyten) mit reduzierter Phagozytose (Aufnahme und Zerstörung von Bakterien) eingeschränkt. Auch die Funktion der Antikörper (Immunglobuline) gegen Bakterien ist bei hohen Blutzuckerwerten eingeschränkt. Dementsprechend müssen Wunden und Infektionen bei Diabetes-Patienten besonders versorgt werden.

Der Edg-1-Rezeptor gehört zur Familie der Edg-Rezeptoren (Edg = Endothelial Differentiation Gene) von gegenwärtig acht identifizierten GPCRs (G-Proteingekoppelten Rezeptoren) der Klasse A. Diese Familie kann in Unterfamilien von durch Sphingosin-1-Phosphat (S1P) aktivierten Rezeptoren (fünf Mitglieder) und durch Lysophosphatidsäure (LPA) aktivierte Rezeptoren (drei Mitglieder) unterteilt werden. Der endogene Ligand S1P ist ein pluripotentes Lysophospholipid, das durch Aktivierung von GPCRs aus der Edg-Rezeptorfamilie, nämlich Edg-1 (= S1P1), Edg-3 (= S1 P3), Edg-5 (= S1 P2), Edg-6 (= S1 P4) und Edg-8 (S1 P5), auf verschiedene Zelltypen wirkt. Wenngleich S1P auch als intrazellulärer Botenstoff beschrieben wird, werden zahlreiche zelluläre Antworten von S1P über die Aktivierung von Edg-Rezeptoren - vermittelt werden. S1P wird durch die Enzymfamilie der Sphingosinkinasen (SPHK) erzeugt und durch verschiedene Phosphatasen oder Lyasen abgebaut.

Gegenstand der vorliegenden Erfindung ist eine Oxazolopyrimidinverbindung der Formel I in einer beliebigen ihrer stereoisomeren Formen oder einer Mischung von stereoisomeren Formen in beliebigem Verhältnis oder ein physiologisch akzeptables Salz davon oder ein physiologisch akzeptables Solvat einer derartigen Verbindung oder eines derartigen Salzes, 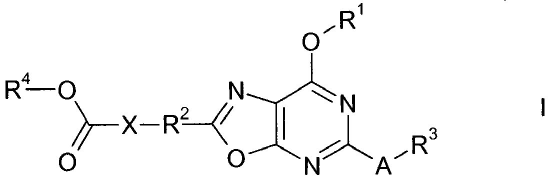 worin
A ausgewählt ist aus NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl und (C₁-C₆)-Alkandiyloxy, die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxygruppe an die Gruppe R² gebunden ist;
R¹ ausgewählt ist aus (C₁-C₆)-Alkyl, (C₂-C₆)-Alkenyl, (C₂-C₆)-Alkinyl, (C₃-C₇)-Cycloalkyl-CₜH₂ₜ- und Het-CₜH₂ₜ-, worin t aus 0, 1, 2 und 3 ausgewählt ist;
R² ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, 0 und S ausgewählt sind, wobei eines der Ringstickstoffatome ein Wasserstoffatom oder einen Substituenten R²¹ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₂-C₆)-Alkenyl, (C₂-C₆)-Alkinyl, (C₃-C₇)-Cyclo-alkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 1 0-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
R⁴ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R²¹ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl;
R³¹ ausgewählt ist aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die unter N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist, wobei alle Zahlen m voneinander unabhängig sind;
wobei alle Cycloalkyl- und Cycloalkandiylgruppen unabhängig voneinander und unabhängig von anderen Substituenten gegebenenfalls durch einen oder mehrere gleiche oder verschiedene Substituenten substituiert sind, die unter Fluor und (C₁-C₄)-Alkyl ausgewählt sind;
wobei alle Alkyl-, Alkandiyl-, CtH2t-, CᵤH₂ᵤ-, CᵥH₂ᵥ-, C_{w}H_{2w}-, C_{z}H_{2z}-, Alkenyl-, Alkendiyl-, Alkinyl- und Alkindiylgruppen unabhängig voneinander und unabhängig von anderen Substituenten gegebenenfalls durch einen oder mehrere Fluorsubstituenten substituiert sind.

Strukturelemente wie Gruppen, Substituenten, Heteroringglieder, Zahlen oder andere Merkmale, beispielsweise Alkylgruppen, Gruppen wie R²² oder R³¹, Zahlen wie m, u und v, die in den Verbindungen der Formel I mehrmals vorkommen können, können alle unabhängig voneinander eine beliebige der angegebenen Bedeutungen besitzen und in jedem Fall gleich oder voneinander verschieden sein. Beispielsweise können die Alkylgruppen in einer Dialkylaminogruppe gleich oder verschieden sein.

Alkyl-, Alkenyl- und Alkinylgruppen können linear, d.h. geradkettig, oder verzweigt sein. Dies gilt auch, wenn sie Teil anderer Gruppen, beispielsweise Alkyloxygruppen (= Alkoxygruppen, Alkyl-O-Gruppen), Alkyloxycarbonylgruppen oder alkylsubstituierter Aminogruppen, sind oder wenn sie substituiert sind. Je nach der jeweiligen Definition kann die Zahl der Kohlenstoffatome in einer Alkylgruppe 1, 2, 3, 4, 5 oder 6 oder 1, 2, 3 oder 4 oder 1, 2 oder 3 betragen. Beispiele für Alkyl sind Methyl, Ethyl, Propyl einschließlich n-Propyl und Isopropyl, Butyl, einschließlich n-Butyl, sek.-Butyl, Isobutyl und tert.-Butyl, Pentyl einschließlich n-Pentyl, 1-Methylbutyl, Isopentyl, Neopentyl und tert.-Pentyl und Hexyl einschließlich n-Hexyl, 3,3-Dimethylbutyl und Isohexyl. Doppelbindungen und Dreifachbindungen in Alkenylgruppen und Alkinylgruppen können in beliebigen Positionen vorliegen. In einer Ausführungsform der Erfindung enthalten Alkenylgruppen eine Doppelbindung und Alkinylgruppen eine Dreifachbindung. In einer Ausführungsform der Erfindung enthält eine Alkenylgruppe oder Alkinylgruppe mindestens drei Kohlenstoffatome und ist über ein Kohlenstoffatom, das nicht Teil einer Doppelbindung oder Dreifachbindung ist, an den Rest des Moleküls gebunden. Beispiele für Alkenyl und Alkinyl sind Ethenyl, Prop-1-enyl, Prop-2-enyl (= Allyl), But-2-enyl, 2-Methylprop-2-enyl, 3-Methylbut-2-enyl, Hex-3-enyl, Hex-4-enyl, Prop-2-inyl (= Propargyl), But-2-inyl, But-3-inyl, Hex-4-inyl oder Hex-5-inyl. Substituierte Alkylgruppen, Alkenylgruppen und Alkinylgruppen können in beliebigen Positionen substituiert sein, mit der Maßgabe, daß die jeweilige Verbindung ausreichend stabil und für den gewünschten Zweck wie die Verwendung als Arzneistoff geeignet ist. Die Voraussetzung, daß eine spezifische Gruppe und eine Verbindung der Formel I ausreichend stabil und für den gewünschten Zweck wie die Verwendung als Arzneistoff geeignet sind, gilt allgemein in bezug auf die Definitionen aller Gruppen in den Verbindungen der Formel I.

Soweit anwendbar, gelten die vorstehenden Erklärungen bezüglich Alkyl-, Alkenyl- und Alkinylgruppen entsprechend für zweiwertige Alkylgruppen wie die Gruppen Alkandiyl, CₜH₂ₜ, CᵤH₂ᵤ, CᵥH₂ᵥ, C_{w}H_{2w} und C_{z}H_{2z} und zweiwertige Alkenylgruppen und Alkinylgruppen, wie die Gruppen Alkendiyl und Alkindiyl, die somit ebenfalls linear und verzweigt sein können. Die Doppelbindungen und Dreifachbindungen in Alkendiyl- und Alkindiylgruppen können in beliebigen Positionen vorliegen. In einer Ausführungsform der Erfindung enthalten Alkendiylgruppen eine Doppelbindung und Alkindiylgruppen eine Dreifachbindung. Beispiele für zweiwertige Alkylgruppen sind -CH₂-(= Methylen), -CH₂-CH₂-, -CH₂-CH₂-CH₂-, -CH₂-CH₂-CH₂-CH₂-, -CH(CH₃)-, -C(CH₃)₂-, -CH(CH₃)-CH₂-, -CH₂-CH(CH₃)-, -C(CH₃)₂-CH₂-, -CH₂-C(CH₃)₂-, Beispiele für zweiwertige Alkenylgruppen sind -CH=CH-, -CH₂-CH=CH-, -CH=CH-CH₂-, -CH₂-CH=CH-CH₂-, -CH₂-CH₂-CH=CH-, -C(CH₃)=C(CH₃)-, und Beispiele für zweiwertige Alkinylgruppen sind -C≡C-, -CH₂-C≡C-, -C≡C-CH₂-, -C(CH₃)₂-C≡C-, -C≡C-C(CH₃)₂-, -CH₂-C≡C-CH₂-, -CH₂-CH₂-C≡C-. Wenn eine Zahl in einer zweiwertigen Gruppe, wie beispielsweise die Zahl t in der Gruppe CₜH₂ₜ, für 0 (= null) steht, sind die beiden Gruppen, die an die in Rede stehende Gruppe, wie CₜH₂ₜ, gebunden sind, über eine Einfachbindung direkt miteinander verbunden.

Die Zahl der Ringkohlenstoffatome in einer Cycloalkylgruppe kann 3, 4, 5, 6 oder 7 betragen. In einer Ausführungsform der Erfindung beträgt die Zahl der Ringkohlenstoffatome in einer Cycloalkylgruppe unabhängig von der Zahl der Ringkohlenstoffatome in einer anderen Cycloalkylgruppe 3, 4, 5 oder 6, in einer anderen Ausführungsform 3, 4 oder 5, in einer anderen Ausführungsform 3 oder 4, in einer anderen Ausführungsform 3, in einer anderen Ausführungsform 5, 6 oder 7, in einer anderen Ausführungsform 5 oder 6, in einer anderen Ausführungsform 6 oder 7, in einer anderen Ausführungsform 6. Dies gilt entsprechend für zweiwertige Cycloalkylgruppen, d.h. Cycloalkandiylgruppen, die über ein oder zwei beliebige Ringkohlenstoffatome an die benachbarten Gruppen gebunden sein können. Beispiele für Cycloalkylgruppen sind Cyclopropyl, Cyclobutyl, Cyclopentyl, Cyclohexyl und Cycloheptyl. Beispiele für zweiwertige Cycloalkylgruppen sind Cyclopropan-1,1-diyl, Cyclopropan-1,2-diyl, Cyclobutan-1,3-diyl, Cyclopentan-1,1-diyl, Cyclopentan-1,2-diyl, Cyclopentan-1,3-diyl, Cyclohexan-1,1-diyl, Cyclohexan-1,2-diyl, Cyclohexan-1,3-diyl, Cyclohexan-1,4-diyl, Cycloheptan-1,4-diyl. Unabhängig voneinander und unabhängig von anderen Substituenten sind Cycloalkylgruppen und Cycloalkandiylgruppen gegebenenfalls durch einen oder mehrere gleiche oder verschiedene (C₁-C₄)-Alkylsubstituenten substituiert, die in beliebigen Positionen stehen können, d.h. Cycloalkylgruppen können durch Alkylsubstituenten unsubstituiert oder durch Alkylsubstituenten, beispielsweise 1, 2, 3 oder 4 oder 1 oder 2 (C₁-C₄)-Alkylsubstituenten, beispielsweise Methylgruppen, substituiert sein. Beispiele für alkylsubstituierte Cycloalkylgruppen und Cycloalkandiylgruppen sind 4-Methylcydohexyl, 4-tert.-Butylcyclohexyl oder 2,3-Dimethylcyclopentyl, 2,2-Dimethylcyclopropan-1,1-diyl, 2,2-Dimethylcyclopropan-1,2-diyl, 2,2-Dimethylcyclopentan-1,3-diyl, 6,6-Dimethylcycloheptan-1,4-diyl. Beispiele für Cycloalkylalkylgruppen, die beispielsweise Gruppen wie (C₃-C₇)-Cycloalkyl-CₜH₂ₜ-repräsentieren können, sind Cyclopropylmethyl, Cyclobutylmethyl, Cyclopentylmethyl, Cyclohexylmethyl, Cycloheptylmethyl, 1-Cyclopropylethyl, 2-Cyclopropylethyl, 1-Cyclobutylethyl, 2-Cyclobutylethyl, 2-Cyclopentylethyl, 2-Cyclohexylethyl, 2-Cycloheptylethyl.

Unabhängig voneinander und unabhängig von anderen Substituenten sind Alkylgruppen, zweiwertige Alkylgruppen, Alkenylgruppen, zweiwertige Alkenylgruppen, Alkinylgruppen, zweiwertige Alkinylgruppen, Cycloalkylgruppen und zweiwertige Cycloalkylgruppen gegebenenfalls durch einen oder mehrere Fluorsubstituenten substituiert, die in beliebigen Positionen stehen können, d.h. diese Gruppen können durch Fluorsubstituenten unsubstituiert oder durch Fluorsubstituenten, beispielsweise 1, 2, 3, 4, 5, 6, 7, 8, 9, 10, 11, 12 oder 13 oder 1, 2, 3, 4, 5, 6, 7, 8 oder 9 oder 1, 2, 3, 4, 5, 6 oder 7 oder 1, 2, 3, 4 oder 5 oder 1, 2 oder 3 oder 1 oder 2 Fluorsubstituenten, substituiert sein. Beispiele für fluorsubstituierte derartige Gruppen sind Trifluormethyl, 2-Fluorethyl, 2,2,2-Trifluorethyl, Pentafluorethyl, 3,3,3-Trifluorpropyl, 2,2,3,3,3-Pentafluorpropyl, 4,4,4-Trifluorbutyl, Heptafluorisopropyl, -CHF-, -CF₂-, -CF₂-CH₂-, -CH₂-CF₂-, -CF₂-CF₂-, -CF(CH₃)-, -C(CF₃)₂-, 1-Fluorcyclopropyl, 2,2-Difluorcyclopropyl, 3,3-Difluorcyclobutyl, 1-Fluorcyclohexyl, 4,4-Difluorcydohexyl, 3,3,4,4,5,5-Hexafluorcyclohexyl, 2,2-Difluorcyclopropan-1,2-diyl. Beispiele für Alkyloxygruppen, in denen die Alkylgruppierung fluorsubstituiert ist, sind Trifluormethoxy, 2,2,2-Trifluorethoxy, Pentafluorethoxy und 3,3,3-Trifluorpropoxy. In einer Ausführungsform der Erfindung beträgt die Gesamtzahl der Fluorsubstituenten und (C₁-C₄)-Alkylsubstituenten, die unabhängig von anderen Substituenten gegebenenfalls an Cycloalkylgruppen und Cycloalkandiylgruppen in den Verbindungen der Formel I vorliegen, 1, 2, 3, 4, 5, 6, 7, 8, 9, 10 oder 11, in einer anderen Ausführungsform 1, 2, 3, 4, 5, 6, 7, 8 oder 9, in einer anderen Ausführungsform 1, 2, 3, 4 oder 5, in einer anderen Ausführungsform 1, 2, 3 oder 4.

Gruppen wie Phenyl, Naphthyl (= Naphthalinyl) und Reste von aromatischen Heterocyclen, die gegebenenfalls durch einen oder mehrere Substituenten substituiert sind, können unsubstituiert oder substituiert sein, beispielsweise durch 1, 2, 3, 4 oder 5 oder 1, 2, 3 oder 4 oder 1, 2 oder 3 oder 1 oder 2 oder 1 gleiche oder verschiedene Substituenten, die in beliebigen Positionen stehen können. In einer Ausführungsform der Erfindung ist die Gesamtzahl der Nitrosubstituenten in einer Verbindung der Formel I nicht größer als zwei. Aromatische Stickstoffheterocyclen, die in dem zugrundeliegenden Ringsystem ein Wasserstoffatom an einem Ringstickstoffatom in einem 5-gliedrigen Ring, wie beispielsweise einem Pyrrol-, Imidazol-, Indol- oder Benzoimidazolring, tragen, können an den Kohlenstoffatomen und/oder an derartigen Ringstickstoffatomen substituiert sein. In einer Ausführungsform der Erfindung sind Substituenten an derartigen Ringstickstoffatomen aus (C₁-C₄)-Alkylgruppen ausgewählt, d.h. derartige Ringstickstoffatome in aromatischen Heterocyclen tragen ein Wasserstoffatom oder einen (C₁-C₄)-Alkylsubstituenten. Wenn bezüglich Ringstickstoffatomen in aromatischen Heterocyclen und anderen Heterocyclen angegeben ist, daß sie ein Wasserstoffatom oder einen Substituenten tragen können, so tragen derartige Ringstickstoffatome entweder ein Wasserstoffatom oder einen Substituenten oder nicht. Ringstickstoffatome, die ein Wasserstoffatom oder einen Substituenten tragen, kommen in einem stickstoffhaltigen aromatischen 5-gliedrigen Ring, wie er beispielsweise in Pyrrol, Imidazol, Indol oder Benzoimidazol vorliegt, und in einem nichtaromatischen Ring einschließlich eines gesättigten Rings vor. Ringstickstoffatome, die kein Wasserstoffatom oder keinen Substituenten tragen, sofern sie nicht in positiv geladener Form vorliegen, einschließlich weiterer Ringstickstoffatome neben den Ringstickstoffatomen, die ein Wasserstoffatom oder einen Substituenten tragen, kommen in einem aromatischen Ring, wie er beispielsweise in Thiazol, Imidazol, Pyridin oder Benzoimidazol vorliegt, und in einem nichtaromatischen Ring, in dem sie Brückenkopfatome oder Teil einer Doppelbindung sind, und als Ringstickstoffatome, über die ein Ring gebunden ist, vor. Geeignete Ringstickstoffatome in aromatischen Heterocyclen in den Verbindungen der Formel I, wie das Ringstickstoffatom in einem Pyridinring, spezifisch ein Ringstickstoffatom in einem aromatischen Heterocyclus, der R² repräsentiert, können auch einen Oxysubstituenten -O⁻ tragen und als N-Oxid vorliegen, und derartige Ringstickstoffatome können auch als quartäres Salz, beispielsweise als N-(C₁-C₄)-Alkylsalz wie N-Methylsalz, vorliegen, wobei in einer Ausführungsform der Erfindung das Gegenanion in einem derartigen quartären Salz ein physiologisch akzeptables Anion ist, das sich von einer Säure, die ein physiologisch akzeptables Salz bildet, ableitet. In monosubstituierten Phenylgruppen kann der Substituent in der 2-Position, der 3-Position oder der 4-Position stehen. In disubstituierten Phenylgruppen können die Substituenten in 2,3-Position, 2,4-Position, 2,5-Position, 2,6-Position, 3,4-Position oder 3,5-Position stehen. In trisubstituierten Phenylgruppen können die Substituenten in 2,3,4-Position, 2,3,5-Position, 2,3,6-Position, 2,4,5-Position, 2,4,6-Position oder 3,4,5-Position stehen. Naphthyl kann 1-Naphthyl (= Naphthalin-1-yl) oder 2-Naphthyl (= Naphthalin-2-yl) sein. In monosubstituierten 1-Naphthylgruppen kann der Substituent in der 2-, 3-, 4-, 5-, 6-, 7- oder 8-Position stehen. In monosubstituierten 2-Naphthylgruppen kann der Substituent in der 1-, 3-, 4-, 5-, 6-, 7- oder 8-Position stehen. In disubstituierten Naphthylgruppen können die Substituenten ebenfalls in beliebigen Positionen sowohl in dem Ring, über den die Naphthylgruppe gebunden ist, und/oder in dem anderen Ring stehen. Diese Aussage bezüglich der einwertigen Reste gilt entsprechend für die jeweiligen zweiwertigen Reste, wie beispielsweise Phenylengruppen, die R² repräsentieren, die somit ebenfalls unsubstituiert oder substituiert sein können, beispielsweise durch 1, 2, 3 oder 4 oder durch 1, 2 oder 3 oder durch 1 oder 2 oder durch 1 gleiche oder verschiedene Substituenten, die in beliebigen Positionen stehen können.

In R² oder R³ repräsentierenden Resten von aromatischen Heterocyclen, die als Heteroaryl- und Heteroarylengruppen bezeichnet sein können, sowie in allen anderen heterocyclischen Ringen in den Verbindungen der Formel I einschließlich der Gruppe Het und der nichtaromatischen heterocyclischen Gruppen, die R³ repräsentieren, sind die Ringheteroatome im allgemeinen aus N, O und S ausgewählt, wobei N Ringstickstoffatome, die ein Wasserstoffatom oder einen Substituenten tragen, sowie Ringstickstoffatome, die kein Wasserstoffatom und keinen Substituenten tragen, einschließt. Ringheteroatome können in beliebigen Positionen stehen, vorausgesetzt, daß das heterocyclische System in der Technik bekannt und stabil und als Untergruppe für den gewünschten Zweck der Verbindung der Formel I wie die Verwendung als Arzneistoff geeignet ist. In einer Ausführungsform der Erfindung können zwei Ringsauerstoffatome nicht in benachbarten Ringpositionen eines Heterocyclus stehen, in einer anderen Ausführungsform können zwei Ringheteroatome, die aus Sauerstoff und Schwefel ausgewählt sind, nicht in benachbarten Ringpositionen eines beliebigen Heterocyclus stehen. Gesättigte Ringe enthalten keine Doppelbindung im Ring. Ungesättigte Ringsysteme können aromatisch oder teilweise ungesättigt einschließlich teilweise aromatisch sein, wobei in letzterem Fall ein Ring in einem bicyclischen Ringsystem aromatisch ist und das Ringsystem über ein Atom im nichtaromatischen Ring gebunden ist. Je nach der jeweiligen Gruppe können ungesättigte Ringe eine, zwei, drei, vier oder fünf Doppelbindungen im Ring enthalten. Aromatische Gruppen enthalten ein cyclisches System von sechs oder zehn delokalisierten pi-Elektronen im Ring. Je nach der jeweiligen Gruppe können gesättigte und nichtaromatisch ungesättigte heterocyclische Ringe einschließlich Het und nichtaromatischen Gruppen, die R³ repräsentieren, 3-gliedrig, 4-gliedrig, 5-gliedrig, 6-gliedrig, 7-gliedrig, 8-gliedrig, 9-gliedrig oder 10-gliedrig sein. In einer Ausführungsform der Erfindung sind aromatische heterocyclische Ringe 5-gliedrige oder 6-gliedrige monocyclische Ringe oder 8-gliedrige, 9-gliedrige oder 10-gliedrige bicyclische Ringe, in einer anderen Ausführungsform 5-gliedrige oder 6-gliedrige monocyclische Ringe oder 9-gliedrige oder 10-gliedrige bicyclische Ringe, in einer anderen Ausführungsform 5-gliedrige oder 6-gliedrige monocyclische Ringe, wobei die 8-gliedrigen, 9-gliedrigen oder 10-gliedrigen bicyclischen Ringe aus zwei anellierten 5-gliedrigen Ringen, einem 5-gliedrigen Ring und einem 6-gliedrigen Ring, die miteinander anelliert sind, bzw. zwei anellierten 6-gliedrigen Ringen zusammengesetzt sind. In bicyclischen aromatischen heterocyclischen Gruppen können ein oder beide Ringe Heteroringglieder enthalten, und ein oder beide Ringe können aromatisch sein. Im allgemeinen werden bicyclische Ringsysteme mit einem aromatischen Ring und einem nichtaromatischen Ring als aromatisch erachtet, wenn sie über ein Kohlenstoffatom im aromatischen Ring gebunden sind, und als nichtaromatisch, wenn sie über ein Kohlenstoffatom im nichtaromatischen Ring gebunden sind. Sofern nicht anders angegeben, können heterocyclische Gruppen einschließlich aromatischer heterocyclischer Gruppen über ein beliebiges geeignetes Ringkohlenstoffatom und im Fall von Stickstoffheterocyclen über ein beliebiges geeignetes Ringstickstoffatom gebunden sein. In einer Ausführungsform der Erfindung ist eine aromatische heterocyclische Gruppe in einer Verbindung der Formel I unabhängig von jeder anderen aromatischen heterocyclischen Gruppe über ein Ringkohlenstoffatom gebunden, in einer anderen Ausführungsformen über ein Ringstickstoffatom. Je nach der Definition der jeweiligen heterocyclischen Gruppe beträgt in einer Ausführungsform der Erfindung die Zahl der Ringheteroatome, die in einer heterocyclischen Gruppe unabhängig von der Zahl von Ringheteroatomen in einer anderen heterocyclischen Gruppe vorliegen kann, 1, 2, 3 oder 4, in einer anderen Ausführungsform 1,2 oder 3, in einer anderen Ausführungsform 1 oder 2, in einer anderen Ausführungsform 1, wobei die Ringheteroatome gleich oder verschieden sein können. Heterocyclische Gruppen, die gegebenenfalls substituiert sind, können unabhängig von jeder anderen heterocyclischen Gruppe unsubstituiert oder durch einen oder mehrere gleiche oder verschiedene Substituenten, beispielsweise 1, 2, 3, 4 oder 5 oder 1, 2, 3 oder 4 oder 1, 2 oder 3 oder 1 oder 2 oder 1 Substituenten, die bei der Definition der jeweiligen Gruppe angegeben sind, substituiert sein. Substituenten an heterocyclischen Gruppen können in beliebigen Positionen stehen. So können Substituenten in einer Pyridin-2-ylgruppe beispielsweise in der 3-Position und/oder 4-Position und/oder 5-Position und/oder 6-Position stehen, in einer Pyridin-3-ylgruppe in der 2-Position und/oder 4-Position und/oder 5-Postion und/oder 6-Position stehen und in einer Pyridin-4-ylgruppe in der 2-Position und/oder 3-Position und/oder 5-Position und/oder 6-Position stehen.

Beispiele für Grundkörper von Heterocyclen, von denen sich heterocyclische Gruppen einschließlich aromatischer heterocyclischer Gruppen, gesättigter heterocyclischer Gruppen und nichtaromatischer ungesättigter heterocyclischer Gruppen ableiten können, sind Azet, Oxet, Pyrrol, Furan, Thiophen, Imidazol, Pyrazol, [1,3]Dioxol, Oxazol (= [1,3]Oxazol), Isoxazol (= [1,2]Oxazol), Thiazol (= [1,3]Thiazol), Isothiazol (= [1,2]Thiazol), [1,2,3]Triazol, [1,2,4]Triazol, [1,2,4]Oxadiazol, [1,3,4]Oxadiazol, [1,2,4]Thiadiazol, [1,3,4]Thiadiazol, Tetrazol, Pyridin, Pyran, Thiopyran, Pyridazin, Pyrimidin, Pyrazin, [1,3]Oxazin, [1,4]Oxazin, [1,3]Thiazin, [1,4]Thiazin, [1,2,3]Triazin, [1,3]dithian, [1,4]dithian, [1,2,4]Triazin, [1,3,5]Triazin, [1,2,4,5]Tetrazin, Azepin, [1,3]Diazepin, [1,4]Diazepin, [1,3]Oxazepin, [1,4]Oxazepin, [1,3]Thiazepin, [1,4]Thiazepin, Azocin, Azecin, Cyclopenta[b]pyrrol, 2-Azabicyclo[3.1.0]hexan, 3-Azabicyclo[3.1.0]hexan, 2-Oxa-5-azabicyclo[2.2.1]heptan, Indol, Isoindol, Benzothiophen, Benzofuran, [1,3]Benzodioxol (= 1,2-Methylendioxybenzol), [1,3]Benzoxazol, [1,3]Benzothiazol, Benzoimidazol, Thieno[3,2-c]pyridin, Chromen, lsochromen, [1,4]Benzodioxin, [1,4]Benzoxazin, [1,4]Benzothiazin, Chinolin, Isochinolin, Cinnolin, Chinazolin, Chinoxalin, Phthalazin, Thienothiophen, [1,8]Naphthyridin und andere Naphtyridine, Pteridin und die jeweiligen gesättigten und teilweise ungesättigten Heterocyclen, in denen eine oder mehrere, beispielsweise eine, zwei, drei, vier oder alle Doppelbindungen im Ringsystem einschließlich der Doppelbindungen im aromatischen Ring durch Einfachbindungen ersetzt sind, wie beispielsweise Azetidin, Oxetan, Pyrrolidin, Tetrahydrofuran, Tetrahydrothiophen, Imidazolidin, Oxazolidin, Thiazolidin, Dihydropyridin, Piperidin, Tetrahydropyran, Piperazin, Morpholin, Thiomorpholin, Azepan, Chroman, Isochroman, [1,4]Benzodioxan (= 1,2-Ethylendioxybenzol), 2,3-Dihydrobenzofuran, 1,2,3,4-Tetrahydrochinolin, 1,2,3,4-Tetrahydroisochinolin.

Beispiele für Reste von aromatischen Heterocyclen, die in den Verbindungen der Formel I vorkommen können, sind Thiophenyl (= Thienyl) einschließlich Thiophen-2-yl und Thiophen-3-yl, Pyridinyl (= Pyridyl) einschließlich Pyridin-2-yl (= 2-Pyridyl), Pyridin-3-yl (= 3-Pyridyl) und Pyridin-4-yl (= 4-Pyridyl), Imidazolyl einschließlich beispielsweise 1H-Imidazol-1-yl, 1 H-Imidazol-2-yl, 1H-Imidazol-4-yl und 1H-Imidazol-5-yl, [1,2,4]Triazolyl einschließlich 1 H-[1,2,4]-Triazol-1-yl und 4H-[1,2,4]-Triazol-3-yl, Tetrazolyl einschließlich 1 H-Tetrazol-1-yl und 1 H-Tetrazol-5-yl, Chinolinyl (= Chinolyl) einschließlich Chinolin-2-yl, Chinolin-3-yl, Chinolin-4-yl, Chinolin-5-yl, Chinolin-6-yl, Chinolin-7-yl und Chinolin-8-yl, die alle gegebenenfalls wie in der Definition der jeweiligen Gruppe angegeben substituiert sind. Beispiele für Reste von gesättigten und teilweise ungesättigten Heterocyclen, die in den Verbindungen der Formel I vorkommen können, sind Azetidinyl, Pyrrolidinyl einschließlich Pyrrolidin-1-yl, Pyrrolidin-2-yl und Pyrrolidin-3-yl, 2,5-Dihydro-1H-pyrrolyl, Piperidinyl einschließlich Piperidin-1-yl, Piperidin-2-yl, Piperidin-3-yl und Piperidin-4-yl, 1,2,3,4-Tetrahydropyridinyl, 1,2,5,6-Tetrahydropyridinyl, 1,2-Dihydropyridinyl, Azepanyl, Azocanyl, Azecanyl, Octahydrocyclopenta[b]pyrrolyl, 2,3-Dihydrobenzofuranyl einschließlich 2,3-Dihydrobenzofuran-7-yl, 2,3-Dihydro-1 H-indolyl, Octahydro-1 H-indolyl, 2,3-Dihydro-1 H-isoindolyl, Octahydro-1 H-isoindolyl, 1,2-Dihydrochinolinyl, 1,2,3,4-Tetrahydrochinolinyl, Decahydrochinolinyl, 1,2-Dihydroisochinolinyl, 1,2,3,4-Tetrahydroisochinolinyl, 1,2,3,4-Tetrahydroisochinolinyl, Decahydroisochinolinyl, Decahydroisochinolinyl, 4,5,6,7-Tetrahydrothieno[3,2-c]pyridinyl, Pyrazolidinyl, Imidazolidinyl, Hexahydropyrimidinyl, 1,2-Dihydropyrimidinyl, Piperazinyl, [1,3]Diazepanyl, [1,4]Diazepanyl, Oxazolidinyl, [1,3]Oxazinanyl, [1,3]Oxazepanyl, Morpholinyl einschließlich Morpholin-2-yl, Morpholin-3-yl und Morpholin-4-yl, [1,4]Oxazepanyl, Thiazolidinyl, [1,3]Thiazinanyl, Thiomorpholinyl einschließlich Thiomorpholin-2-yl, Thiomorpholin-3-yl und Thiomorpholin-4-yl, 3,4-Dihydro-2H-[1,4]thiazinyl, [1,3]Thiazepanyl, [1,4]Thiazepanyl, [1,4]Thiazepanyl, Oxetanyl, Tetrahydrofuranyl, Tetrahydrothienyl, Isoxazolidinyl, Isothiazolidinyl, Oxazolidinyl, [1,2,4]-Oxadiazolidinyl, [1,2,4]-Thiadiazolidinyl, [1,2,4]Triazolidinyl, [1,3,4]Oxadiazolidinyl, [1,3,4]Thiadiazolidinyl, [1,3,4]Triazolidinyl, 2,3-Dihydrofuranyl, 2,5-Dihydrofuranyl, 2,3-Dihydrothienyl, 2,5-Dihydrothienyl, 2,3-Dihydropyrrolyl, 2,3-Dihydroisoxazolyl, 4,5-Dihydroisoxazolyl, 2,5-Dihydroisoxazolyl, 2,3-Dihydroisothiazolyl, 4,5-Dihydroisothiazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Tetrahydropyridazinyl, Tetrahydropyrimidinyl, Tetrahydropyrazinyl, Tetrahydro[1,3,5]triazinyl, [1,3]Dithianyl, Tetrahydropyranyl, Tetrahydrothiopyranyl, [1,3]Dioxolanyl, 3,4,5,6-Tetrahydropyridinyl, 4H-[1,3]Thiazinyl, 1,1-Dioxo-2,3,4,5-tetrahydrothienyl, 2-Azabicyclo[3.1.0]hexyl einschließlich 2-Azabicyclo[3.1.0]hex-2-yl, 3-Azabicyclo[3.1.0]hexyl einschließlich 3-Azabicyclo[3.1.0]hex-3-yl, 2-Oxa-5-azabicyclo[2.2.1]heptyl einschließlich 2-Oxa-5-azabicyclo[2.2.1]hept-5-yl, die alle über ein geeignetes Ringkohlenstoffatom oder Ringstickstoffatom gebunden sind und gegebenenfalls wie in der Definition der jeweiligen Gruppe angegebenen substituiert sind.

Halogen steht für Fluor, Chlor, Brom oder lod. In einer Ausführungsform der Erfindung ist jedes Halogen in einer Verbindung der Formel I unabhängig von jedem anderen Halogen aus Fluor, Chlor und Brom ausgewählt, in einer anderen Ausführungsform aus Fluor und Chlor.

Wenn eine Oxogruppe an ein Kohlenstoffatom gebunden ist, ersetzt sie zwei Wasserstoffatome an einem Kohlenstoffatom des zugrundeliegenden Systems. Somit wird eine CH₂-Gruppe in einer Kette oder einem Ring dann, wenn sie durch Oxo, d.h. durch ein doppelt gebundenes Sauerstoffatom, substituiert ist, zu einer C(O)-Gruppe (= C(=O)-Gruppe). Offensichtlich kann eine Oxogruppe nicht als Substituent an einem Kohlenstoffatom in einem aromatischen Ring wie beispielsweise in einer Phenylgruppe vorkommen. Wenn ein Ringschwefelatom in einer heterocyclischen Gruppe eine oder zwei Oxogruppen tragen kann, handelt es sich in dem Fall, daß es keine Oxogruppe trägt, um ein nichtoxidiertes Schwefelatom S, oder in dem Fall, daß es eine Oxogruppe trägt, um eine S(O)-Gruppe (Sulfoxidgruppe, S-Oxid-Gruppe) oder in dem Fall, daß es zwei Oxogruppen trägt, um eine S(O)₂-Gruppe (= Sulfongruppe, S,S-Dioxid-Gruppe).

Die vorliegende Erfindung schließt alle stereoisomeren Formen der Verbindungen der Formel I und ihre Salze und Solvate ein. Bezüglich jedes chiralen Zentrums können die Verbindungen der Formel I unabhängig von jedem anderen chiralen Zentrum in S-Konfiguration oder weitgehend S-Konfiguration oder in R-Konfiguration oder weitgehend R-Konfiguration oder als Mischung des S-Isomers und des R-Isomers in beliebigen Verhältnissen vorliegen. Die Erfindung schließt alle möglichen Enantiomere und Diastereomere und Mischungen von zwei oder mehr Stereoisomeren, zum Beispiel Gemische von Enantiomeren und/oder Diastereomeren, in allen Verhältnissen ein. Somit können erfindungsgemäße Verbindungen, die als Enantiomere existieren können, in enantiomerenreiner Form sowohl als linksdrehende als auch rechtsdrehende Antipoden und in Form von Mischungen der beiden Enantiomere in allen Verhältnissen einschließlich Racematen vorliegen. Im Fall einer E/Z-Isomerie bzw. cis/trans-Isomerie, beispielsweise an Doppelbindungen oder Ringen wie Cycloalkylringen, schließt die Erfindung sowohl die E-Form als auch die Z-Form bzw. die cis-Form und die trans-Form sowie Mischungen dieser Formen in allen Verhältnissen ein. In einer Ausführungsform der Erfindung handelt es sich bei einer Verbindung, die in zwei oder mehr stereoisomeren Formen vorkommen kann, um ein reines oder weitgehend reines einzelnes Stereoisomer. Die Herstellung von einzelnen Stereoisomeren kann beispielsweise durch Trennung einer Mischung von Isomeren nach üblichen Methoden, beispielsweise durch Chromatographie oder Kristallisation, durch Verwendung von stereochemisch einheitlichen Ausgangsstoffen bei der Synthese oder durch stereoselektive Synthese erfolgen. Gegebenenfalls kann vor einer Trennung von Stereoisomeren eine Derivatisierung durchgeführt werden. Die Trennung einer Mischung von Stereoisomeren kann auf der Stufe der Verbindung der Formel I oder auf der Stufe eines Ausgangsstoffs oder eines Zwischenprodukts im Verlauf der Synthese durchgeführt werden. Die vorliegende Erfindung schließt auch alle tautomeren Formen der Verbindungen der Formel I und ihre Salze und Solvate ein.

Wenn die Verbindungen der Formel I eine oder mehrere saure und/oder basische Gruppen, d.h. salzbildende Gruppen, enthalten, schließt die Erfindung auch ihre entsprechenden physiologisch oder toxikologisch akzeptablen Salze, d.h. nichttoxischen Salze, insbesondere ihre pharmazeutisch akzeptablen Salze, ein. Somit können die Verbindungen der Formel I, die eine saure Gruppe, wie eine Hydroxycarbonylgruppe (= Carboxygruppe = C(O)-OH-Gruppe), enthalten, an derartigen Gruppen vorliegen und erfindungsgemäß verwendet werden, beispielsweise als Alkalimetallsalze, Erdalkalimetallsalze oder als Ammoniumsalze. Genauere Beispiele für derartige Salze sind Natriumsalze, Kaliumsalze, Calciumsalze, Magnesiumsalze, quartäre Ammoniumsalze wie Tetraalkylammoniumsalze oder Säureadditionssalze mit Ammoniak oder organischen Aminen wie beispielsweise Ethylamin, Ethanolamin, Triethanolamin oder Aminosäuren. Verbindungen der Formel I, die eine basische Gruppe, d.h. eine protonierbare Gruppe wie eine Aminogruppe oder einen Stickstoffheterocyclus, enthalten, können in Form ihrer Additionssalze mit anorganischen und organischen Säuren an derartigen Gruppen vorliegen und erfindungsgemäß verwendet werden. Beispiele für geeignete Säuren sind Chlorwasserstoff, Bromwasserstoff, Phosphorsäure, Schwefelsäure, Methansulfonsäure, Oxalsäure, Essigsäure, Trifluoressigsäure, Weinsäure, Milchsäure, Benzoesäure, Malonsäure, Fumarsäure, Maleinsäure, Citronensäure und andere dem Fachmann bekannten Säuren. Wenn eine Verbindung der Formel I gleichzeitig eine saure Gruppe und eine basische Gruppe im Molekül enthält, so schließt die Verbindung zusätzlich zu den erwähnten Salzformen auch innere Salze (= Betaine, Zwitterionen) ein. Die Salze der Verbindungen der Formel I sind nach dem Fachmann bekannten üblichen Methoden erhältlich, beispielsweise durch Inberührungbringen der Verbindung der Formel I in einem Lösungsmittel oder Verdünnungsmittel mit einer organischen oder anorganischen Säure oder Base oder durch Anionenaustausch oder Kationenaustausch aus einem anderen Salz. Die Erfindung schließt auch alle Salze der Verbindungen der Formel I ein, die aufgrund geringer physiologischer Verträglichkeit der salzbildenden Säure oder Base nicht direkt für eine Verwendung in Pharmazeutika geeignet sind, aber zum Beispiel als Zwischenprodukte für chemische Reaktionen oder zur Herstellung von physiologisch akzeptablen Salzen verwendet werden können.

Die vorliegende Erfindung schließt alle Solvate von Verbindungen der Formel I, beispielsweise Hydrate oder Addukte mit Alkoholen wie (C₁-C₄)-Alkanolen, aktive Metaboliten der Verbindungen der Formel I und auch Prodrugs und Derivate der Verbindungen der Formel I, die in vitro nicht unbedingt pharmakologische Wirkung zeigen, aber in vivo in pharmakologisch wirksame Verbindungen umgewandelt werden, beispielsweise Ester oder Amide von Carbonsäuregruppen, ein.

In einer Ausführungsform der Erfindung ist A aus NH und O ausgewählt, in einer anderen Ausführungsform ist A unter NH und S ausgewählt, in einer anderen Ausführungsform ist A unter O und S ausgewählt, in einer anderen Ausführungsform steht A für NH, in einer anderen Ausführungsform steht A für O, in einer anderen Ausführungsform steht A für S.

Die Alkandiyl-, Alkendiyl- und Alkindiylgruppen, die in der Gruppe X vorkommen, können linear oder verzweigt sein, wie bereits bezüglich derartiger Gruppen im allgemeinen angegeben, und diese Gruppen sowie Cycloalkandiylgruppen, die X repräsentieren, können über beliebige Positionen an die benachbarten Gruppen, d.h. die Gruppe R⁴O-C(O) und die Gruppe R² oder im Fall der Gruppe Alkandiyloxy an das Sauerstoffatom der Alkandiyloxygruppe, gebunden sein. Die benachbarten Gruppen können an das gleiche Kohlenstoffatom oder verschiedene Kohlenstoffatome in der Gruppe X gebunden sein. In einer Ausführungsform besteht die Kette von Kohlenstoffatomen in einer Alkandiyl-, Alkendiyl- und Alkindiylgruppe, die in der Gruppe X vorkommt, die die Gruppe R⁴O-C(O) direkt mit der Gruppe R² oder im Fall der Gruppe Alkandiyloxy mit dem Sauerstoffatom der Alkandiyloxygruppe verbindet, aus 1, 2, 3 oder 4 Kohlenstoffatomen, in einer anderen Ausführungsform aus 1, 2 oder 3 Kohlenstoffatomen, in einer anderen Ausführungsform aus 1 oder 2 Kohlenstoffatomen, in einer anderen Ausführungsform aus 1 Kohlenstoffatom. Im Fall einer X repräsentierenden Cycloalkandiylgruppe sind in einer Ausführungsform die Gruppen R⁴O-C(O) und R² an zwei Ringkohlenstoffatome gebunden, die in 1,2-Position, 1,3-Position oder 1,4-Position zueinander stehen, in einer anderen Ausführungsform in 1,2-Position oder 1,3-Position zueinander, in einer anderen Ausführungsform in 1,2-Position zueinander, in einer anderen Ausführungsform in 1,4-Position zueinander. In einer Ausführungsform ist X ausgewählt aus (C₁-C₆)-Alkandiyl, (C₂-C₆)-Alkendiyl, (C₃-C₇)-Cycloalkandiyl und (C₁-C₆)-Alkandiyloxy, in einer anderen Ausführungsform aus (C₁-C₆)-Alkandiyl, (C₂-C₆)-Alkendiyl und (C₁-C₆)-Alkandiyloxy, in einer anderen Ausführungsform aus (C₁-C₆)-Alkandiyl, (C₃-C₇)-Cycloalkandiyl und (C₁-C₆)-Alkandiyloxy, in einer Ausführungsform aus (C₁-C₆)-Alkandiyl und (C₁-C₆)-Alkandiyloxy, in einer anderen Ausführungsform aus (C₁-C₆)-Alkandiyl, (C₂-C₆)-Alkendiyl, (C₂-C₆)-Alkindiyl und (C₃-C₇)-Cycloalkandiyl, in einer anderen Ausführungsform aus (C₁-C₆)-Alkandiyl, (C₂-C₆)-Alkendiyl und (C₃-C₇)-Cycloalkandiyl, in einer anderen Ausführungsform aus (C₁-C₆)-Alkandiyl und (C₂-C₆)-Alkendiyl, in einer anderen Ausführungsform steht X für (C₁-C₆)-Alkandiyl, in einer anderen Ausführungsform steht X für (C₂-C₆)-Alkendiyl, in einer anderen Ausführungsform steht X für (C₃-C₇)-Cycloalkandiyl, und in einer anderen Ausführungsform steht X für (C₁-C₆)-Alkandiyloxy, die alle gegebenenfalls wie angegeben substituiert sind. In einer Ausführungsform ist eine (C₁-C₆)-Alkandiylgruppe, die in X vorkommt, eine (C₁-C₄)-Alkandiylgruppe, in einer anderen Ausführungsform eine (C₁-C₃)-Alkandiyl-gruppe, in einer anderen Ausführungsform eine (C₁-C₂)-Alkandiylgruppe. In einer Ausführungsform sind die (C₂-C₆)-Alkendiyl- und (C₂-C₆)-Alkindiylgruppen, die X repräsentieren, (C₂-C₄)-Alkendiyl- und (C₂-C₄)-Alkindiylgruppen, in einer anderen Ausführungsform (C₂-C₃)-Alkendiyl- und (C₂-C₃)-Alkindiylgruppen. In einer Ausführungsform ist eine (C₃-C₇)-Cycloalkandiylgruppe, die X repräsentiert, eine (C₃-C₆)-Cycloalkandiylgruppe, in einer anderen Ausführungsform eine (C₃-C₄)-Cycloalkandiylgruppe, in einer anderen Ausführungsform eine Cyclopropandiylgruppe, in einer anderen Ausführungsform eine Cyclohexandiylgruppe. Beispiele für Gruppen X, aus denen die jeweilige X repräsentierende Gruppe in den oben aufgeführten Ausführungsformen gewählt sein kann oder aus denen in einer anderen Ausführungsform der Erfindung X ausgewählt sein kann, sind Methylen, -CH(CH₃)-(Ethan-1,1-diyl), -CH₂-CH₂-(Ethan-1,2-diyl, 1,2-Ethylen), -C(CH₃)₂-(1-Methylethan-1,1-diyl),] -CH₂-CH₂-CH₂-(Propan-1,3-diyl, 1,3-Propylen), -CH₂-CH(CH₃)- und -CH(CH₃)-CH₂- (Propan-1,2-diyl, 1,2-Propylen), die die Gruppe (C₁-C₆)-Alkandiyl exemplifizieren, -CH=CH- (Ethen-1,2-diyl), -CH=CH-CH₂- und -CH₂-CH=CH- (Prop-1-en-1,3-diyl und Prop-2-en-1,3-diyl) und -CH=C(CH₃)- und -C(CH₃)=CH- (Prop-1-en-1,2-diyl), die die Gruppe (C₂-C₆)-Alkendiyl exemplifizieren, -C≡C- (Ethindiyl) und -CH₂-C≡C- und -C≡C-CH₂- (Prop-1-in-1,3-diyl und Prop-2-in-1,3-diyl), die die Gruppe (C₂-C₆)-Alkindiyl exemplifizieren, Cyclopropan-1,1-diyl, Cyclopropan-1,2-diyl und Cyclohexan-1,4-diyl, die die Gruppe (C₃-C₇)-Cycloalkandiyl exemplifizieren, -CH₂-O- (Methylenoxy), -CH₂-CH₂-O- (Ethan-1,2-diyloxy), -CH(CH₃)-O- (Ethan-1,1-diyloxy), -C(CH₃)₂-O- (1-Methylethan-1,1-diyloxy), -CH₂-CH₂-CH₂-O- (Propan-1,3-diyloxy) und -CH₂-CH₂-CH₂-CH₂-O- (Butan-1,4-diyloxy), die die Gruppe (C₁-C₆)-Alkandiyloxy exemplifizieren, wobei alle diese Gruppen gegebenenfalls wie angegeben substituiert sind. So ist in einer Ausführungsform X ausgewählt aus -CH₂-O-, -CH₂-CH₂-O-, -CH(CH₃)-O- und -C(CH₃)₂-O-, in einer anderen Ausführungsform aus -CH₂-O-, -CH₂-CH₂-O- und -CH(CH₃)-O-, in einer anderen Ausführungsform aus -CH₂-O- und - CH(CH₃)-O-, und in einer anderen Ausführungsform steht X für -CH₂-O-, wobei alle diese Gruppen gegebenenfalls wie angegeben substituiert sind und wobei das Sauerstoffatom an die Gruppe R² gebunden ist. In einer Ausführungsform ist die Zahl der Substituenten, die gegebenenfalls in X vorliegen, 1, 2, 3 oder 4, in einer anderen Ausführungsform 1, 2 oder 3, in einer anderen Ausführungsform 1 oder 2, in einer anderen Ausführungsform 1, und in einer anderen Ausführungsform ist die Gruppe X nicht durch aus Fluor und Hydroxy ausgewählte Substituenten substituiert. In einer Ausführungsform ist die Zahl der Hydroxysubstituenten in X nicht größer als 2, in einer anderen Ausführungsform nicht größer als 1. In einer Ausführungsform liegt an einem einzelnen Kohlenstoffatom in X nicht mehr als ein Hydroxysubstituent vor. In einer Ausführungsform liegen an Kohlenstoffatomen, die Teil einer Doppelbindung in der Gruppe (C₂-C₆)-Alkendiyl sind, keine Hydroxysubstituenten vor. In einer Ausführungsform liegen an dem Kohlenstoffatom in der Gruppe (C₁-C₆)-Alkandiyloxy, das an das Sauerstoffatom gebunden ist, keine Hydroxysubstituenten vor, in einer anderen Ausführungsform liegen an dem Kohlenstoffatom in der Gruppe (C₁-C₆)-Alkandiyloxy, das an das Sauerstoffatom gebunden ist, keine Substituenten vor, d.h. in dieser letztgenannten Ausführungsform sind alle Kohlenstoffatome, die nicht an das Sauerstoffatom gebunden sind, gegebenenfalls durch einen oder mehrere gleiche oder verschiedene Substituenten substituiert, die aus Fluor und Hydroxy ausgewählt sind. Die Doppelbindung in der Gruppe (C₂-C₆)-Alkendiyl kann E-Konfiguration oder Z-Konfiguration haben. In einer Ausführungsform hat sie E-Konfiguration, in einer anderen Ausführungsform hat sie Z-Konfiguration.

In einer anderen Ausführungsform der Erfindung ist die Zahl t aus 0, 1 oder 2 ausgewählt, in einer anderen Ausführungsform aus 0 oder 1, in einer anderen Ausführungsform aus 1, 2 oder 3, in einer anderen Ausführungsform aus 1 oder 2, in einer anderen Ausführungsform steht t für 0, in einer anderen Ausführungsform steht t für 1. In einer Ausführungsform ist R¹ aus (C₁-C₆)-Alkyl, (C₃-C₇)-Cycloalkyl-CₜH₂ₜ- und Het-CₜH₂ₜ- ausgewählt, in einer anderen Ausführungsform aus (C₁-C₆)-Alkyl und (C₃-C₇)-Cycloalkyl-CₜH₂ₜ-, in einer anderen Ausführungsform steht R¹ für (C₁-C₆)-Alkyl, in einer anderen Ausführungsform steht R¹ für (C₃-C₇)-Cycloalkyl-CₜH₂ₜ-, und in einer anderen Ausführungsform steht R¹ für Het-CₜH₂ₜ-. In einer Ausführungsform steht R¹ für (C₃-C₇)-Cycloalkyl-CₜH₂ₜ-, worin t aus 0, 1 und 2 ausgewählt ist, in einer anderen Ausführungsform steht R¹ für (C₃-C₇)-Cycloalkyl-CₜH₂ₜ-, worin t aus 0 und 1 ausgewählt ist, in einer anderen Ausführungsform steht R¹ für (C₃-C₇)-Cycloalkyl-CH₂-, in einer anderen Ausführungsform steht R¹ für (C₃-C₇)-Cycloalkyl, in einer anderen Ausführungsform steht R¹ für Het-CₜH₂ₜ-, worin t unter 0, 1 und 2 ausgewählt ist, in einer anderen Ausführungsform steht R¹ für Het-CₜH₂ₜ-, worin t unter 0 und 1 ausgewählt ist, in einer anderen Ausführungsform steht R¹ für Het-CH₂-, in einer anderen Ausführungsform steht R¹ für Het. In einer Ausführungsform ist eine (C₁-C₆)-Alkylgruppe, die R¹ repräsentiert, (C₂-C₆)-Alkyl, in einer anderen Ausführungsform (C₂-C₅)-Alkyl, in einer anderen Ausführungsform (C₃-C₅)-Alkyl. In einer Ausführungsform sind eine (C₂-C₆)-Alkenylgruppe und eine (C₂-C₆)-Alkinylgruppe, die R¹ repräsentieren, (C₃-C₆)-Alkenyl bzw. (C₃-C₆)-Alkinyl, in einer anderen Ausführungsform (C₃-C₄)-Alkenyl bzw. (C₃-C₄)-Alkinyl. In einer Ausführungsform ist eine (C₃-C₇)-Cycloalkylgruppe, die in R¹ vorliegt, (C₃-C₆)-Cycloalkyl, in einer anderen Ausführungsform (C₃-C₅)-Cycloalkyl, in einer anderen Ausführungsform (C₃-C₄)-Cycloalkyl, in einer anderen Ausführungsform Cyclopropyl. In einer Ausführungsform ist eine Gruppe Het, die R¹ repräsentiert, ein 4-gliedriger bis 6-gliedriger, in einer anderen Ausführungsform ein 4-gliedriger bis 5-gliedriger, in einer anderen Ausführungsform ein 4-gliedriger, gesättigter monocyclischer Heterocyclus, der über ein Ringkohlenstoffatom gebunden ist, der 1 oder 2 gleiche oder verschiedene Ringheteroatome, in einer anderen Ausführungsform 1 Ringheteroatom, enthält, die aus N, O und S, in einer anderen Ausführungsform aus O und S, ausgewählt sind und in einer anderen Ausführungsform für O-Atome stehen. In einer Ausführungsform ist eine Gruppe Het, die R¹ repräsentiert, eine Oxetanylgruppe, beispielsweise eine Oxetan-3-ylgruppe. In einer Ausführungsform ist die Zahl der Substituenten, die gegebenenfalls an einer R¹ repräsentierenden Gruppe Het vorliegen, eins, zwei oder drei, in einer anderen Ausführungsform eins oder zwei, in einer anderen Ausführungsform eins, und in einer anderen Ausführungsform ist eine derartige Gruppe Het unsubstituiert. In einer Ausführungsform ist ein (C₁-C₄)-Alkylsubstituent, der an einer R¹ repräsentierenden Gruppe Het vorkommt, eine Methylgruppe.

In einer Ausführungsform der Erfindung ist die Zahl der Ringheteroatome in einem aromatischen Heterocyclus, der R² repräsentiert, 1 oder 2, in einer anderen Ausführungsform 1. In einer Ausführungsform der Erfindung ist R² aus Phenylen und einem zweiwertigen Rest eines aromatischen, 6-gliedrigen monocyclischen Heterocyclus, der 1, 2 oder 3 Ringstickstoffatome, in einer anderen Ausführungsform 1 oder 2 Ringstickstoffatome, in einer anderen Ausführungsform 1 Ringstickstoffatom enthält, ausgewählt, wobei eines der Ringstickstoffatome einen Substituenten R²¹ tragen kann, der Oxy ist, d.h. wobei eines der Ringstickstoffatome zum N-Oxid oxidiert sein kann, und wobei das Phenylen und der zweiwertige Rest eines aromatischen Heterocyclus gegebenenfalls an einem oder mehreren Ringkohlenstoffatomen durch gleiche oder verschiedene Substituenten R²² substituiert sind. In einer anderen Ausführungsform steht R² für Phenylen, wobei das Phenylen gegebenenfalls an einem oder mehreren Ringatomen durch gleiche oder verschiedene Substituenten R²² substituiert ist, und in einer anderen Ausführungsform steht R² für Pyridindiyl, wobei das Ringstickstoffatom einen Substituenten R²¹, der Oxy ist, tragen kann, d.h. wobei das Ringstickstoffatom zum N-Oxid oxidiert sein kann, und wobei das Pyridindiyl gegebenenfalls an einem oder mehreren Ringkohlenstoffatomen durch gleiche oder verschiedene Substituenten R²² substituiert ist. In einer anderen Ausführungsform steht R² für einen zweiwertigen Rest eines aromatischen 5-gliedrigen Heterocyclus, der 1, 2 oder 3 gleiche oder verschiedene Ringheteroatome, die aus N, O und S ausgewählt sind, enthält, wobei eines der Ringstickstoffatome ein Wasserstoffatom oder einen Substituenten R²¹ tragen kann und wobei der zweiwertige Rest eines aromatischen Heterocyclus gegebenenfalls an einem oder mehreren Ringkohlenstoffatomen durch gleiche oder verschiedene Substituenten R²² substituiert ist. In einer Ausführungsform ist ein zweiwertiger Rest einer aromatischen heterocyclischen Gruppe, die R² repräsentiert, aus Furandiyl, Thiophendiyl, Oxazoldiyl, Thiazoldiyl, Pyridindiyl, Pyridazindiyl, Pyrimidindiyl und Pyrazindiyl ausgewählt, in einer anderen Ausführungsform aus Furandiyl, Thiophendiyl, Thiazoldiyl, Pyridindiyl, Pyridazindiyl, Pyrimidindiyl und Pyrazindiyl, in einer anderen Ausführungsform aus Furandiyl, Thiophendiyl, Pyridindiyl, Pyridazindiyl, Pyrimidindiyl und Pyrazindiyl, in einer anderen Ausführungsform aus Furandiyl, Thiophendiyl, Pyridindiyl und Pyrimidindiyl, in einer anderen Ausführungsform aus Furandiyl, Thiophendiyl und Pyridindiyl, die alle gegebenenfalls wie in bezug auf R² angegeben substituiert sind.

Die Ringkohlenstoffatome, über die die Phenylengruppe und der zweiwertige Rest eines aromatischen Heterocyclus, die bzw. der R² repräsentieren, an den Oxazolopyrimidinring und die Gruppe X gebunden sind, können in beliebigen Positionen vorliegen. Eine R² repräsentierende Phenylengruppe kann 1,2-Phenylen sein, d.h. der Oxazolopyrimidinring und die Gruppe X können in 1,2-Position oder ortho-Position zueinander gebunden sein, sie kann 1,3-Phenylen sein, d.h. der Oxazolopyrimidinring und die Gruppe X können in 1,3-Position oder meta-Position zueinander gebunden sein, und sie kann 1,4-Phenylen sein, d.h. der Oxazolopyrimidinring und die Gruppe X können in 1,4-Position oder para-Position zueinander gebunden sein. In einer Ausführungsform ist eine R² repräsentierende Phenylengruppe ausgewählt aus 1,3-Phenylen und 1,4-Phenylen, in einer anderen Ausführungsform ist sie 1,3-Phenylen, und in einer anderen Ausführungsform ist sie 1,4-Phenylen, wobei alle diese Gruppen gegebenenfalls wie in bezug auf R² angegeben substituiert sind. In einer Ausführungsform ist R² ausgewählt aus einer oder mehreren der Gruppen Phenylen, Furan-2,5-diyl, Thiophen-2,4-diyl, Thiophen-2,5-diyl, Pyridin-2,4-diyl, Pyridin-2,5-diyl, Pyridin-3,5-diyl, Pyridin-2,6-diyl und Pyrimidin-2,5-diyl, in einer anderen Ausführungsform aus den Gruppen Furan-2,5-diyl, Thiophen-2,4-diyl, Thiophen-2,5-diyl, Pyridin-2,4-diyl, Pyridin-2,5-diyl, Pyridin-3,5-diyl, Pyridin-2,6-diyl und Pyrimidin-2,5-diyl, in einer anderen Ausführungsform aus Pyridin-2,4-diyl, Pyridin-2,5-diyl, Pyridin-3,5-diyl und Pyridin-2,6-diyl, in einer anderen Ausführungsform aus Phenylen, Pyridin-2,4-diyl, Pyridin-2,5-diyl, Pyridin-3,5-diyl und Pyridin-2,6-diyl, die alle gegebenenfalls wie in bezug auf R² angegeben substituiert sind. In einer Ausführungsform ist die Zahl der Substituenten R²², die gegebenenfalls an Ringkohlenstoffatomen in R² vorliegen können, 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Ringkohlenstoffatome in R², die keinen Substituenten R²² tragen, tragen ein Wasserstoffatom.

In einer Ausführungsform der Erfindung ist R³ aus (C₁-C₆)-Alkyl, (C₂-C₆)-Alkenyl und (C₂-C₆)-Alkinyl ausgewählt, in einer anderen Ausführungsform steht R³ für (C₁-C₆)-Alkyl, in einer anderen Ausführungsform steht R³ für (C₂-C₅)-Alkyl, und in einer anderen Ausführungsform steht R³ für (C₁-C₄)-Alkyl, mit der Maßgabe, daß R³ nicht für eine Alkylgruppe stehen kann, wenn A für S steht. In einer anderen Ausführungsform ist R³ aus (C₃-C₇)-Cycloalkyl-CᵤH₂ᵤ- und Het-CᵥH₂ᵥ- ausgewählt, in einer anderen Ausführungsform steht R³ für (C₃-C₇)-Cycloalkyl-CᵤH₂ᵤ-, und in einer anderen Ausführungsform steht R³ für Het-CᵥH₂ᵥ-, wobei in dieser Ausführungsform u und v unabhängig voneinander aus 1 und 2 ausgewählt sind. In einer Ausführungsform steht u für 1, in einer anderen Ausführungsform steht u für 2. In einer Ausführungsform steht v für 1, in einer anderen Ausführungsform steht v für 2. In einer Ausführungsform ist die Gruppe (C₃-C₇)-Cycloalkyl-CᵤH₂ᵤ-, die R³ repräsentiert, aus Cyclopropyl-CᵤH₂ᵤ-, Cyclobutyl-CᵤH₂ᵤ- und Cyclopentyl-CᵤH₂ᵤ- ausgewählt und die Gruppe Het-CᵥH₂ᵥ-, die R³ repräsentiert, ist Tetrahydrofuranyl- CᵥH₂ᵥ-. In einer Ausführungsform ist R³ aus Cyclopropyl-CᵤH₂ᵤ-, Cyclobutyl-CᵤH₂ᵤ- und Cyclopentyl-CᵤH₂ᵤ₋ ausgewählt.

In einer Ausführungsform ist R³ aus (C₃-C₇)-Cycloalkyl-CuH₂ᵤ- und Het-CᵥH₂ᵥ-ausgewählt, oder R³ steht für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome, die unter N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, und in einer anderen Ausführungsform steht R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome, die aus N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist. In einer Ausführungsform ist die Zahl der Ringheteroatome im Ring, der R³ repräsentiert, 0, 1, 2 oder 3, in einer anderen Ausführungsform 0, 1 oder 2, in einer anderen Ausführungsform 0 oder 1, in einer anderen Ausführungsform 0, in einer anderen Ausführungsform ist sie 1, 2, 3 oder 4, in einer anderen Ausführungsform 1, 2 oder 3, in einer anderen . Ausführungsform 1 oder 2, in einer anderen Ausführungsform 1. Der Rest des Rings, der R³ repräsentiert, kann somit carbocydisch oder heterocyclisch sein. In einer Ausführungsform sind die Ringheteroatome in R³ aus N und O ausgewählt, in einer anderen Ausführungsform aus N und S, in einer anderen Ausführungsform aus O und S, in einer anderen Ausführungsform stehen sie für N, wobei Ringstickstoffatome ein Wasserstoffatom oder einen (C₁-C₄)-Alkylsubstituenten tragen können, wie es beispielsweise in gesättigten oder teilweise ungesättigten Heterocyclen oder in 5-gliedrigen aromatischen Ringen in Heterocyclen wie Pyrrol oder Benzoimidazol vorkommt, oder kein Wasserstoffatom und keinen (C₁-C₄)-Alkylsubstituenten tragen, wie es in aromatischen Heterocyclen wie beispielsweise Imidazol oder Pyridin vorkommt. In einem Rest eines R³ repräsentierenden Heterocyclus, der ein oder mehrere Ringschwefelatome enthält, ist in einer Ausführungsform eines der Ringschwefelatome nicht oxidiert oder trägt eine oder zwei Oxogruppen, und alle anderen Ringschwefelatome sind nicht oxidiert. Der Rest eines monocyclischen oder bicyclischen Rings, der R³ repräsentiert, kann über ein beliebiges geeignetes Ringkohlenstoffatom oder Ringstickstoffatom an die Gruppe A gebunden sein. In einer Ausführungsform ist er über ein Ringkohlenstoffatom gebunden, in einer anderen Ausführungsform ist er über ein Ringkohlenstoffatom oder dann, wenn A für NH steht, über ein Ringstickstoffatom gebunden, und in einer anderen Ausführungsform ist er über ein Ringstickstoffatom gebunden. Der Rest eines monocyclischen oder bicyclischen Rings, der R³ repräsentiert, kann ungesättigt sein und in diesem Fall 1, 2, 3, 4 oder 5 oder 1, 2, 3 oder 4 oder 1, 2 oder 3 oder 1 oder 2 oder 1 Doppelbindungen im Ring enthalten und kann in jedem der beiden Ringe aromatisch oder nichtaromatisch sein, oder er kann gesättigt sein und in diesem Fall keine Doppelbindungen im Ring enthalten. In einer Ausführungsform ist der Rest des Rings, der R³ repräsentiert, gesättigt oder aromatisch, in einer anderen Ausführungsform ist er gesättigt, und in einer anderen Ausführungsform ist er aromatisch. In einer Ausführungsform ist der Rest des 3-gliedrigen oder 4-gliedrigen Rings, der R³ repräsentiert, gesättigt. Wenn R³ Ringstickstoffatome enthält, die ein Wasserstoffatom oder einen (C₁-C₄)-Alkylsubstituenten tragen können, kann ein derartiges Ringstickstoffatom bzw. können zwei derartige Ringstickstoffatome vorliegen. In einer Ausführungsform ist die Zahl der fakultativen Substituenten R³¹ an Ringkohlenstoffatomen in dem R³ repräsentierenden Ring 1, 2, 3, 4, 5 oder 6, in einer anderen Ausführungsform 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1.

Der Ring, der R³ repräsentieren kann, kann 3-gliedrig, 4-gliedrig, 5-gliedrig, 6-gliedrig, 7-gliedrig, 8-gliedrig, 9-gliedrig oder 10-gliedrig sein. In einer Ausführungsform ist R³ 4-gliedrig bis 10-gliedrig, in einer anderen Ausführungsform 4-gliedrig bis 9-gliedrig, in einer anderen Ausführungsform 4-gliedrig bis 8-gliedrig, in einer anderen Ausführungsform 4-gliedrig bis 7-gliedrig, in einer anderen Ausführungsform 5-gliedrig bis 7-gliedrig, in einer anderen Ausführungsform 5-gliedrig oder 6-gliedrig, in einer anderen Ausführungsform 6-gliedrig, in einer anderen Ausführungsform 8-gliedrig bis 10-gliedrig, in einer anderen Ausführungsform 9-gliedrig bis 10-gliedrig. In einer Ausführungsform enthält ein 3-gliedriger Ring, der R³ repräsentiert, keine Ringheteroatome. In einer Ausführungsform ist R³ monocyclisch, in einer anderen Ausführungsform bicyclisch. In einer Ausführungsform ist eine bicyclische Gruppe, die R³ repräsentiert, mindestens 7-gliedrig. Unter anderem kann der Rest eines Rings, der R³ repräsentiert, eine Cycloalkylgruppe, eine Phenylgruppe, eine Naphthylgruppe, ein Rest einer ungesättigten, aromatischen oder nichtaromatischen heterocyclischen Gruppe oder ein Rest einer gesättigten heterocyclischen Gruppe sein, die alle gegebenenfalls an Ringkohlenstoffatomen und Ringstickstoffatomen wie in bezug auf R³ angegeben substituiert sind. Soweit anwendbar, gelten alle oben angegebenen Erklärungen in bezug auf derartige Gruppen entsprechend für R³. Ein anderes Beispiel für Gruppen, die R³ repräsentieren können, sind Cycloalkenylgruppen wie (C₅-C₇)-Cycloalkenylgruppen, die über ein beliebiges Ringkohlenstoffatom gebunden sein können und gegebenenfalls wie in bezug auf R³ angegeben substituiert sind. In einer Ausführungsform sind fakultative Substituenten R³¹ an einer R³ repräsentierenden Cycloalkenylgruppe aus Fluor und (C₁-C₄)-Alkyl ausgewählt. In einer Ausführungsform enthalten Cycloalkenylgruppen eine Doppelbindung im Ring, die in einer beliebigen Position vorliegen kann. Beispiele für Cycloalkenyl sind Cyclopentenyl einschließlich Cyclopent-1-enyl, Cyclopent-2-enyl und Cyclopent-3-enyl, Cyclohexenyl einschließlich Cyclohex-1-enyl, Cyclohex-2-enyl und Cyclohex-3-enyl und Cycloheptenyl einschließlich Cyclohept-1-enyl, Cyclohept-2-enyl, Cyclopent-3-enyl und Cyclohept-4-enyl. Beispiele für Reste von Ringen, aus denen R³ in einer Ausführungsform der Erfindung ausgewählt ist, sind Cyclopropyl, Cyclobutyl, Cyclopentyl, Cyclohexyl, Phenyl, Oxetanyl einschließlich Oxetan-3-yl, Tetrahydrofuranyl einschließlich Tetrahydrofuran-3-yl, Tetrahydrothiophenyl einschließlich Tetrahydrothiophen-3-yl, Tetrahydropyranyl einschließlich Tetrahydropyran-4-yl, Azetidinyl einschließlich Azetidin-1-yl, Pyrrolidinyl, Piperidinyl, Imidazolidinyl, Piperazinyl, Morpholinyl einschließlich Morpholin-1-yl, Thiomorpholinyl, Furanyl einschließlich Furan-3-yl, Thiophenyl einschließlich Thiophen-3-yl, Pyrazolyl einschließlich Pyrazol-3-yl, Imidazolyl, Thiazolyl einschließlich Thiazol-2-yl, Pyridinyl einschließlich Pyridin-2-yl, Pyridin-3-yl und Pyridin-4-yl, Pyridazinyl einschließlich Pyridazin-3-yl, wobei in allen davon, sofern anwendbar, ein oder zwei der Ringstickstoffatome ein Wasserstoffatom oder (C₁-C₄)-Alkyl tragen können und wobei alle davon gegebenenfalls an einem oder mehreren Ringkohlenstoffatomen durch gleiche oder verschiedene Substituenten R³¹ substituiert sind und wobei in allen davon, sofern anwendbar, ein Ringschwefelatom nichtoxidiert sein kann, d.h. als Schwefelatom vorliegen kann, oder eine oder zwei Oxogruppen tragen kann, d.h. in Form eines Sulfoxids oder Sulfons vorliegen kann.

In einer Ausführungsform ist R³ aus Phenyl und einem Rest eines gesättigten oder ungesättigten 3-gliedrigen bis 7-gliedrigen, monocyclischen Rings ausgewählt, in einer anderen Ausführungsform aus Phenyl und einem Rest eines gesättigten oder ungesättigten 5-gliedrigen bis 7-gliedrigen, monocyclischen Rings, in einer anderen Ausführungsform aus Phenyl, Pyridinyl und einem Rest eines gesättigten 3-gliedrigen bis 7-gliedrigen, monocyclischen Rings, in einer anderen Ausführungsform aus Phenyl, Pyridinyl und einem Rest eines gesättigten 5-gliedrigen bis 7-gliedrigen, monocyclischen Rings, in einer anderen Ausführungsform aus Phenyl und einem Rest eines gesättigten 3-gliedrigen bis 7-gliedrigen, monocyclischen Rings, in einer anderen Ausführungsform aus Phenyl und einem Rest eines gesättigten 5-gliedrigen bis 7-gliedrigen, monocyclischen Rings, wobei in allen diesen Ausführungsformen der monocyclische Ring 1 oder 2 gleiche oder verschiedene Ringheteroatome, die aus N, O und S ausgewählt sind, enthält, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei das Phenyl, das Pyridinyl und der Rest eines Rings gegebenenfalls an einem oder mehreren Ringkohlenstoffatomen durch gleiche oder verschiedene Substituenten R³¹ substituiert sind und wobei Pyridinyl Pyridin-2-yl, Pyridin-3-yl und Pyridin-4-yl einschließt. In einer Ausführungsform steht R³ für Phenyl, das gegebenenfalls durch einen oder mehrere gleiche oder verschiedene Substituenten R³¹ substituiert ist.

In einer Ausführungsform der Erfindung ist die Zahl z aus 0 und 1 ausgewählt, in einer anderen Ausführungsform steht sie für 0, in einer anderen Ausführungsform steht sie für 1. In einer Ausführungsform der Erfindung ist die Gruppe R⁴ aus Wasserstoff und (C₁-C₄)-Alkyl ausgewählt, in einer anderen Ausführungsform ist R⁴ aus Wasserstoff, Methyl, Ethyl, n-Propyl, n-Butyl und Isopropyl ausgewählt, in einer anderen Ausführungsform aus Wasserstoff, Methyl und Ethyl, in einer anderen Ausführungsform steht R⁴ für Wasserstoff, in einer anderen Ausführungsform steht R⁴ für (C₁-C₄)-Alkyl, in einer anderen Ausführungsform steht R⁴ für Methyl, und in einer anderen Ausführungsform steht R⁴ für Ethyl. In einer Ausführungsform ist eine (C₃-C₇)-Cycloalkylgruppe, die in R⁴ vorliegt, (C₃-C₆)-Cycloalkyl, in einer anderen Ausführungsform Cyclopropyl.

In einer Ausführungsform der Erfindung ist die Zahl w aus 0 und 1 ausgewählt, in einer anderen Ausführungsform steht sie für 0, in einer anderen Ausführungsform steht sie für 1. In einer Ausführungsform ist eine (C₃-C₇)-Cycloalkylgruppe, die in R²¹ vorliegt, (C₃-C₆)-Cycloalkyl, in einer anderen Ausführungsform (C₃-C₅)-Cycloalkyl, in einer anderen Ausführungsform Cyclopropyl. In einer Ausführungsform ist R²¹ aus (C₁-C₄)-Alkyl und Oxy ausgewählt, in einer anderen Ausführungsform steht R²¹ für (C₁-C₄)-Alkyl, in einer anderen Ausführungsform für (C₁-C₃)-Alkyl, in einer anderen Ausführungsform für Methyl und in einer anderen Ausführungsform für Oxy.

In einer Ausführungsform der Erfindung sind die Substituenten R²², die gegebenenfalls an der Gruppe R² vorliegen, aus Halogen, Hydroxy, (C₁-C₄)-Alkyl-, (C₁-C₄)-Alkyloxy-, (C₁-C₄)-Alkyl-S(O)ₘ-, Amino, Nitro und Cyano ausgewählt, in einer anderen Ausführungsform aus Halogen, Hydroxy, (C₁-C₄)-Alkyl-, (C₁-C₄)-Alkyloxy-, Amino und Cyano, in einer anderen Ausführungsform aus Halogen, Hydroxy, (C₁-C₄)-Alkyl- und (C₁-C₄)-Alkyloxy-, in einer anderen Ausführungsform aus Fluor, Chlor, Hydroxy, (C₁-C₄)-Alkyl- und (C₁-C₄)-Alkyloxy-, in einer anderen Ausführungsform aus Fluor, Chlor und (C₁-C₄)-Alkyl-, und in einer anderen Ausführungsform sind sie (C₁-C₄)-Alkylsubstituenten.

In einer Ausführungsform sind 1, 2 oder 3 der Substituenten R²², in einer anderen Ausführungsform 1 oder 2 der Substituenten R²² und in einer anderen Ausführungsform 1 der Substituenten R²², die gegebenenfalls an der Gruppe R² vorliegen, wie in der allgemeinen Definition von R²² definiert und somit aus Halogen, Hydroxy, (C₁-C₄)-Alkyl-, (C₁-C₄)-Alkyloxy-, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl ausgewählt, und jegliche weiteren Substituenten R²², die gegebenenfalls an der Gruppe R² vorliegen, beispielsweise 1, 2 oder 3 weitere Substituenten R²², oder 1 oder 2 weitere Substituenten R²² oder 1 weiterer Substituent R²², sind aus Halogen, Hydroxy, (C₁-C₄)-Alkyl-, (C₁-C₄)-Alkyloxy-, (C₁-C₄)-Alkyl-S(O)ₘ-, Amino, Nitro und Cyano ausgewählt, wobei alle Alkylgrupppen unabhängig voneinander gegebenenfalls durch einen oder mehrere Fluorsubstituenten substituiert sind, wie es allgemein für Alkylgruppen gilt. In einer Ausführungsform sind die Substituenten R²², die gegebenenfalls an der Gruppe R² vorliegen und in der oben aufgeführten Ausführungsform wie in der allgemeinen Definition von R²² definiert sind, beispielsweise 1 oder 2 derartige Substituenten R²² oder 1 derartiger Substituent R²², aus Halogen, Hydroxy, (C₁-C₄)-Alkyl-, (C₁-C₄)-Alkyloxy-, (C₁-C₄)-Alkyl-S(O)ₘ-, Amino und Cyano ausgewählt. In einer Ausführungsform befinden sich die Substituenten R²², die gegebenenfalls an der Gruppe R² vorliegen und in der oben aufgeführten Ausführungsform wie in der allgemeinen Definition von R²² definiert sind, beispielsweise 1 oder 2 derartige Substituenten R²² oder 1 derartiger Substituent R²², nicht an Ringkohlenstoffatomen in der Gruppe R², die dem Atom, über das die Gruppe R² an den in Formel I dargestellten Oxazolopyrimidinring gebunden ist, benachbart ist. In einer Ausführungsform sind die weiteren Substituenten R²², die gegebenenfalls an der Gruppe R² vorliegen, beispielsweise 1, 2 oder 3 weitere Substituenten R²² oder 1 oder 2 weitere Substituenten R²² oder 1 weiterer Substituent R²², aus Halogen, Hydroxy, (C₁-C₄)-Alkyl-, (C₁-C₄)-Alkyloxy-, Amino, Cyano ausgewählt, in einer anderen Ausführungsform aus Halogen, Hydroxy, (C₁-C₄)-Alkyl-, (C₁-C₄)-Alkyloxy-, in einer anderen Ausführungsform aus Halogen, (C₁-C₄)-Alkyl- und (C₁-C₄)-Alkyloxy-, in einer anderen Ausführungsform aus Halogen und (C₁-C₄)-Alkyl-, wobei in allen diesen Ausführungsformen alle Alkylgruppen unabhängig voneinander gegebenenfalls durch einen oder mehrere Fluorsubstituenten substituiert sind.

In einer Ausführungsform der Erfindung ist R³¹ aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkyl-sulfonylamino, Cyano, (C₁-C₄)-Alkylcarbonyl, Aminosulfonyl, (C₁-C₄)-Alkyl-aminosulfonyl und Di((C₁-C₄)-alkyl)aminosulfonyl ausgewählt, in einer anderen Ausführungsform aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, Cyano, Aminosulfonyl, (C₁-C₄)-Alkylaminosulfonyl und Di((C₁-C₄)-alkyl)aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O),-, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino und Di((C₁-C₄)-alkyl)amino, in einer anderen Ausführungsform aus Halogen, (C₁-C₄)-Alkyl, (C₃-C₇)-Cycloalkyl, (C₁-C₄)-Alkyloxy und Di((C₁-C₄)-alkyl)amino, in einer anderen Ausführungsform aus Halogen, (C₁-C₄)-Alkyl, (C₃-C₇)-Cycloalkyl, Hydroxy und (C₁-C₄)-Alkyloxy, in einer anderen Ausführungsform aus Halogen, (C₁-C₄)-Alkyl und (C₁-C₄)-Alkyloxy, in einer anderen Ausführungsform aus Fluor, Chlor, (C₁-C₄)-Alkyl, (C₃-C₇)-Cycloalkyl, Hydroxy und (C₁-C₄)-Alkyloxy, wobei in allen diesen Ausführungsformen alle Alkylgruppen unabhängig voneinander gegebenenfalls durch einen oder mehrere Fluorsubstituenten substituiert sind.

In einer Ausführungsform sind die fakultativen Substituenten R³¹ an dem Rest eines aromatischen Rings, der R³ repräsentiert, beispielsweise an einer Phenylgruppe oder Pyridinylgruppe, die R³ repräsentiert, unter Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Cyano, (C₁-C₄)-Alkylcarbonyl, Aminosulfonyl, (C₁-C₄)-Alkylaminosulfonyl und Di((C₁-C₄)-alkyl)aminosulfonyl ausgewählt, in einer anderen Ausführungsform aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, Cyano, Aminosulfonyl, (C₁-C₄)-Alkylaminosulfonyl und Di((C₁-C₄)-alkyl)aminosulfonyl, in einer anderen Ausführungsform aus Halogen, (C₁-C₄)-Alkyl, (C₃-C₇)-Cycloalkyl, Hydroxy, (C₁-C₄)-Alkyloxy, (C₁-C₄)-Alkyl-S(O)ₘ-, Amino, (C₁-C₄)-Alkylamino, Di((C₁-C₄)-alkyl)amino, Cyano und Aminosulfonyl, in einer anderen Ausführungsform aus Halogen, (C₁-C₄)-Alkyl, (C₃-C₇)-Cycloalkyl, Hydroxy, (C₁-C₄)-Alkyloxy, Amino, (C₁-C₄)-Alkylamino, Di((C₁-C₄)-alkyl)amino, Cyano und Aminosulfonyl, in einer anderen Ausführungsformaus Halogen, (C₁-C₄)-Alkyl, (C₃-C₇)-Cycloalkyl, Hydroxy, (C₁-C₄)-Alkyloxy, Amino, (C₁-C₄)-Alkylamino und Di((C₁-C₄)-alkyl)amino, in einer anderen Ausführungsform aus Halogen, (C₁-C₄)-Alkyl, (C₃-C₇)-Cycloalkyl, (C₁-C₄)-Alkyloxy und Di((C₁-C₄)-alkyl)amino, in einer anderen Ausführungsform aus Halogen, (C₁-C₄)-Alkyl, (C₃-C₇)-Cycloalkyl, Hydroxy und (C₁-C₄)-Alkyloxy, in einer anderen Ausführungsform aus Halogen, (C₁-C₄)-Alkyl und (C₁-C₄)-Alkyloxy, in einer anderen Ausführungsform aus Fluor, Chlor, (C₁-C₄)-Alkyl, (C₃-C₇)-Cycloalkyl, Hydroxy und (C₁-C₄)-Alkyloxy, wobei in allen diesen Ausführungsformen alle Alkylgruppen unabhängig voneinander gegebenenfalls durch einen oder mehrere Fluorsubstituenten substituiert sind.

In einer Ausführungsform sind die fakultativen Substituenten R³¹ an dem Rest eines gesättigten oder nichtaromatischen ungesättigten Rings, der R³ repräsentiert, aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkyl-carbonylamino, (C₁-C₄)-Alkylsulfonylamino und Cyano ausgewählt, in einer anderen Ausführungsform aus Halogen, (C₁-C₄)-Alkyl, (C₃-C₇)-Cycloalkyl, Hydroxy, (C₁-C₄)-Alkyloxy, Oxo, Amino, (C₁-C₄)-Alkylamino, Di((C₁-C₄)-alkyl)amino und Cyano, in einer anderen Ausführungsform aus Halogen, (C₁-C₄)-Alkyl, (C₃-C₇)-Cycloalkyl, Hydroxy, (C₁-C₄)-Alkyloxy und Oxo, in einer anderen Ausführungsform aus Halogen, (C₁-C₄)-Alkyl, Hydroxy, (C₁-C₄)-Alkyloxy und Oxo, in einer anderen Ausführungsform aus Fluor, Chlor, (C₁-C₄)-Alkyl, Hydroxy, (C₁-C₄)-Alkyloxy und Oxo, in einer anderen Ausführungsform aus (C₁-C₄)-Alkyl, Hydroxy und Oxo, in einer anderen Ausführungsform aus Alkyl und Hydroxy, und in einer anderen Ausführungsform stehen sie für (C₁-C₄)-Alkyl, wobei in allen diesen Ausführungsformen alle Alkylgruppen unabhängig voneinander gegebenenfalls durch einen oder mehrere Fluorsubstituenten substituiert sind. Wenn der Rest eines Rings, der R³ repräsentiert, Oxogruppen als Substituenten R³¹ enthält, liegen in einer Ausführungsform nicht mehr als zwei derartige Oxosubstituenten vor, und in einer anderen Ausführungsform liegt nicht mehr als ein derartiger Oxosubstituent vor.

In einer Ausführungsform der Erfindung sind die Ringheteroatome in Het aus N und O ausgewählt, in einer anderen Ausführungsform aus O und S, in einer anderen Ausführungsform stehen sie für O-Atome. In einer anderen Ausführungsform ist die Zahl der Ringheteroatome in Het 1. In einer Ausführungsform liegen zwei Ringsauerstoffatome in Het nicht in benachbarten Ringpositionen vor, in einer anderen Ausführungsform liegen zwei aus O und S ausgewählte Ringheteroatome nicht in benachbarten Ringpositionen vor, in einer anderen Ausführungsform liegen zwei Ringheteroatome nicht in benachbarten Ringpositionen vor. Ringstickstoffatome in Het tragen ein Wasserstoffatom oder einen Substituenten wie angegeben. In einer Ausführungsform sind fakultative Substituenten an Ringstickstoffatomen in Het (C₁-C₄)-Alkylsubstituenten. In einer Ausführungsform sind fakultative Substituenten an Ringstickstoffatomen und Ringkohlenstoffatomen in Het (C₁-C₄)-Alkylsubstituenten. In einer Ausführungsform ist die Zahl fakultativer Substituenten an Het 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Het kann über ein beliebiges geeignetes Ringkohlenstoffatom gebunden sein. In einer Ausführungsform ist Het über ein Ringkohlenstoffatom gebunden, das nicht einem Ringheteroatom benachbart ist. Het kann 4-gliedrig, 5-gliedrig, 6-gliedrig oder 7-gliedrig sein. In einer Ausführungsform ist Het 4-gliedrig oder 5-gliedrig, in einer anderen Ausführungsform 5-gliedrig bis 7-gliedrig, in einer anderen Ausführungsform 5-gliedrig oder 6-gliedrig, in einer anderen Ausführungsform 4-gliedrig. Beispiele für Het, aus denen Het in einer Ausführungsform ausgewählt ist, sind Oxetanyl einschließlich Oxetan-2-yl und Oxetan-3-yl, Tetrahydrofuranyl einschließlich Tetrahydrofuran-2-yl und Tetrahydrofuran-3-yl, Tetrahydropyranyl einschließlich Tetrahydropyran-2-yl, Tetrahydropyran-3-yl und Tetrahydropyran-4-yl, Oxepanyl einschließlich Oxepan-2-yl, Oxepan-3-yl und Oxepan-4-yl, [1,3]Dioxolanyl einschließlich [1,3]Dioxolan-2-yl und [1,3]Dioxolan-4-yl, [1,4]Dioxanyl einschließlich [1,4]Dioxan-2-yl, Thietanyl einschließlich Thietan-2-yl und Thietan-3-yl, Tetrahydrothiophenyl einschließlich Tetrahydrothiophen-2-yl und Tetrahydrothiophen-3-yl, Tetrahydrothiopyranyl einschließlich Tetrahydrothiopyran-2-yl, Tetrahydrothiopyran-3-yl und Tetrahydrothiopyran-4-yl, [1,4]Dithianyl einschließlich [1,4]Dithian-2-yl, Azetidinyl einschließlich Azetidin-2-yl und Azetidin-3-yl, Pyrrolidinyl einschließlich Pyrrolidinyl-2-yl und Pyrrolidinyl-3-yl, Piperidinyl einschließlich Piperidinyl-2-yl, Piperidinyl-3-yl und Piperidinyl-4-yl, Azepanyl einschließlich Azepan-2-yl, Azepan-3-yl und Azepan-4-yl, Oxazolidinyl einschließlich Oxazolidin-2-yl, Oxazolidin-4-yl und Oxazolidin-5-yl, Thiazolidinyl einschließlich Thiazolidin-2-yl, Thiazolidin-4-yl und Thiazolidin-5-yl, Morpholinyl einschließlich Morpholin-2-yl und Morpholin-3-yl, Thiomorpholinyl einschließlich Thiomorpholin-2-yl und Thiomorpholin-3-yl, die alle gegebenenfalls wie in bezug auf Het angegeben substituiert sind.

Gegenstand der Erfindung sind alle Verbindungen der Formel I, worin ein oder mehrere Strukturelemente wie Gruppen, Substituenten und Zahlen wie in einer der angegebenen Ausführungsformen oder Definitionen der Elemente definiert sind oder eine oder mehrere der spezifischen Bedeutungen, die hier als Beispiele für Elemente angegeben sind, besitzen, wobei alle Kombinationen einer oder mehrerer angegebener Ausführungsformen und/oder Definitionen und/oder spezifischer Bedeutungen der Elemente Gegenstand der vorliegenden Erfindung sind. Auch in bezug auf alle derartigen Verbindungen der Formel I sind alle ihre stereoisomeren Formen und Mischungen von stereoisomeren Formen in beliebigem Verhältnis und ihre physiologisch annehmbaren Salze und die physiologisch annehmbaren Solvate davon Gegenstand der vorliegenden Erfindung.

Ein Beispiel für erfindungsgemäße Verbindungen, die in bezug auf beliebige Strukturelemente wie in den angegebenen Ausführungsformen der Erfindung oder Definitionen derartiger Elemente definiert sind und die Gegenstand der Erfindung sind, sind Verbindungen der Formel I, worin
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1 oder 2 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
und alle anderen Gruppen und Zahlen wie in der allgemeinen Definition der Verbindungen der Formel I oder einer der angegebenen Ausführungsformen der Erfindung oder Definitionen von Strukturelementen definiert sind.

Ein anderes derartiges Beispiel sind Verbindungen der Formel I in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes, worin A aus O und S ausgewählt ist;
X ausgewählt ist aus (C₁-C₆)-Alkandiyl, (C₂-C₆)-Alkandiyl und (C₁-C₆)-Alkandiyloxy;
R¹ ausgewählt ist aus (C₁-C₆)-Alkyl, (C₃-C₇)-Cycloalkyl-CₜH₂ₜ- und Het-CₜH₂ₜ-, worin t aus 0, 1 und 2 ausgewählt ist;
R² ausgewählt ist aus Phenylen und Pyridindiyl, worin das Phenylen und das Pyridindiyl gegebenenfalls an einem oder mehreren Ringstickstoffatomen durch gleiche oder verschiedene Substituenten R²² substituiert sind;
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1 oder 2 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Ein weiteres derartiges Beispiel sind Verbindungen der Formel I in einer beliebigen ihrer stereoisomeren Formen oder einer Mischung von stereoisomeren Formen in beliebigem Verhältnis und ihre physiologisch akzeptablen Salze und die physiologisch akzeptablen Solvate einer derartigen Verbindung oder eines derartigen Salzes, worin A für O steht;
X aus (C₁-C₆)-Alkandiyl und (C₁-C₆)-Alkandiyloxy ausgewählt ist;
R¹ aus (C₁-C₆)-Alkyl und (C₃-C₆)-Cycloalkyl-CₜH₂ₜ-, worin t aus 0 und 1 ausgewählt ist, ausgewählt ist;
R² für Phenylen steht, das gegebenenfalls an einem oder mehreren Ringkohlenstoffatomen durch gleiche oder verschiedene Substituenten R²² substituiert ist;
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 7-gliedrigen, monocyclischen oder bicyclischen Rings, der 0, 1 oder 2 gleiche oder verschiedene Ringheteroatome, die unter N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
R⁴ aus Wasserstoff und (C₁-C₄)-Alkyl ausgewählt ist;
R²² ausgewählt ist aus Halogen, Hydroxy, (C₁-C₄)-Alkyl- und (C₁-C₄)-Alkyloxy;
R³¹ ausgewählt ist aus Halogen, (C₁-C₄)-Alkyl, (C₃-C₇)-Cycloalkyl, Hydroxy und (C₁-C₄)-Alkyloxy;
Het für einen Rest eines gesättigten, 4-gliedrigen bis 6-gliedrigen, monocyclischen Heterocyclus, der 1 Ringheteroatom, das aus O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind; wobei alle Alkyl-, Alkandiyl-, CₜH₂ₜ-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

Ebenso gilt auch in bezug auf alle hier offenbarten spezifischen Verbindungen, wie die Beispielverbindungen, die Ausführungsformen der Erfindung repräsentieren, in denen die verschiedenen Gruppen und Zahlen in der allgemeinen Definition der Verbindungen der Formel I die in der jeweiligen spezifischen Verbindung vorliegenden spezifischen Bedeutungen besitzen, daß sie in einer beliebigen ihrer stereoisomeren Formen und/oder einer Mischung von stereoisomeren Formen in beliebigem Verhältnis und in Form ihrer physiologisch akzeptablen Salze und in Form der physiologisch akzeptablen Solvate derartiger Verbindungen oder derartiger Salze Gegenstand der vorliegenden Erfindung sind. Unabhängig davon, ob eine spezifische Verbindung hier als freie Verbindung und/oder als spezifisches Salz offenbart wird, ist sie sowohl in Form der freien Verbindung als auch in Form aller ihrer physiologisch akzeptablen Salze und bei Offenbarung eines spezifischen Salzes zusätzlich in Form dieses spezifischen Salzes und in Form der physiologisch akzeptablen Solvate einer derartigen Verbindung oder derartiger Salze Gegenstand der Erfindung. Gegenstand der Erfindung ist somit auch eine Verbindung der Formel I, die aus einer oder mehreren der hier offenbarten spezifischen Verbindungen der Formel I einschließlich der nachstehend angeführten Beispielverbindungen ausgewählt ist, und die physiologisch akzeptablen Salze davon und die physiologisch akzeptablen Solvate einer derartigen Verbindung oder derartiger Salze, wobei die Verbindung der Formel I in einer beliebigen ihrer stereoisomeren Formen oder als Mischung von stereoisomeren Formen in beliebigem Verhältnis, sofern anwendbar, Gegenstand der Erfindung ist. Als Beispiel genannt ist eine Verbindung der Formel I oder ein physiologisch akzeptables Solvat davon, die ausgewählt ist aus
{4-[5-(2,5-Difluorphenoxy)-7-propoxyoxazolo[5,4-d]pyrimidin-2-yl]-2,6-dimethylphenoxy}essigsäure,
[4-(5-Cyclopentyloxy-7-propoxyoxazolo[5,4-d]pyrimidin-2-yl)-2,6-dimethylphenoxy]essigsäure,
(4-[5-(trans-2-Fluorcyclohexyloxy)-7-propoxyoxazolo[5,4-d]pyrimidin-2-yl]-2,6-dimethylphenoxy}essigsäure,
{4-[5-(2-Fluorphenoxy)-7-propoxyoxazolo[5,4-d]pyrimidin-2-yl]-2,6-dimethylphenoxy}essigsäure,
{4-[5-(5-Fluor-2-methylphenoxy)-7-propoxyoxazolo[5,4-d]pyrimidin-2-yl]-2,6-dimethylphenoxy}essigsäure,
{4-[5-(3-Fluor-4-methylphenoxy)-7-propoxyoxazolo[5,4-d]pyrimidin-2-yl]-2,6-dimethylphenoxy}essigsäure,
{2,6-Dimethyl-4-[7-propoxy-5-(pyridin-3-yloxy)-oxazolo[5,4-d]pyrimidin-2-yl]phenoxy}essigsäure,
{4-[5-(2,4-Difluorphenoxy)-7-propoxyoxazolo[5,4-d]pyrimidin-2-yl]-2,6-dimethylphenoxy}essigsäure,
[2,6-Dimethyl-4-(5-phenoxy-7-propoxyoxazolo[5,4-d]pyrimidin-2-yl)-phenoxy]-essigsäure,
{4-[5-(3-Chlorphenoxy)-7-propoxyoxazolo[5,4-d]pyrimidin-2-yl]-2,6-dimethylphenoxy}-essigsäure,
[4-(5-Cyclohexylmethoxy-7-propoxyoxazolo[5,4-d]pyrimidin-2-yl)-2,6-dimethyl-phenoxy]essigsäure,
[4-(5-lsobutoxy-7-propoxyoxazolo [5,4-d]pyrim id in- 2-yl)-2, 6-dimethylphenoxy]-essigsäure,
[4-(5-Cyclobutylmethoxy-7-propoxyoxazolo[5,4-d]pyrimidin-2-yl)-2,6-dimethylphenoxy]essigsäure,
[4-(5-Cyclobutoxy-7-propoxyoxazolo[5,4-d]pyrimidin-2-yl)-2,6-dimethylphenoxy]-essigsäure,
[4-(5,7-Dipropoxyoxazolo[5,4-d]pyrimidin-2-yl)-2,6-dimethylphenoxy]essigsäure,
{2,6-Dimethyl-4-[7-propoxy-5-(3,3,3-trifluorpropoxy)oxazolo[5,4-d]pyrimidin-2-yl]-phenoxy}essigsäure,
[4-(5-Ethoxy-7-propoxyoxazolo[5,4-d]pyrimidin-2-yl)-2,6-dimethylphenoxy]essigsäure,
[4-(5-Cyclopentylmethoxy-7-propoxyoxazolo[5,4-d]pyrimidin-2-yl)-2,6-dimethylphenoxy]essigsäure,
{2,6-Dimethyl-4-[7-propoxy-5-(tetrahydrofuran-2-ylmethoxy)oxazolo[5,4-d]pyrimidin-2-yl]phenoxy}essigsäure,
[4-(5-sek.-Butoxy-7-propoxyoxazolo[5,4-d]pyrimidin-2-yl)-2,6-dimethylphenoxy]-essigsäure,
{2,6-Dimethyl-4-[7-propoxy-5-(3,3,3-trifluor-1-methylpropoxy)oxazolo[5,4-d]pyrimidin-2-yl]phenoxy}essigsäure,
{2,6-Dimethyl-4-[5-(3-methylbutoxy)-7-propoxyoxazolo[5,4-d]pyrimidin-2-yl]phenoxy}-essigsäure,
{4-[5-(2-Cyclopropylethoxy)-7-propoxyoxazolo[5,4-d]pyrimidin-2-yl]-2,6-dimethyl-phenoxy}essigsäure, .
{2,6-Dimethyl-4-[7-propoxy-5-(2,2,2-trifluor-1-methylethoxy)oxazolo[5,4-d]pyrim id in-2-yl]phenoxy}essigsäure,
{4-[5-(3-Fluorphenoxy)-7-propoxyoxazolo[5,4-d]pyrimidin-2-yl]-2,6-dimethylphenoxy}-essigsäure,
{2,6-Dimethyl-4-[7-propoxy-5-(3-trifluormethylphenoxy)oxazolo[5,4-d]pyrimidin-2-yl]-phenoxy}essigsäure,
[2,6-Dimethyl-4-(7-propoxy-5-{3-methylphenoxy}oxazolo[5,4-d]pyrimidin-2-yl)-phenoxy]essigsäure,
{4-[5-(3-Ethylphenoxy)-7-propoxyoxazolo[5,4-d]pyrimidin-2-yl]-2,6-dimethylphenoxy}-essigsäure,
{4-[5-(3-Chlor-4-fluorphenoxy)-7-propoxyoxazolo[5,4-d]pyrimidin-2-yl]-2,6-dimethyl-phenoxy}essigsäure,
{4-[5-(3-Chlor-4-methytphenoxy)-7-propoxyoxazolo[5,4-d]pyrimidin-2-yl]-2,6-dimethylphenoxy}essigsäure,
{4-[5-(5-Chlor-2-methylphenoxy)-7-propoxyoxazolo[5,4-d]pyrimidin-2-yl]-2,6-dimethylphenoxy}essigsäure,
{4-[5-(3-Chlor-2-methylphenoxy)-7-propoxyoxazolo[5,4-d]pyrimidin-2-yl]-2,6-dimethylphenoxy}essigsäure,
{4-[5-(3-Chlor-2-fluorphenoxy)-7-propoxyoxazolo[5,4-d]pyrimidin-2-yl]-2,6-dimethylphenoxy}essigsäure,
{4-[5-(5-Chlor-2-fluorphenoxy)-7-propoxyoxazolo[5,4-d]pyrimidin-2-yl]-2,6-dimethylphenoxy}essigsäure,
{4-[5-(3,4-Difluorphenoxy)-7-propoxyoxazolo[5,4-d]pyrimidin-2-yl]-2,6-dimethylphenoxy}essigsäure,
{4-[5-(4-Fluor-3-methylphenoxy)-7-propoxyoxazolo[5,4-d]pyrimidin-2-yl]-2,6-dimethylphenoxy}essigsäure,
{4-[5-(2,3-Difluorphenoxy)-7-propoxyoxazolo[5,4-d]pyrimidin-2-yl]-2,6-dimethylphenoxy}essigsäure,
{4-[5-(3,5-Difluorphenoxy)-7-propoxyoxazolo[5,4-d]pyrimidin-2-yl]-2,6-dimethylphenoxy}essigsäure,
{4-[5-(3-Chlor-5-fluorphenoxy)-7-propoxyoxazolo[5,4-d]pyrimidin-2-yl]-2,6-dimethylphenoxy}essigsäure,
{4-[5-(3-Fluor-5-trifluormethylphenoxy)-7-propoxyoxazolo[5,4-d]pyrimidin-2-yl]-2,6-dimethylphenoxy}essigsäure,
{4-[5-(3-Fluor-5-methylphenoxy)-7-propoxyoxazolo[5,4-d]pyrimidin-2-yl]-2,6-dimethylphenoxy}essigsäure,
{4-[5-(4-Fluorphenoxy)-7-propoxyoxazolo[5,4-d]pyrimidin-2-yl]-2,6-dimethylphenoxy}-essigsäure,
{4-[5-(2-Fluor-5-methylphenoxy)-7-propoxyoxazolo[5,4-d]pyrimidin-2-yl]-2,6-dimethylphenoxy}essigsäure,
{4-[5-(2-Chlor-5-fluorphenoxy)-7-propoxyoxazolo[5,4-d]pyrimidin-2-yl]-2,6-dimethylphenoxy}essigsäure,
{4-[5-(4-Chlor-3-fluorphenoxy)-7-propoxyoxazolo[5,4-d]pyrimidin-2-yl]-2,6-dimethylphenoxy}essigsäure,
[2,6-Dimethyl-4-(7-propoxy-5-{4-methylphenoxy}oxazolo[5,4-d]pyrimidin-2-yl)phenoxy]essigsäure,
[2,6-Dimethyl-4-(7-propoxy-5-{2-methylphenoxy}oxazolo[5,4-d]pyrimidin-2-yl)phenoxy]essigsäure,
{4-[5-(2-Chlorphenoxy)-7-propoxyoxazolo[5,4-d]pyrimidin-2-yl]-2,6-dimethylphenoxy}-essigsäure,
{4-[5-(2-Chlor-3-trifluormethylphenoxy)-7-propoxyoxazolo[5,4-d]pyrimidin-2-yl]-2,6-dimethylphenoxy}essigsäure,
{4-[5-(2-Chlor-5-trifluormethylphenoxy)-7-propoxyoxazolo[5,4-d]pyrimidin-2-yl]-2,6-dimethylphenoxy}essigsäure,
{4-[5-(4-Chlor-3-trifluormethylphenoxy)-7-propoxyoxazolo[5,4-d]pyrimidin-2-yl]-2,6-dimethylphenoxy}essigsäure,
{4-[5-(4-Fluor-3-trifluormethylphenoxy)-7-propoxyoxazolo[5,4-d]pyrimidin-2-yl]-2,6-dimethylphenoxy}essigsäure,
(4-[5-(2-Fluor-5-trifluormethylphenoxy)-7-propoxyoxazolo[5,4-d]pyrimidin-2-yl]-2,6-dimethylphenoxy}essigsäure,
{4-[5-(2-Fluor-3-trifluormethylphenoxy)-7-propoxyoxazolo[5,4-d]pyrimidin-2-yl]-2,6-dimethylphenoxy}essigsäure,
{2,6-Dimethyl-4-[7-propoxy-5-(2-trifluormethylphenoxy)oxazolo[5,4-d]pyrimidin-2-yl]-phenoxy}essigsäure,
{2,6-Dimethyl-4-[7-propoxy-5-(4-trifl uormethylphenoxy)oxazolo[5,4-d]pyrim id in-2-yl]-phenoxy}essigsäure,
{4-[5-(4-Chlor-2-fluorphenoxy)-7-propoxyoxazolo[5,4-d]pyrimidin-2-yl]-2,6-dimethylphenoxy}essigsäure,
{4-[5-(2-Chlor-4-fluorphenoxy)-7-propoxyoxazolo[5,4-d]pyrimidin-2-yl]-2,6-dimethylphenoxy}essigsäure,
{4-[5-(3-Methoxyphenoxy)-7-propoxyoxazolo[5,4-d]pyrimidin-2-yl]-2,6-dimethylphenoxy}essigsäure,
{2,6-Dimethyl-4-[7-propoxy-5-(3-trifluormethoxyphenoxy)oxazolo[5,4-d]pyrimidin-2-yl]phenoxy}essigsäure,
{2,6-Dimethyl-4-[7-propoxy-5-(3-trifluormethylsulfanylphenoxy)oxazolo[5,4-d]pyrimidin-2-yl]phenoxy}essigsäure,
{4-[5-(Indan-5-yloxy)-7-propoxyoxazolo[5,4-d]pyrimidin-2-yl]-2,6-dimethylphenoxy}-essigsäure,
{4-[5-(Indan-4-yloxy)-7-propoxyoxazolo[5,4-d]pyrimidin-2-yl]-2,6-dimethylphenoxy}-essigsäure,
{2,6-Dimethyl-4-[5-(naphthal in-2-yloxy)-7-propoxyoxazolo[5,4-d]pyrim id in-2-yl]-phenoxy}essigsäure,
{2,6-Dimethyl-4-[5-(2-methylbenzothiazol-5-yloxy)-7-propoxyoxazolo[5,4-d]pyrimidin-2-yl]phenoxy}essigsäure,
{4-[5-(Benzothiazol-6-yloxy)-7-propoxyoxazolo[5,4-d]pyrimidin-2-yl]-2,6-dimethyl-phenoxy}essigsäure,
{2,6-Dimethyl-4-[5-(6-methylpyridin-3-yloxy)-7-propoxyoxazolo[5,4-d]pyrimidin-2-yl]-phenoxy}essigsäure,
{2,6-Dimethyl-4-[5-(2-methylpyridin-3-yloxy)-7-propoxyoxazolo[5,4-d]pyrimidin-2-yl]-phenoxy}essigsäure,
{2,6-Dimethyl-4-[5-(5-methylpyridin-3-yloxy)-7-propoxyoxazolo[5,4-d]pyrimidin-2-yl]-phenoxy}essigsäure,
{4-[5-(5-Chlorpyridin-3-yloxy)-7-propoxyoxazolo[5,4-d]pyrimidin-2-yl]-2,6-dimethyl-phenoxy}essigsäure,
{4-[5-(5-Fluorpyridin-3-yloxy)-7-propoxyoxazolo[5,4-d]pyrimidin-2-yl]-2,6-dimethylphenoxy}essigsäure,
{2,6-Dimethyl-4-[7-propoxy-5-([1,2,5]thiadiazol-3-yloxy)oxazolo[5,4-d]pyrimidin-2-yl]-phenoxy}essigsäure,
{4-[5-(Isothiazol-3-yloxy)-7-propoxyoxazolo[5,4-d]pyrimidin-2-yl]-2,6-dimethylphenoxy}essigsäure,
{2,6-Dimethyl-4-[7-propoxy-5-(5-trifluormethylthiophen-3-yloxy)oxazolo[5,4-d]pyrimidin-2-yl]phenoxy}essigsäure,
{2,6.Dimethyl-4-[7-propoxy-5-(thiazol-2-ylsulfanyl)oxazolo[5,4-d]pyrimidin-2-yl]-phenoxy}essigsäure,
{2,6-Dimethyl-4-[5-(4-methylthiazol-2-ylsulfanyl)-7-propoxyoxazolo[5,4-d]pyrimidin-2-yl]phenoxy}essigsäure,
{4-[5-(1,1-Dioxotetrahydrothiophen-3-ylsulfanyl)-7-propoxyoxazolo[5,4-d]pyrimidin-2-yl]-2,6-dimethylphenoxy}essigsäure,
{4-[5-(2,5-Dimethylfuran-3-ylsulfanyl)-7-propoxyoxazolo[5,4-d]pyrimidin-2-yl]-2,6-dimethylphenoxy}essigsäure,
wobei eine Verbindung wie {4-[5-(trans-2-Fluorcyclohexyloxy)-7-propoxyoxazolo[5,4-d]pyrimidin-2-yl]-2,6-dimethylphenoxy}essigsäure, {2,6-Dimethyl-4-[7-propoxy-5-(tetrahydrofüran-2-ylmethoxy)oxazolo[5,4-d]pyrimidin-2-yl]phenoxy}essigsäure, [4-(5-sek.-Butoxy-7-propoxyoxazolo[5,4-d]pyrimidin-2-yl)-2,6-dimethylphenoxy]essigsäure, {2,6-Dimethyl-4-[7-propoxy-5-(3,3,3-trifluor-1-methylpropoxy)oxazolo[5,4-d]pyrimidin-2-yl]phenoxy}essigsäure-oder {2,6-Dimethyl-4-[7-propoxy-5-(2,2,2-trifluor-1-methyl-ethoxy)oxazolo[5,4-d]pyrimidin-2-yl]phenoxy}essigsäure, die in S-Konfiguration oder R-Konfiguration vorliegen kann, in S-Konfiguration oder R-Konfiguration oder einer Mischung der enantiomeren Formen in beliebigem Verhältnis Gegenstand der Erfindung ist.

Ein weiterer Gegenstand der vorliegenden Erfindung sind Verfahren zur Herstellung der Verbindungen der Formel I und ihrer Salze und Solvate, nach denen die Verbindungen erhältlich sind und die im folgenden umrissen werden. Bei einem Verfahren setzt man eine Verbindung der Formel II mit einer Verbindung der Formel III zu einer Verbindung der Formel I um, 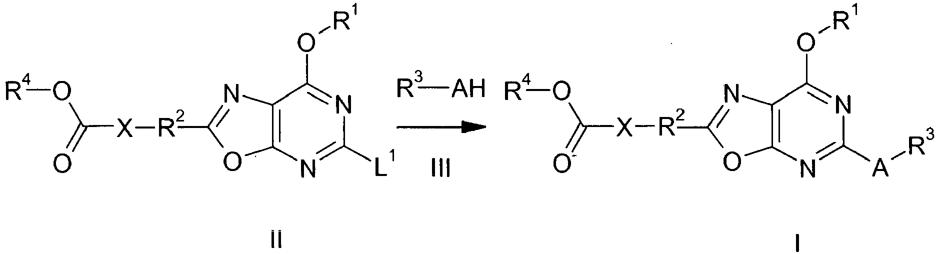 worin die Gruppen A, X, R¹, R², R³ und R⁴ in den Verbindungen der Formeln II und III wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppe L¹ in den Verbindungen der Formel II ist eine Abgangsgruppe, die in einer nukleophilen aromatischen Substitutionsreaktion ausgetauscht werden kann, wie ein Halogenatom, beispielsweise Chlor oder Brom, oder eine Sulfoxidgruppe oder eine Sulfongruppe, beispielsweise eine Gruppe der Formel -S(O)-Alk oder -S(O)₂-Alk, worin Alk eine (C₁-C₄)-Alkylgruppe, beispielsweise Methyl oder Ethyl, ist.

Die Reaktion der Verbindungen der Formeln II und III ist eine nukleophile aromatische Substitutionsreaktion an dem Kohlenstoffatom in der 5-Position des Oxazolo[5,4-d]pyrimidinrings, d.h. in der Pyrimidingruppierung, und kann unter Standardbedingungen für derartige Reaktionen, die dem Fachmann gut bekannt sind, durchgeführt werden. Im allgemeinen wird die Reaktion in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie Tetrahydrofuran (THF), Dioxan, Dibutylether, Diisopropylether oder 1,2-Dimethoxyethan (DME), einem Keton wie Aceton oder Butan-2-on, einem Ester wie Essigsäureethylester oder Essigsäurebutylester, einem Nitril wie Acetonitril, einem Amin wie N,N-Dimethylformamid (DMF) oder N-Methylpyrrolidin-2-on (NMP) oder einer Lösungsmittelmischung bei Temperaturen von etwa 20°C bis etwa 160°C, beispielsweise bei Temperaturen von etwa 40°C bis etwa 100°C, je nach den Besonderheiten des jeweiligen Falls, durchgeführt. Im allgemeinen ist es günstig, zur Erhöhung der Nukleophilie der Verbindung der Formel III eine Base zuzusetzen, beispielsweise ein tertiäres Amin, wie Triethylamin, Ethyldiisopropylamin oder N-Methylmorpholin, oder eine anorganische Base wie ein Erdalkalimetallhydrid, - hydroxid, -carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid. Eine Verbindung der Formel III kann auch vor der Reaktion mit der Verbindung der Formel II separat mit einer Base behandelt und in ein Salz umgewandelt werden.

Die Ausgangsverbindungen der Formeln II und III sind nach in der Literatur beschriebenen Verfahrensweisen oder in Anlehnung daran erhältlich und in vielen Fällen im Handel erhältlich. Verbindungen der Formel IIa, d.h. Verbindungen der Formel II, in denen L¹ beispielsweise für eine Sulfoxidgruppe der Formel Alk-S(O)-oder eine Sulfongruppe der Formel Alk-S(O)₂- steht, sind durch Umsetzung eines Aminomalonsäureesters der Formel IV mit einem aktivierten Carbonsäurederivat der Formel V zu einer Verbindung der Formel VI, Umsetzung der letzteren Verbindung mit Thioharnstoff der Formel VII zu einer Verbindung der Formel VIII, Alkylierung des Thiols mit einem Alkylierungsreagens der Formel IX zum Thioether der Formel X, Cyclisierung der letzteren Verbindung unter Bildung des Oxazolo[5,4-d]pyrimidinringsystems zur Verbindung der Formel XI, Alkylierung der letzteren Verbindung am Sauerstoffatom der Ketogruppe bzw. der tautomeren Hydroxygruppe mit einem Alkylierungsreagens der Formel XII, wobei der Rest R⁴O-C(O)-X- durch Umsetzung einer Verbindung der Formel XIV in die Verbindung der Formel III eingeführt wird, und Oxidation der Thioethergruppierung in der erhaltenen Verbindung der Formel XV zu dem entsprechenden Sulfoxid oder Sulfon der Formel IIa erhältlich.

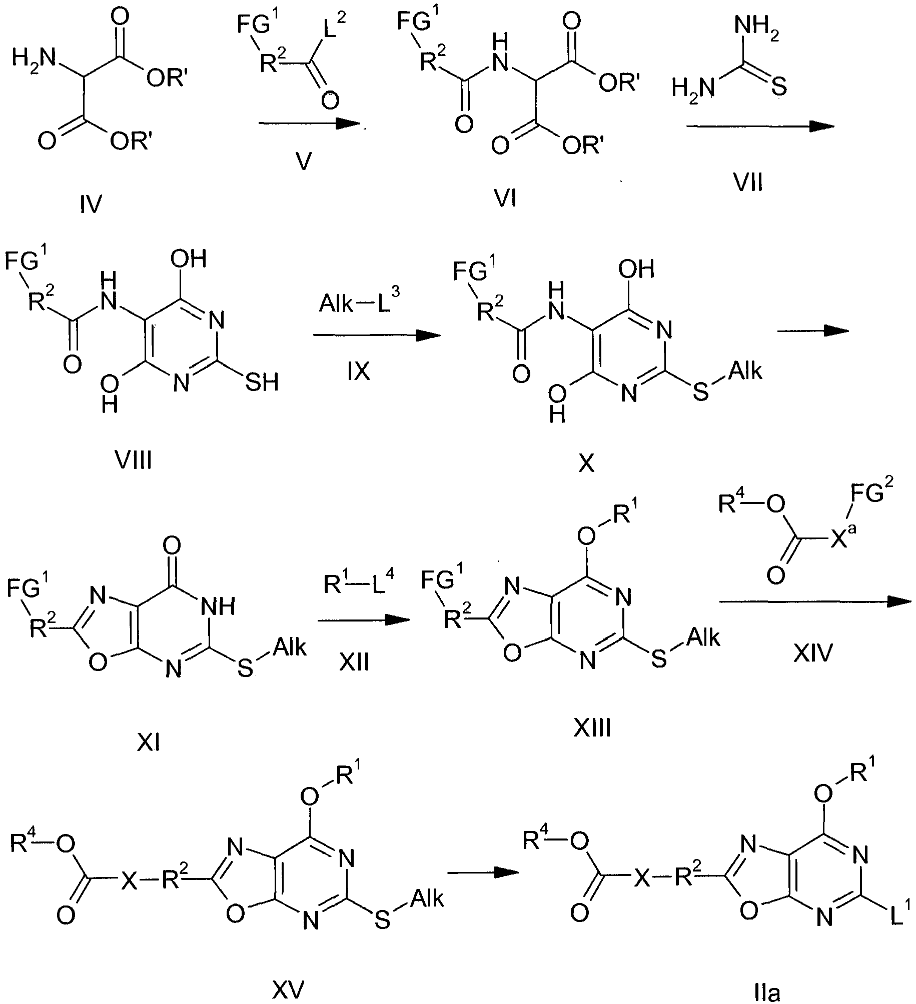

Die Gruppen X, R¹, R² und R⁴ in den Verbindungen der Formeln IIa, V, VI, VIII, X, XI, XII, XIII, XIV und XV sind wie in den Verbindungen der Formel I definiert, und zusätzlich können funktionelle Gruppen in geschützter Form oder in Form einer Vorläufergruppe, die später in die endgültige Gruppe umgewandelt wird, vorliegen. Die Gruppe X^{a} in den Verbindungen der Formel XIV ist wie die Gruppe X in den Verbindungen der Formel I definiert oder umfaßt einen Teil der Gruppe X in der gewünschten Verbindung der Formel II, so daß nach der Umsetzung der Verbindungen der Formeln XIII und XIV die Gruppe X^{a} und jegliche in der Verbindung der Formel XV verbleibende Teile der Gruppen FG¹ und FG² zusammen die gewünschte Gruppe X bilden. So kann beispielsweise in dem Fall, daß die Gruppe X für eine Alkandiyloxygruppe steht, die Gruppe X^{a} in der Verbindung der Formel XIV die gewünschte Alkandiyloxygruppe sein und die Gruppe FG² kann ein an das Sauerstoffatom gebundenes Wasserstoffatom sein, oder die Gruppe X^{a} kann der Alkandiylteil sein, die Gruppe FG² ist eine Abgangsgruppe, und die Gruppe FG¹ in der Verbindung der Formel XIII ist eine Hydroxygruppe, deren Sauerstoffatom zusammen mit dem Alkandiylteil dann nach Alkylierung der Verbindung der Formel XIII mit der Verbindung der Formel XIV die gewünschte Alkandiyloxygruppe bildet.

Die Gruppen FG¹ und FG² in den Verbindungen der Formeln V, VI, VIII, X, XI, XIII und XIV sind funktionelle Gruppen, die für den zur Bildung der gewünschten Gruppe X aus der Gruppe X^{a} und jeglichem in der Verbindung der Formel XV verbleibenden Teil der Gruppen FG¹ und FG² verwendeten Kupplungstyp geeignet sind. Beispielsweise kann es sich dann, wenn die Gruppe X^{a} über eine nukleophile Substitutionsreaktion an die Gruppe R² oder an ein Atom in der Gruppe FG¹, wie ein Sauerstoffatom in einer Hydroxygruppe, die FG¹ repräsentiert, wie oben erwähnt, gebunden wird, bei FG² um eine Abgangsgruppe wie ein Halogenatom wie Chlor, Brom oder Iod oder eine Sulfonyloxygruppe wie Methansulfonyloxy, Trifluormethansulfonyloxy oder Toluolsulfonyloxy handeln. Wenn die Gruppe X^{a} über eine übergangsmetallkatalysierte Reaktion an die Gruppe R² gebunden wird, kann es sich bei FG² um eine Abgangsgruppe wie eine Boronsäure-, Boronsäureester-, Dialkylboran- oder Stannangruppe handeln, und in diesem Fall kann FG¹ Halogen sein. Bei FG² kann es sich auch um ein Wasserstoffatom oder ein Kohlenstoffatom, das Teil einer Doppelbindung in einer Alkendiylgruppe, die X^{a} repräsentiert, handeln, wenn zur Verknüpfung von X^{a} mit R² eine Heck-Reaktion verwendet wird, und in diesem Fall kann FG¹ Halogen sein. Bei Verwendung einer Wittig-Reaktion oder Wittig-Horner-Reaktion zur Verknüpfung von X^{a} mit R² kann es sich bei FG² um eine Phosphoniogruppe wie Triphenylphosphonio oder eine Phosphonylgruppe wie Diethylphosphonyl handeln, und die Verbindung der Formel XIV kann ein Phosphoniumsalz oder ein Phosphonsäureester sein, und in diesem Fall kann FG¹ eine Aldehydgruppe -C(O)-H oder Ketongruppe -C(O)-Alkyl sein, und umgekehrt. Im Allgemeinen liegt die Gruppe FG¹ an dem Kohlenstoffatom in der Phenylengruppe oder heterocyclischen Gruppe, die R² repräsentiert, die in den Verbindungen der Formeln XV, IIa und I die Gruppe X trägt, vor. Die Gruppe FG¹ in den Verbindungen der Formeln V, VI, VIII, X und XI kann auch in geschützter Form oder in Form einer Vorläufergruppe, die später in die Gruppe umgewandelt wird, die in der Verbindung der Formel XIII mit der Verbindung der Formel XIV reagiert, vorliegen. So kann beispielsweise eine Hydroxygruppe, die in der Verbindung der Formel XIII FG¹ repräsentiert, in den Verbindungen der Formeln V, VI, VIII, X und XI in geschützter Form vorliegen, beispielsweise in Form einer veretherten Hydroxygruppe wie eines Benzylethers oder eines Alkylethers wie eines Methylethers. Derartige Ether können nach dem Fachmann gutbekannten Methoden gespalten werden. Eine Zusammenfassung von Methoden zur Abspaltung von Schutzgruppen findet sich in der Literatur, beispielsweise in P. J. Kocienski, Protecting Groups (Thieme Verlag, 1994), oder T. W. Greene und P. G. M. Wuts, Protective Groups in Organic Synthesis (John Wiley & Sons, 1999).

Die Gruppe L¹ in den Verbindungen der Formel IIa ist wie oben angegeben definiert. Die Gruppe L² in den Verbindungen der Formel V ist eine nukleophil substituierbare Abgangsgruppe und kann insbesondere ein Halogenatom, wie Chlor oder Brom, sein, und die Verbindung der Formel V kann somit ein Carbonsäurehalogenid sein. L² kann auch eine Gruppe der Formel FG¹-R²-C(O)-O sein, und die Verbindung der Formel V kann somit beispielsweise ein Carbonsäureanhydrid sein. Die Gruppen L³ und L⁴ sind Abgangsgruppen, die in einer nukleophilen Substitutionsreaktion ersetzt werden können, und können insbesondere ein Halogenatom wie Chlor, Brom oder Iod oder eine Sulfonyloxygruppe wie Methansulfonyloxy, Trifluormethansulfonyloxy oder Toluolsulfonyloxy sein, d.h. die Verbindungen der Formeln IX und XII können beispielsweise organische Halogenide oder Sulfonate sein. Die Gruppe R' in den Verbindungen der Formeln IV und VI kann Alkyl wie beispielsweise (C₁-C₃)-Alkyl, wie Methyl oder Ethyl, sein. Wie erwähnt, können die Verbindungen der Formel XI auch in einer anderen tautomeren Form vorliegen, beispielsweise in Form der jeweiligen 7-Hydroxyoxazolo[5,4-d]pyrimidinderivate, in denen das mobile Wasserstoffatom, das in Formel XI an das Ringstickstoffatom in der 6-Position des Oxazolopyrimidinringsystems gebunden ist, an das an das Ringkohlenstoffatom in der 7-Position gebundene Sauerstoffatom gebunden ist. Soweit anwendbar, gilt für alle Verbindungen, die bei der Herstellung der Verbindungen der Formel I vorkommen, daß sie in einer anderen tautomeren Form als der in ihren Formeln dargestellten vorliegen können. Bei den Reaktionen dieses Verfahrens zur Herstellung der Verbindungen der Formel II können wie bei allen anderen Reaktionen, die bei der Herstellung der Verbindungen der Formel I durchgeführt werden, Ausgangsverbindungen auch in Form eines Salzes eingesetzt und/oder Produkte in Form eines Salzes erhalten werden. So können beispielsweise Verbindungen der Formeln IV in Form eines Säureadditionssalzes wie des Hydrochlorids eingesetzt werden.

Die Umsetzung der Verbindungen der Formeln IV und V kann unter Standardbedingungen für die Acylierung eines Amins mit einem aktivierten Carbonsäurederivat wie einem Säurehalogenid oder-anhydrid durchgeführt werden. Im allgemeinen wird die Umsetzung in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Keton wie Aceton oder Butan-2-on, einem Ester wie Essigsäureethylester oder Essigsäurebutylester oder Wasser, oder einer Mischung von Lösungsmitteln, bei Temperaturen von etwa -10°C bis etwa 40°C, beispielsweise bei Temperaturen von etwa 0°C bis etwa 30°C durchgeführt. Im allgemeinen wird die Umsetzung unter Zugabe einer Base, beispielsweise eines tertiären Amins, wie Triethylamin, Ethyldiisopropylamin oder N-Methylmorpholin oder einer anorganischen Base wie eines Alkalimetallhydroxids, -carbonats oder-hydrogencarbonats wie Natriumhydroxid, Kaliumhydroxid, Natriumcarbonat, Kaliumcarbonat oder Natriumhydrogencarbonat durchgeführt. Die Umsetzung der Verbindungen der Formeln VI und VII wird im allgemeinen in einem inerten Lösungsmittel, beispielsweise einem Alkohol, wie Methanol, Ethanol oder Isopropanol, oder einem Ether wie THF, Dioxan oder DME oder einer Mischung von Lösungsmitteln bei Temperaturen von etwa 20°C bis etwa 80°C, beispielsweise Temperaturen von etwa 40°C bis etwa 80°C, in Gegenwart einer Base, beispielsweise eines Alkoxids wie Natriummethoxid, Natriumethoxid, Kaliummethoxid oder Kalium-tert.-butoxid, durchgeführt.

Die Umsetzung der Verbindungen der Formeln VIII und IX ist eine nukleophile Substitutionsreaktion am Kohlenstoffatom in der Gruppe Alk, die die Gruppe L³ trägt, und kann unter Standardbedingungen für derartige Umsetzungen, die dem Fachmann gut bekannt sind, durchgeführt werden. Im allgemeinen wird die Umsetzung je nach den Besonderheiten des jeweiigen Falls in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie DMF oder NMP, oder einer Mischung von Lösungsmitteln einschließlich zweiphasiger Mischungen mit wäßrigen Lösungen bei Temperaturen von etwa -20°C bis etwa 100°C, beispielsweise bei Temperaturen von etwa -10°C bis etwa 30°C durchgeführt. Im allgemeinen ist es günstig, zur Erhöhung der Nukleophilie der Verbindung der Formel VIII und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie ein Alkalimetallhydrid, -hydroxid, -carbonat oder-hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert-butoxid, Natriumamid oder Lithiumdiisopropylamid zuzusetzen. Eine Verbindung der Formel VIII kann auch vor der Umsetzung mit der Verbindung der Formel IX separat mit einer Base behandelt und in ein Salz umgewandelt werden.

Die Cyclisierung der Verbindung der Formel X zu der Verbindung der Formel XI kann günstigerweise in Gegenwart eines Phosphorhalogenids, wie Phosphorpentachlorid oder Phosphoroxidchlorid oder einer Mischung davon, in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan bei Temperaturen von etwa 20°C bis etwa 100°C, beispielsweise Temperaturen von etwa 50°C bis etwa 80°C, durchgeführt werden.

Die Umsetzung der Verbindungen der Formeln XI und XII ist eine weitere nukleophile Substitutionsreaktion an dem Kohlenstoffatom in der Gruppe R¹, das die Gruppe L⁴ trägt, und kann unter Standardbedingungen für derartige Umsetzungen, die dem Fachmann gut bekannt sind, durchgeführt werden. Im allgemeinen wird die Umsetzung je nach den Besonderheiten des jeweiligen Falls in einem inerten Lösungsmittel, beispielsweise einem Kohlenwasserstoff oder chloriertem Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie DMF oder NMP, oder einer Mischung von Lösungsmitteln bei Temperaturen von etwa 20°C bis etwa 100°C, beispielsweise bei Temperaturen von etwa 40°C bis etwa 80°C durchgeführt. Im allgemeinen ist es günstig, zur Erhöhung der Nukleophilie der Verbindung der Formel XI und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie ein Alkalimetallhydrid, -hydroxid, -carbonat oder-hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid zuzusetzen. Eine Verbindung der Formel XI kann noch vor der Umsetzung mit der Verbindung der Formel XII separat mit einer Base behandelt werden und in ein Salz umgewandelt werden. Eine Verbindung der Formel XI kann nicht nur durch Umsetzung mit einer Verbindung der Formel XII hergestellt werden, sondern auch durch Umsetzung mit dem jeweiligen Alkohol der Formel R¹-OH, worin R¹ wie in den Verbindungen der Formel I definiert ist und zusätzlich funktionelle Gruppen in geschützter Form oder in Form einer Vorläufergruppe vorliegen können, unter den Bedingungen der Mitsunobu-Reaktion in Gegenwart eines Azodicarboxylats wie Diethylazodicarboxylat oder Diisopropylazodicarboxylat und eines Phosphins wie Triphenylphosphin oder Tributylphosphin in einem inerten aprotischen Lösungsmittel, beispielsweise einem Ether wie THF oder Dioxan, in eine Verbindung der Formel XIII umgewandelt werden (siehe O. Mitsunobu, Synthesis (1981), 1-28).

Die Kupplung von Verbindungen der Formel XIII mit Verbindungen der Formel XIV kann durch Reaktionen verschiedener Typen durchgeführt werden, wie oben bereits angegeben, beispielsweise über eine Alkylierungsreaktion. So kann die Gruppe R² beispielsweise dann, wenn sie eine Hydroxygruppe, die FG¹ repräsentiert, trägt, unter Verwendung einer Verbindung der Formel XIV, in der FG² für eine für nukleophile Substitutionsreaktionen geeignete Abgangsgruppe wie ein Halogenatom wie Chlor, Brom oder Iod oder eine Sulfonyloxygruppe wie Methansulfonyloxy oder Toluolsulfonyloxy steht, alkyliert werden. Die nukleophile Substitutionsreaktion an dem Kohlenstoffatom der Gruppe XIV, die die Gruppe FG² trägt, kann unter Standardbedingungen für derartige Reaktionen, die dem Fachmann gut bekannt sind, durchgeführt werden. Im Allgemeinen wird die Umsetzung je nach den Besonderheiten des jeweiligen Falls in einem inerten Lösungsmittel, beispielsweise einem Kohlenwasserstoff oder chlorierten Wasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie N,N-Dimethylformamid oder N-Methylpyrrolidin-2-on, oder einer Mischung von Lösungsmitteln bei Temperaturen von etwa 20°C bis etwa 100°C, beispielsweise bei Temperaturen von etwa 40°C bis etwa 80°C durchgeführt. Im Allgemeinen ist es günstig, zur Erhöhung der Nukleophilie der Verbindung der Formel XIII und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie Alkalimetallhydrid, -hydroxid, - carbonat oder -hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid zuzusetzen. Eine Verbindung der Formel XIII, worin FG¹ für Hydroxy steht, kann auch vor der Umsetzung mit der Verbindung der Formel XIV separat mit einer Base behandelt und in ein Salz umgewandelt werden. Eine Verbindung der Formel XIII, worin FG¹ für Hydroxy steht, kann nicht nur durch Umsetzung mit einer Verbindung der Formel XIV, worin FG² für eine Abgangsgruppe wie angegeben steht, in eine Verbindung der Formel XV umgewandelt werden, sondern auch durch Umsetzung mit dem entsprechenden Alkohol, d.h. mit einer Verbindung der Formel XIV, worin FG² für Hydroxy steht, unter den oben angegebenen Bedingungen der Mitsunobu-Reaktion. Die Kupplung von Verbindungen der Formel XIII mit Verbindungen der Formel XIV über eine übergangsmetallkatalysierte Reaktion kann auch unter den Bedingungen von palladiumkatalysierten Kreuzkupplungsreaktionen wie der Heck-, Stille- oder Suzuki-Kupplungsreaktion durchgeführt werden (siehe A. de Meijere und F. Diederich (Hrsg.), Metal-Catalyzed Cross-Coupling Reactions (Wiley-VCH, 2004)).

Die Oxidation der Alk-S-Gruppe in den Verbindungen der Formel XV zur Sulfoxidgruppe oder Sulfongruppe in den Verbindungen der Formel II kann mit Hilfe von Wasserstoffperoxid oder einer Persäure wie 3-Chlorperbenzoesäure oder Monoperoxyphthalsäure in einem inerten Lösungsmittel, beispielsweise einem chlorierten Kohlenwasserstoff wie Dichlormethan oder Chloroform oder einem Ester wie Essigsäureethylester oder Essigsäurebutylester, bei Temperaturen von etwa 0°C bis etwa 40°C, beispielsweise bei etwa 20°C, durchgeführt werden.

Man kann auch die Reihenfolge der Schritte bei der Herstellung der Verbindungen der Formel X ändern und zunächst einen Aminomalonsäureester der Formel IV wie den Diethylester in Gegenwart eines Alkalimetallalkoxids wie Natriumethoxid mit Thioharnstoff umsetzen, dann das Schwefelatom alkylieren, beispielsweise mit lodmethan methylieren, und das erhaltene Produkt mit einer Verbindung der Formel V acylieren (siehe M. H. Holschbach et al., Eur. J. Med. Chem. 41 (2006), 7-15). Weitere Verbindungen der Formel I sind aus geeigneten, nach den obenbeschriebenen Verfahren hergestellten Verbindungen durch Funktionalisierung oder Modifizierung von enthaltenen funktionellen Gruppen nach Standardverfahrensweisen erhältlich, beispielsweise durch Veresterung, Amidierung, Hydrolyse, Veretherung, Alkylierung, Acylierung, Sulfonylierung, Reduktion, Oxidation, Umwandlung in Salze u.a. So kann beispielsweise eine Hydroxygruppe, die aus einer Ethergruppe durch Etherspaltung, beispielsweise mit Hilfe von Bortribromid, oder aus einer geschützten Hydroxygruppe durch Entschützung freigesetzt werden kann, zu einem Carbonsäureester oder einem Sulfonsäureester verestert oder verethert werden. Veretherungen von Hydroxygruppen können günstigerweise durch Alkylierung mit der jeweiligen Halogenverbindung, beispielsweise einem Bromid oder Iodid, in Gegenwart einer Base, beispielsweise eines Alkalimetallcarbonats wie Kaliumcarbonat oder Cäsiumcarbonat, in einem inerten Lösungsmittel, beispielsweise einem Amid wie DMF oder NMP oder einem Keton wie Aceton oder Butan-2-on oder mit dem jeweiligen Alkohol unter den oben angesprochenen Bedingungen der Mitsunobu-Reaktion durchgeführt werden. Eine Hydroxygruppe kann durch Behandlung mit einem Halogenierungsmittel in ein Halogenid umgewandelt werden. Ein Halogenatom kann in einer Substitutionsreaktion, bei der es sich auch um eine übergangsmetallkatalysierte Reaktion handeln kann, durch verschiedene Gruppen ersetzt werden. Eine Nitrogruppe kann zu einer Aminogruppe reduziert werden, beispielsweise durch katalytische Hydrierung. Eine Aminogruppe kann unter Standardbedingungen für die Alkylierung, beispielsweise durch Umsetzung mit einer Halogenverbindung oder durch reduktive Aminierung einer Carbonylverbindung, oder für die Acylierung oder Sulfonylierung, beispielsweise durch Umsetzung mit einem reaktiven Carbonsäurederivat wie einem Säurechlorid oder Anhydrid oder einem Sulfonsäurechlorid oder mit einer aktivierten Carbonsäure, die aus der Carbonsäure beispielsweise durch Behandlung mit einem Kupplungsmittel wie N,N'-Carbonyldiimidazol (CDI), einem Carbodiimid wie 1,3-Dicyclohexylcarbodiimid (DCC) oder 1-(3-Dimethylaminopropyl)-3-ethylcarbodiimid-hydrochlorid (EDC), O-(7-Azabenzotriazol-1-yl)-N,N,N',N'-tetramethyluroniumhexafluorophosphat (HATU), O-(Cyano(ethoxycarbonyl)methylenamino)-N,N,N',N'-tetramethyluroniumtetrafluoroborat (TOTU) oder [(Benzotriazol-1-yloxy)dimethylaminomethylen]dimethylammoniumtetrafluoroborat (TBTU) erhältlich ist, modifiziert werden. Eine Carbonsäureestergruppe kann unter sauren oder basischen Bedingungen zu einer Carbonsäure hydrolysiert werden. Eine Carbonsäuregruppe kann wie oben erwähnt aktiviert oder in ein reaktives Derivat umgewandelt und mit einem Alkohol oder einem Amin oder Ammoniak zu einem Ester oder Amid umgesetzt werden. Ein primäres Amid kann zu einem Nitril dehydratisiert werden. Ein Schwefelatom, beispielsweise in einer Alkyl-S-Gruppe oder in einem heterocyclischen Ring, kann mit einem Peroxid wie Wasserstoffperoxid oder einer Persäure zu einer Sulfoxidgruppierung S(O) oder einer Sulfongruppierung S(O)₂ oxidiert werden. Eine Carbonsäuregruppe, eine Carbonsäureestergruppe und eine Ketongruppe können zu einem Alkohol reduziert werden, beispielsweise mit Hilfe eines komplexen Hydrids wie Lithiumaluminiumhydrid, Lithiumborhydrid oder Natriumborhydrid. Eine Verbindung der Formel I oder ein Zwischenprodukt wie eine Verbindung der Formel II, die bzw. das eine Doppelbindung oder eine Dreifachbindung in der Gruppe X enthält, die über eine übergangsmetallkatalysierte Kupplungsreaktion leicht aus einer Verbindung der Formel XIV mit einer Doppel- oder Dreifachbindung in der Gruppe X^{a} und einer Verbindung der Formel XIII wie oben beschrieben erhältlich ist, kann durch Hydrierung in Gegenwart von Hydrierkatalysator wie einem Palladiumkatalysator in eine Verbindung überführt werden, in der X für eine gesättigte Gruppe steht.

Alle bei den oben beschriebenen Synthesen der Verbindungen der Formel I verwendeten Reaktionen sind dem Fachmann an sich gut bekannt und können unter Standardbedingungen gemäß oder in Analogie zu in der Literatur, beispielsweise in Houben-Weyl, Methoden der Organischen Chemie (Methods of Organic Chemistry), Thieme-Verlag, Stuttgart, oder Organic Reactions, John Wiley & Sons, New York, beschriebenen Verfahrensweisen durchgeführt werden. Falls gewünscht, können die erhaltenen Verbindungen der Formel I sowie etwaige Zwischenverbindungen nach herkömmlichen Reinigungsverfahrensweisen gereinigt werden, beispielsweise durch Umkristallisieren oder Chromatographie. Wie bereits erwähnt, können alle bei den oben beschriebenen Synthesen eingesetzten Ausgangsverbindungen und Zwischenprodukte, die eine saure oder basische Gruppe enthalten, auch in Form von Salzen eingesetzt werden und alle Zwischenprodukte und entgültigen Zielverbindungen können auch in Form von Salzen erhalten werden. Wie ebenfalls oben erwähnt, kann es je nach den Umständen des jeweiligen Falls zur Vermeidung eines unerwünschten Verlaufs einer Reaktion oder von Nebenreaktionen im Lauf der Synthese einer Verbindung im allgemeinen erforderlich oder vorteilhaft sein, funktionelle Gruppen durch die Einführung von Schutzgruppen zeitweilig zu blockieren und sie in einer späteren Stufe der Synthese wieder zu entschützen oder funktionelle Gruppen in Form von Vorläufergruppen, die später in die gewünschten funktionellen Gruppen umgewandelt werden, einzuführen. Als Beispiele für Schutzgruppen seien Aminoschutzgruppen genannt, bei denen es sich um Acylgruppen oder Alkyloxycarbonylgruppen, beispielsweise eine tert.-Butyloxycarbonylgruppe (=Boc), die durch Behandung mit Trifluoressigsäure (=TFA) abgespalten werden kann, eine Benzyloxycarbonylgruppe, die durch katalytische Hydrierung abgespalten werden kann, oder eine Fluoren-9-ylmethoxycarbonylgruppe,die durch Behandlung mit Piperidin abgespalten werden kann, handeln kann, und Schutzgruppen von Carbonsäuregruppen, die als Estergruppen, wie tert.-Butylester, die durch Behandlung mit Trifluoressigsäure entschützt werden können, oder Benzylester, die durch katalytische Hydrierung entschützt werden können, geschützt werden können. Als Beispiel für eine Vorläufergruppe sei die Nitrogruppe genannt, die durch Reduktion, beispielsweise durch katalytische Hydrierung, in eine Aminogruppe umgewandelt werden kann. Derartige Synthesestrategien und Schutzgruppen und Vorläufergruppen, die in einem bestimmten Fall geeignet sind, sind dem Fachmann bekannt.

Ein weiterer Gegenstand der vorliegenden Erfindung sind die neuen Ausgangsverbindungen und Zwischenprodukte, die bei der Synthese der Verbindungen der Formel I vorkommen, einschließlich der Verbindungen der Formeln II, IIa, III, IV, V, VI, VIII, X, XI, XII, XIII, XIV und XV, worin A, X, X^{a}, R¹, R², R³, R⁴, R', Alk, FG¹, FG², L¹, L² und L⁴ wie oben definiert sind, in einer beliebigen ihrer stereoisomeren Formen oder einer Mischung von stereoisomeren Formen in beliebigem Verhältnis und ihre Salze und Solvate derartiger Verbindungen oder derartiger Salze und ihre Verwendung als Zwischenprodukte. Die Erfindung schließt auch alle tautomeren Formen der Zwischenprodukte und Ausgangsverbindungen ein. Alle oben bezüglich der Verbindungen der Formel I angegebenen Erklärungen und Ausführungsformen gelten entsprechend auch für die Zwischenprodukte und Ausgangsverbindungen. Gegenstand der Erfindung sind insbesondere die hier offenbarten neuen spezifischen Ausgangsverbindungen und Zwischenprodukte. Unabhängig davon, ob sie als freie Verbindung und/oder als spezifisches Salz offenbart sind, sind sie sowohl in Form der freien Verbindungen als auch in Form ihrer Salze und im Fall der Offenbarung eines spezifischen Salzes zusätzlich in Form dieses spezifischen Salzes und in Form von Solvaten derartiger Verbindungen oder derartiger Salze Gegenstand der Erfindung.

Die Verbindungen der Formel I, gegebenenfalls in Kombination mit anderen pharmakologisch wirksamen Verbindungen, können Tieren, vorzugsweise Säugetieren einschließlich Menschen, als Pharmazeutika für sich alleine, in Mischungen miteinander oder in Form pharmazeutischer Zusammensetzungen verabreicht werden. Die Verabreichung kann oral, beispielsweise in Form von Tabletten, Filmtabletten, Dragees, Granulaten, Hart- und Weichgelatinekapseln, Lösungen-einschließlich wäßriger, alkoholischer und öliger Lösungen, Säften, Tropfen, Sirupen, Emulsionen oder Suspensionen, rektal, beispielsweise in Form von Suppositorien, oder parenteral, beispielsweise in Form von Lösungen zur subkutanen, intramuskulären oder intravenösen Injektion oder Infusion, insbesondere wäßrigen Lösungen, durchgeführt werden. Die Verbindungen der Formel I können des weiteren in Modi der lokalen Arzneistoffzufuhr verwendet werden, beispielsweise in beschichteten Stents zur Verhinderung oder Verringerung der In-Stent-Restenose oder durch lokale Anwendung mit Hilfe eines Katheters. Die geeignete Verabreichungsform hängt u.a. von der zu behandelnden Erkrankung und ihrer Schwere ab.

Die Menge einer Verbindung der Formel I und/oder ihrer physiologisch akzeptablen Salze und/oder Solvate in den pharmazeutischen Zusammensetzungen liegt normalerweise im Bereich von etwa 0,2 bis etwa 800 mg, beispielsweise von etwa 0,5 bis etwa 500 mg, beispielsweise von etwa 1 bis etwa 200 mg, pro Einheitsdosis, kann aber je nach Art der pharmazeutischen Zusammensetzung auch höher sein. Die pharmazeutischen Zusammensetzungen enthalten in der Regel etwa 0,5 bis etwa 90 Gew.-% der Verbindung der Formel I und/oder ihrer physiologisch akzeptablen Salze und/oder Solvate. Die pharmazeutischen Zusammensetzungen können auf an sich bekannte Art und Weise hergestellt werden. Hierzu bringt man eine oder mehrere Verbindungen der Formel I und/oder ihre physiologisch akzeptablen Salze und/oder Solvate zusammen mit einer oder mehreren festen oder flüssigen pharmazeutischen Trägersubstanzen oder Vehikeln und/oder Additiven oder Hilfssubstanzen und dann, wenn ein Kombinationsmedikament gewünscht ist, anderen pharmakologisch wirksamen Verbindungen mit therapeutischer oder prophylaktischer Wirkung in eine für die Verabreichung und Dosierung geeignete Form, die dann in der Human- oder Tiermedizin verwendet werden kann. Als Trägersubstanzen und Additive können geeignete organische und anorganische Substanzen verwendet werden, die mit den Verbindungen der Formel I oder ihren physiologisch akzeptablen Salzen oder Solvaten nicht in unerwünschter Weise reagieren. Als Beispiele für Additivtypen, die in den pharmazeutischen Zusammensetzungen und Medikamenten enthalten sein können, seien Gleitmittel, Konservierungsstoffe, Verdicker, Stabilisatoren, Sprengmittel, Netzmittel, Mittel zur Erzielung eines Depoteffekts, Emulgatoren, Salze, beispielsweise zur Beeinflussung des osmotischen Drucks, Puffersubstanzen, Farbmittel, Geschmacksstoffe und aromatische Substanzen genannt. Beispiele für Trägersubstanzen und Additive sind Wasser, physiologische Natriumchloridlösung, Pflanzenöle, Wachse, Alkohole wie Ethanol, Isopropanol, 1,2-Propandiol, Benzylalkohole oder Glycerin, Polyole, Mannit, Polyethylenglykole, Polypropylenglykole, Glycerintriacetat, Polyvinylpyrrolidon, Gelatine, Cellulose, Kohlenhydrate wie Lactose, Glucose, Saccharose oder Stärke wie Maisstärke, Stearinsäure und Stearinsäuresalze wie Magnesiumstearat, Talk, Lanolin, Vaseline oder Mischungen davon, beispielsweise Mischungen von Wasser mit einem oder mehreren organischen Lösungsmitteln wie Mischungen von Wasser mit Alkoholen. Man kann die Verbindungen der Formel I und ihre physiologisch akzeptablen Salze und Solvate auch lyophilisieren und die erhaltenen Lyophilisate beispielsweise zur Herstellung von Injektionszusammensetzungen verwenden.

Die Dosierung einer zu verabreichenden Verbindung der Formel I und/oder eines physiologisch akzeptablen Salzes und/oder Solvats davon hängt vom Einzelfall ab und ist wie üblich zur Erzielung einer optimalen Wirkung vom Arzt nach den üblichen Regeln und Verfahrensweisen den individuellen Gegebenheiten anzupassen. So hängt sie beispielsweise ab von der Art und Schwere der zu behandelnden Störung, von Geschlecht, Alter, Gewicht und individueller Ansprechbarkeit des zu behandelnden Menschen oder Tiers, von der Effizienz und Wirkdauer der verwendeten Verbindung, davon, ob die Behandlung für die Therapie einer akuten oder chronischen Erkrankung oder prophylaktisch ist, oder davon, ob neben einer Verbindung der Formel I weitere Wirkstoffe verabreicht werden. Im allgemeinen ist eine Tagesdosis von beispielsweise etwa 0,01 mg/kg bis etwa 100 mg/kg oder von etwa 0,1 mg/kg bis etwa 10 mg/kg oder von etwa 0,3 mg/kg bis etwa 5 mg/kg (jeweils mg pro kg Körpergewicht) zur Verabreichung an einen 75 kg schweren Erwachsenen zur Erzielung der gewünschten Ergebnisse angemessen. Die Tagesdosis kann dabei als Einzeldosis verabreicht oder, insbesondere bei Verabreichung größerer Mengen, in mehrere, beispielsweise zwei, drei oder vier, Einzeldosen aufgeteilt werden. Die Verabreichung kann auch kontinuierlich durchgeführt werden, beispielsweise durch kontinuierliche Infusion oder Injektion. Im Einzelfall kann es je nach dem individuellen Verhalten erforderlich sein, von den angegebenen Dosierungen nach oben oder nach unten abzuweichen.

Die folgenden Beispiele erläutern die Erfindung.

Wenn Beispielsverbindungen mit einer basischen Gruppe durch präparative Hochdruck-Flüssigkeitschromatographie (HPLC) an Umkehrphasen-Säulenmaterial (RP-Säulenmaterial) gereinigt wurden und es sich bei dem Elutionsmittel wie üblich um eine Gradientenmischung von Wasser und Acetonitril mit Trifluoressigsäure (TFA) handelte, wurden sie je nach den Einzelheiten der Aufarbeitung wie Verdampfungs-oder Lyophilisierungsbedingungen zum Teil in Form ihres Säureadditionssalzes mit Trifluoressigsäure erhalten. In den Namen der Beispielverbindungen und ihren Strukturformeln ist jegliche derartige enthaltene Trifluoressigsäure nicht angegeben.

Die hergestellten Verbindungen wurden im allgemeinen durch spektroskopische Daten und chromatografische Daten, insbesondere Massenspektrum (MS) und HPLC-Retentionszeiten (Rt; in min), die durch kombinierte analytische HPLC/MS-Charakterisierung (LC/MS) erhalten wurden, und/oder NMR-Spektren (NMR=kernmagnetische Resonanz), charakterisiert. Bei der NMR-Charakterisierung sind die chemische Verschiebung δ (in ppm), die Zahl der Wasserstoffatome und die Multiplizität (s = Singulett, d = Dublett, dd = doppeltes Dublett, t = Triplett, dt = doppeltes Triplett, q = Quartett, m = Multiplett; br = breit) der Signale angegeben. Bei der MS-Charakterisierung ist im allgemeinen die Massenzahl (m/z) des Peaks des Molekülions M, z.B. M⁺, oder eines verwandten Ions wie des Ions M+1, z.B. [M+1]⁺, d.h. des protonierten Molekülions [M+H]⁺, das je nach der verwendeten Ionisierungsmethode gebildet wurde, angegeben. Bei der Ionisierungsmethode handelte es sich im allgemeinen um Elektrospray-Ionisierung (ESI). Es wurden die folgenden LC/MS-Bedingungen verwendet.

### Methode LC1

Säule: UPLC BEH C18, 50 x 2,1 mm, 1,7 µm; Durchfluß: 0,9 ml/min; Elutionsmittel A: Acetonitril + 0,08 % Ameisensäure; Elutionsmittel B: Wasser + 0,1% Ameisensäure; Gradient: von 5% A + 95% B bis 95% A + 5% B in 1,1 min, dann 95% A + 5% B für 0,6 min; MS-Ionisationsmethode: ESI⁺

### Methode LC2

Säule: UPLC BEH C18, 50 x 2,1 mm, 1,7 µm; Durchfluß: 0,9 ml/min; Elutionsmittel A: Acetonitril + 0,035 % Ameisensäure; Elutionsmittel B: Wasser + 0,05% Ameisensäure; Gradient: von 5% A + 95% B bis 95% A + 5% B in 1,1 min, dann 95% A + 5% B für 0,6 min; MS-Ionisationsmethode: ESI⁺

### Methode LC3

Säule: Waters Xbridge C18, 50 x 4,6 mm, 2,5 µm; Durchfluß: 1,3 ml/min; Elutionsmittel A: Acetonitril + 0,1 % Ameisensäure; Elutionsmittel B: Wasser + 0,1% Ameisensäure; Gradient: von 3% A + 97% B bis 60% A + 40% B in 3,5 min, von 60% A + 40% B bis 98% A + 2% B in 0,5 min, dann bis 98% A + 2% B frü 1 min; MS-Ionisationsmethode: ESI⁺

### Beispiel 1

### {4-[5-(2,5-Difluorphenoxy)-7-propoxyoxazolo[5,4-d]pyrimidin-2-yl]-2,6-dimethyl-phenoxy}essigsäure

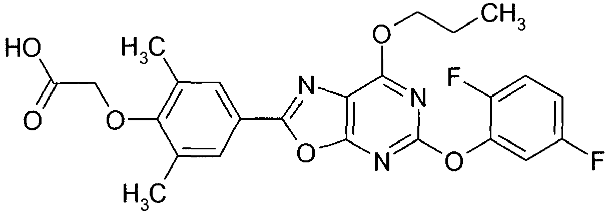

(a) 2-(4-Methoxy-3,5-dimethylbenzoylamino)malonsäurediethylester
   116,8 g Aminomalonsäurediethylester-hydrochlorid wurden in 700 ml Dichlormethan gelöst und unter Kühlung in einem Eisbad mit 231 ml Triethylamin versetzt. Dann wurde langsam eine Lösung von 109,6 g 4-Methoxy-3,5-dimethyl-benzoylchlorid in 400 ml Dichlormethan zugetropft. Nach 2 h bei 0°C wurden langsam 200 ml Wasser zugegeben. Nach Phasentrennung wurde die wässrige Phase zweimal mit 200 ml Dichlormethan extrahiert. Die vereinigten organischen Phasen wurden mit 2 M Salzsäure und danach mit Wasser gewaschen, mit Natriumsulfat getrocknet, filtriert und eingedampft. Der Rückstand wurde mit Methyl-tert.-butylether behandelt, wonach der erhaltene Niederschlag abfiltriert wurde, was 178,7 g der Titelverbindung ergab.
(b) Natrium-4,6-dihydroxy-5-(4-methoxy-3,5-dimethylbenzoylamino)pyrimidin-2-thiolat
   20,6 g Thioharnstoff in 900 ml absolutem Ethanol wurden mit 1,5 Äquivalenten Natriummethoxid (30%ige Lösung im Methanol) versetzt. Nach Zugabe von 91 g 2-(4-Methoxy-3,5-dimethylbenzoylamino)malonsäurediethylester in kleinen Portionen wurde die Mischung 3 h bei 60°C gerührt. Dann wurde die Mischung auf Raumtemperatur abgekühlt und der Niederschlag abgesaugt, mit 100 ml Ethanol und 100 ml Diethylether gewaschen und im Vakuum getrocknet. Es wurden 78,2 g der rohen Titelverbindung erhalten.
(c) N-(4,6-Dihydroxy-2-methylsulfanylpyrimidin-5-yl)-4-methoxy-3,5-dimethyl-benzamid
   19,1 g Natrium-4,6-dihydroxy-5-(4-methoxy-3,5-dimethylbenzoylamino)pyrimidin-2-thiolat in 190 ml Wasser und 80 ml N-Methylpyrrolidin-2-on wurden auf 0°C abgekühlt. Unter Kühlung wurden 5,9 g Natriumhydroxid zugegeben, wonach die Mischung 30 min bei 0°C gerührt wurde. Dann wurde eine Lösung von 3,7 ml lodmethan in 4,3 ml N-Methylpyrrolidin-2-on zugegeben. Nach Abschluss der Reaktion (2 h) wurde die Mischung mit konzentrierter Salzsäure angesäuert. Der erhaltene Niederschlag wurde abgesaugt, mit Wasser gewaschen und im Vakuum getrocknet. Es wurden 10,1 g der Titelverbindung erhalten.
   LC/MS (Methode LC1): Rt = 1,03 min; m/z = 336,1 [M+H]⁺
(d) 2-(4-Methoxy-3,5-dimethylphenyl)-5-methylsulfanyloxazolo[5,4-d]pyrimidin-7-ol
   10,1 g N-(4,6-Dihydroxy-2-methylsulfanylpyrimidin-5-yl)-4-methoxy-3,5-dimethylbenzamid in 55 ml Phosphoroxidchlorid wurden 3 h auf 60°C erhitzt. Nach Abkühlen wurde der erhaltene Feststoff abgesaugt und mit Methyl-tert.-butylether gewaschen. Dann wurde der Feststoff in einer Mischung von Dichlormethan und Tetrahydrofuran gelöst, mit gesättigter wässriger Natriumhydrogencarbonatlösung gewaschen, getrocknet und im Vakuum aufkonzentriert. Es wurden 5,9 g der Titelverbindung erhalten.
   LC/MS (Methode LC1): Rt = 1,24 min; m/z = 318,08 [M+H]⁺
(e) 2-(4-Methoxy-3,5-dimethylphenyl)-5-methylsulfanyl-7-propoxyoxazolo[5,4-d]pyrimidin
   5,9 g 2-(4-Methoxy-3,5-dimethylphenyl)-5-methylsulfanyloxazolo[5,4-d]pyrimidin-7-ol wurden in 150 ml N,N-Dimethylformamid gelöst und mit 7,7 g Kaliumcarbonat und dann 2,7 g 1-Brompropan versetzt. Die Lösung wurde 5 h bei 60°C gerührt und dann nach Abkühlen auf 150 ml Wasser gegossen. Der Niederschlag wurde abgesaugt. Das erhaltene Regioisomerengemisch wurde durch Kieselgelchromatographie (50-g-Siliciumdioxid-Isolute-Kartusche, Heptan/Essigsäureethylester 9/1) gereinigt. Neben 1,4 g 2-(4-Methoxy-3,5-dimethylphenyl)-5-methylsulfanyl-6-propyl-6H-oxazolo[5,4-d]pyrimidin-7-on (LC/MS (Methode LC1): Rt = 1,43 min; m/z = 360,13 [M+H]⁺) wurden 2,5 g der Titelverbindung erhalten.
   LC/MS (Methode LC1): Rt = 1,51 min; m/z = 360,13 [M+H]⁺
(f) 2,6-Dimethyl-4-(5-methylsulfanyl-7-propoxyoxazolo[5,4-d]pyrimidin-2-yl)phenol
   Eine Lösung von 2,5 g 2-(4-Methoxy-3,5-dimethylphenyl)-5-methylsulfanyl-7-propoxyoxazolo[5,4-d]pyrimidin in 50 ml Dichlormethan wurde bei -20°C langsam mit 0,70 ml Bortribromid versetzt. Nach 1 h bei -20°C und 2 h bei Raumtemperatur wurde die Mischung durch Zugabe von gesättigter wässriger Natriumhydrogen-carbonatlösung gequencht, wobei die Temperatur unter 5°C gehalten wurde. Nach Phasentrennung wurde die wässrige Phase zweimal mit Dichlormethan extrahiert. Die vereinigten organischen Phasen wurden getrocknet und im Vakuum aufkonzentriert. Es wurden 2,0 g der Titelverbindung erhalten.
   LC/MS (Methode LC1): Rt = 1,41 min; m/z = 346,11 [M+H]⁺
(g) [2,6-Dimethyl-4-(5-methylsulfanyl-7-propoxyoxazolo[5,4-d]pyrimidin-2-yl)-phenoxy]essigsäure-tert.-butylester
   Eine Lösung von 2,00 g 2,6-Dimethyl-4-(5-methylsulfanyl-7-propoxyoxazolo[5,4-d]pyrimidin-2-yl)phenol in 20 ml N,N-Dimethylformamid wurde mit 3,20 g Kaliumcarbonat gefolgt von 0,93 ml Bromessigsäure-tert-butylester versetzt. Die Mischung wurde 1 h bei 60°C umgesetzt und dann abkühlen gelassen und auf Wasser gegossen. Der erhaltene Niederschlag wurde abgesaugt und im Vakuum getrocknet. Es wurden 2,45 g der Titelverbindung erhalten.
   LC/MS (Methode LC1): Rt = 1,52 min; m/z = 460,18 [M+H]⁺
(h) [4-(5-Methansulfonyl-7-propoxyoxazolo[5,4-d]pyrimidin-2-yl)-2,6-dimethyl-phenoxy]essigsäure-tert.-butylester
   250 mg [2,6-Dimethyl-4-(5-methylsulfanyl-7-propoxyoxazolo[5,4-d]pyrimidin-2-yl)phenoxy]essigsäure-tert.-butylester wurden in 5 ml Dichlormethan gelöst. Dann wurden bei 0°C 268 mg 3-Chlorperbenzoesäure zugegeben, wonach die Mischung 12 h bei Raumtemperatur gerührt wurde. Nach Behandlung der Mischung mit 1 M Natronlauge wurden die Schichten getrennt, wonach die organische Schicht zweimal mit Dichlormethan extrahiert wurde. Die vereinigten organischen Phasen wurden mit 10%iger wässriger Natriumhydrogensulfitlösung gewaschen, über Natriumsulfat getrocknet, filtriert und im Vakuum eingedampft. Es wurden 268 mg der Titelverbindung erhalten.
   LC/MS (Methode LC1): Rt = 1,38 min; m/z = 492,17 [M+H]⁺
(i) {4-[5-(2,5-Difluorphenoxy)-7-propoxyoxazolo[5,4-d]pyrimidin-2-yl]-2,6-dimethylphenoxy}essigsäure-tert.-butylester
   Eine Lösung von 100 mg [4-(5-Methansulfonyl-7-propoxyoxazolo[5,4-d]pyrimidin-2-yl)-2,6-dimethylphenoxy]essigsäure-tert.-butylester in 1,5 ml N,N-Dimethylformamid wurde mit 62 mg Kaliumcarbonat und 32 mg 2,5-Difluorphenol versetzt. Die Mischung wurde 12 h bei Raumtemperatur gerührt. Dann wurde die Mischung auf Wasser gegossen, durch Zugabe von 10%iger wässriger Natriumhydrogensulfatlösung neutralisiert und zweimal mit Essigsäureethylester extrahiert. Die vereinigten organischen Schichten wurden getrocknet und im Vakuum aufkonzentriert. Nach Filtration wurde das Lösungsmittel im Vakuum abdestilliert und der Rückstand durch präparative HPLC gereinigt, was 69 mg der Titelverbindung ergab.
   LC/MS (Methode LC1): Rt = 1,47 min; m/z = 542,20 [M+H]⁺
(j) {4-[5-(2,5-Difluorphenoxy)-7-propoxyoxazolo[5,4-d]pyrimidin-2-yl]-2,6-dimethylphenoxy}essigsäure
   69 mg {4-[5-(2,5-Difluorphenoxy)-7-propoxyoxazolo[5,4-d]pyrimidin-2-yl]-2,6-dimethylphenoxy}essigsäure-tert.-butylester wurden in 1,6 ml Dichlormethan gelöst und mit 0,8 ml Trifluoressigsäure behandelt. Nach 16 h wurde die Mischung aufkonzentriert und gefriergetrocknet. Es wurden 71 mg der Titelverbindung erhalten. LC/MS (Methode LC1): Rt = 1,35 min; m/z = 486,33 [M+H]⁺

### Beispiel 2

### [4-(5-Cyclopentyloxy-7-propoxyoxazolo[5,4-d]pyrimidin-2-yl)-2,6-dimethylphenoxy]-essigsäure

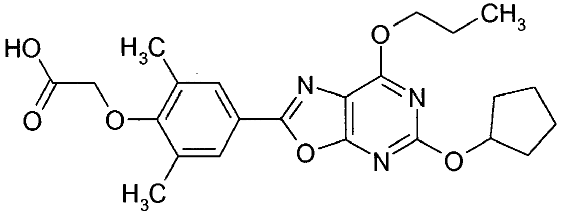

### (a) {4-[5-(Cyclopentyloxy)-7-propoxyoxazolo[5,4-d]pyrimidin-2-yl]-2,6-dimethylphenoxy}essigsäure-tert.-butylester

Eine Lösung von 98 mg [4-(5-Methansulfonyl-7-propoxyoxazolo[5,4-d]pyrimidin-2-yl)-2,6-dimethylphenoxy]essigsäure-tert.-butylester, 19 mg Cyclopentanol und 69 mg (tert.-Butylimino)tris(pyrrolidino)phospharan wurde 16 h bei Raumtemperatur umgesetzt und anschließend 10 min in einem Mikrowellenreaktor auf 100°C erhitzt. Die Mischung wurde auf Wasser gegossen und zweimal mit Essigsäureester extrahiert. Die vereinigten organischen Schichten wurden mit einer 10%igen wäßrigen Lösung aus Citronensäure und Kochsalzlösung gewaschen, getrocknet und eingedampft. Nach der Aufreinigung durch präparative HPLC wurden 27 mg der Titelverbindung erhalten.
LC/MS (Methode LC1): Rt = 1,56 min; m/z = 498,25 [M+H]⁺

### b) {4-[5-(Cyclopentyloxy)-7-propoxyoxazolo[5,4-d]pyrimidin-2-yl]-2,6-dimethylphenoxy}essigsäure

25 mg {4-[5-(Cyclopentyloxy)-7-propoxyoxazolo[5,4-d]pyrimidin-2-yl]-2,6-dimethylphenoxylessigsäure-tert.-butylester wurden in 2 ml Dichlormethan gelöst und mit 1 ml Trifluoressigsäure behandelt. Nach 16 h wurde die Mischung aufkonzentriert und gefriergetrocknet. Es wurden 24 mg der Titelverbindung erhalten.
LC/MS (Methode LC1): Rt = 1,31 min; m/z = 442,1 [M+H]⁺

### Beispiel 3

### {4-[5-(trans-2-Fluorcyclohexyloxy)-7-propoxyoxazolo[5,4-d]pyrimidin-2-yl]-2,6-dimethylphenoxy}essigsäure

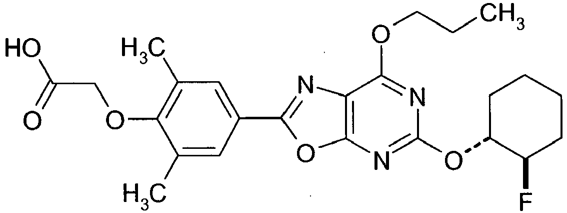

### (a) f4-[5-(trans-2-Fluorcyclohexyloxy)-7-propoxyoxazolo[5,4-d]pyrimidin-2-yl]-2,6-dimethylphenoxy}essigsäure-tert.-butylester

Eine Suspension von 10 mg Natriumhydrid (60%ig in Mineralöl) in 2 ml N,N-Dimethylformamid wurde unter Argonatmosphäre bei 0°C mit 26 mg trans-2-Fluorcyclohexanol versetzt. Nach 15 min wurde langsam eine Lösung von 100 mg [4-(5-Methansulfonyl-7-propoxyoxazolo[5,4-d]pyrimidin-2-yl)-2,6-dimethylphenoxy]-essigsäure-tert.-butylester in 1 ml N,N-Dimethylformamid zugegeben. Nach 12 h bei Raumtemperatur wurde die Mischung durch Zugabe von Wasser gequencht und zweimal mit Essigsäureethylester extrahiert. Die vereinigten organischen Schichten wurden getrocknet und im Vakuum aufkonzentriert. Es wurden 101 mg der Titelverbindung erhalten.
LC/MS (Methode LC1): Rt = 1,49 min; m/z = 530,26 [M+H]⁺ und 552,26 [M+Na]⁺

### (b) {4-[5-(trans-2-Fluorcyclohexyloxy)-7-propoxyoxazolo[5,4-d]pyrimidin-2-yl]-2,6-dimethylphenoxy}essigsäure

98 mg {4-[5-(trans-2-Fluorcyclohexyloxy)-7-propoxyoxazolo[5,4-d]pyrimidin-2-yl]-2,6-dimethylphenoxy}essigsäure-tert.-butylester wurden in 3,5 ml Dichlormethan gelöst und mit 1,5 ml Trifluoressigsäure behandelt. Nach 16 h wurde die Mischung aufkonzentriert und gefriergetrocknet. Es wurden 96 mg der Titelverbindung erhalten.
LC/MS (Methode LC1): Rt = 1,35 min; m/z = 474,20 [M+H]⁺

In Analogie zur Herstellung der oben beschriebenen Beispielverbindungen wurden die in Tabelle 1 aufgeführten Verbindungen der Formel I hergestellt. Sie wurden teilweise in Form ihres Trifluoressigsäuresalzes erhalten.

**Tabelle 1. Beispielverbindungen der Formel I**

| Beispiel | Name | LC/MS | m/z [M+H]⁺ | Rt [min] |
|---|---|---|---|---|
| 4 | {4-[5-(2-Fluorphenoxy)-7-propoxyoxazolo[5,4- d]pyrimidin-2-yl]-2,6-dimethylphenoxy}essigsäure | LC1 | 468,4 | 1,38 |
| 5 | {4-[5-(5-Fluor-2-methylphenoxy)-7-propoxy- oxazolo[5,4-d]pyrimidin-2-yl]-2,6-dimethyl- phenoxy}essigsäure | LC1 | 482,26 | 1,36 |
| 6 | {4-[5-(3-Fluor-4-methylphenoxy)-7- propoxyoxazolo[5,4-d]pyrimidin-2-yl]-2,6- dimethylphenoxy}essigsäure | LC1 | 482,37 | 1,30 |
| 7 | {2,6-Dimethyl-4-[7-propoxy-5-(pyridin-3-yloxy)- oxazolo[5,4-d]pyrimidin-2-yl]phenoxy}essigsäure | LC1 | 451,34 | 1,22 |
| 8 | {4-[5-(2,4-Difluorphenoxy)-7-propoxyoxazolo[5,4- d]pyrimidin-2-yl]-2,6-dimethylphenoxy}essigsäure | LC1 | 486,32 | 1,35 |
| 9 | [2,6-Dimethyl-4-(5-phenoxy-7-propoxyoxazolo[5,4- d]pyrimidin-2-yl)phenoxy]essigsäure | LC1 | 450,32 | 1,35 |
| 10 | {4-[5-(3-Chlorphenoxy)-7-propoxyoxazolo[5,4- d]pyrimidin-2-yl]-2,6-dimethylphenoxy}essigsäure | LC1 | 483,86 | 1,30 |
| 11 | [4-(5-Cyclohexylmethoxy-7-propoxyoxazolo[5,4- d]pyrimidin-2-yl)-2,6-dimethylphenoxy]essigsäure | LC1 | 470,44 | 1,38 |
| 12 | [4-(5-Isobutoxy-7-propoxyoxazolo[5,4-d]pyrimidin-2- yl)-2,6-dimethylphenoxy]essigsäure | LC1 | 430,29 | 1,42 |
| 13 | [4-(5-Cyclobutylmethoxy-7-propoxyoxazolo[5,4- d]pyrimidin-2-yl)-2,6-dimethylphenoxy]essigsäure | LC1 | 442,30 | 1,43 |
| 14 | [4-(5-Cyclobutoxy-7-propoxyoxazolo[5,4-d]pyrimidin- 2-yl)-2,6-dimethylphenoxy]essigsäure | LC1 | 428,27 | 1,40 |
| 15 | [4-(5,7-Dipropoxyoxazolo[5,4-d]pyrimidin-2-yl)-2,6-dimethylphenoxy]essigsäure | LC1 | 416,28 | 1,38 |
| 16 | {2,6-Dimethyl-4-[7-propoxy-5-(3,3,3-trifluor- propoxy)oxazolo[5,4-d]pyrimidin-2- yl]phenoxy}essigsäure | LC1 | 470,25 | 1,35 |
| 17 | [4-(5-Ethoxy-7-propoxyoxazolo[5,4-d]pyrimidin-2-yl)- 2,6-dimethylphenoxy]essigsäure | LC1 | 402,26 | 1,34 |
| 18 | [4-(5-Cyclopentylmethoxy-7-propoxyoxazolo[5,4- d]pyrimidin-2-yl)-2,6-dimethylphenoxy]essigsäure | LC1 | 456,30 | 1,46 |
| 19 | {2,6-Dimethyl-4-[7-propoxy-5-(tetrahydrofuran-2- ylmethoxy)oxazolo[5,4-d]pyrimidin-2-yl]- phenoxy}essigsäure | LC1 | 458,28 | 1,31 |
| 20 | [4-(5-sek.-Butoxy-7-propoxyoxazolo[5,4-d]pyrimidin- 2-yl)-2,6-dimethylphenoxy]essigsäure | LC1 | 430,29 | 1,40 |
| 21 | {2,6-Dimethyl-4-[7-propoxy-5-(3,3,3-trifluor-1- methylpropoxy)oxazolo[5,4-d]pyrimidin-2-yl]- phenoxy}essigsäure | LC1 | 484,28 | 1,37 |
| 22 | {2,6-Dimethyl-4-[5-(3-methylbutoxy)-7-propoxy- oxazolo[5,4-d]pyrimidin-2-yl]phenoxy}essigsäure | LC1 | 444,32 | 1,44 |
| 23 | {4-[5-(2-Cyclopropylethoxy)-7-propoxyoxazolo[5,4- d]pyrimidin-2-yl]-2,6-dimethylphenoxy}essigsäure | LC1 | 442,31 | 1,41 |
| 24 | {2,6-Dimethyl-4-[7-propoxy-5-(2,2,2-trifluor-1- methylethoxy)oxazolo[5,4-d]pyrimidin-2-yl]- phenoxy}essigsäure | LC1 | 470,25 | 1,38 |
| 25 | {4-[7-Ethoxy-5-(3-fluorphenoxy)-oxazolo[5,4- d]pyrimidin-2-yl]-2,6-dimethyl-phenoxy}-essigsäure | LC2 | 454,15 | 1,35 |
| 26 | {4-[7-Ethoxy-5-(2-fluorphenoxy)-oxazolo[5,4- d]pyrimidin-2-yl]-2,6-dimethyl-phenoxy}-essigsäure | LC2 | 454,16 | 1,34 |
| 27 | {4-[5-(Isothiazol-3-yloxy)-7-propoxy-oxazolo[5,4- d]pyrimidin-2-yl]-2,6-dimethylphenoxy}-essigsäure | LC2 | 457,11 | 1,31 |
| 28 | {2,6-Dimethyl-4-[7-propoxy-5-([1,2,5]thiadiazol-3- yloxy)-oxazolo[5,4-d]pyrimidin-2-yl]-phenoxy}- essigsäure | LC2 | 458,09 | 1,34 |
| 29 | [2,6-Dimethyl-4-(7-propoxy-5-(3-methylphenoxy)- oxazolo[5,4-d]pyrimidin-2-yl)-phenoxy]-essigsäure | LC2 | 464,17 | 1,41 |
| 30 | [2,6-Dimethyl-4-(7-propoxy-5-(2-methylphenoxy)-oxazolo[5,4-d]pyrimidin-2-yl)-phenoxy]-essigsäure | LC2 | 464,17 | 1,41 |
| 31 | [2,6-Dimethyl-4-(7-propoxy-5-(4-methylphenoxy)-oxazolo[5,4-d]pyrimidin-2-yl)-phenoxy]-essigsäure | LC3 | 464,17 | 4,98 |
| 32 | {2,6-Dimethyl-4-[5-(6-methylpyridin-3-yloxy)-7- propoxy-oxazolo[5,4-d]pyrimidin-2-yl]-phenoxy}- essigsäure | LC3 | 465,16 | 4,2 |
| 33 | {2,6-Dimethyl-4-[5-(5-methylpyridin-3-yloxy)-7- propoxy-oxazolo[5,4-d]pyrimidin-2-yl]-phenoxy}- essigsäure | LC2 | 465,19 | 1,28 |
| 34 | {4-[5-(3-Fluorphenoxy)-7-propoxy-oxazolo[5,4- d]pyrimidin-2-yl]-2,6-dimethyl-phenoxy}-essigsäure | LC2 | 468,15 | 1,39 |
| 35 | {4-[5-(4-Fluorphenoxy)-7-propoxy-oxazolo[5,4- d]pyrimidin-2-yl]-2,6-dimethylphenoxy}-essigsäure | LC2 | 468,16 | 1,38 |
| 36 | {4-[7-Ethoxy-5-(5-fluor-2-methylphenoxy)- oxazolo[5,4-d]pyrimidin-2-yl]-2,6-dimethyl-phenoxy}- essigsäure | LC2 | 468,17 | 1,38 |
| 37 | {4-[5-(5-Fluorpyridin-3-yloxy)-7-propoxy-oxazolo[5,4- d]pyrimidin-2-yl]-2,6-dimethyl-phenoxy}-essigsäure | LC2 | 469,14 | 1,31 |
| 38 | {4-[5-(3-Chlorphenoxy)-7-ethoxy-oxazolo[5,4- d]pyrimidin-2-yl]-2,6-dimethyl-phenoxy}-essigsäure | LC2 | 470,13 | 1,39 |
| 39 | {4-[5-(2,5-Difluorphenoxy)-7-ethoxy-oxazolo[5,4- d]pyrimidin-2-yl]-2,6-dimethyl-phenoxy}-essigsäure | LC2 | 472,14 | 1,35 |
| 40 | {4-[5-(3-Ethylphenoxy)-7-propoxy-oxazolo[5,4- d]pyrimidin-2-yl]-2,6-dimethyl-phenoxy}-essigsäure | LC2 | 478,19 | 1,44 |
| 41 | {4-[5-(3-Methoxyphenoxy)-7-propoxy-oxazolo[5,4- d]pyrimidin-2-yl]-2,6-dimethylphenoxy}-essigsäure | LC1 | 480,2 | 1,24 |
| 42 | {4-[5-(3-Fluor-5-methylphenoxy)-7-propoxy- oxazolo[5,4-d]pyrimidin-2-yl]-2,6-dimethyl-phenoxy}- essigsäure | LC2 | 482,15 | 1,42 |
| 43 | {4-[5-(4-Fluor-3-methylphenoxy)-7-propoxy- oxazolo[5,4-d]pyrimidin-2-yl]-2,6-dimethyl-phenoxy}- essigsäure | LC2 | 482,18 | 1,41 |
| 44 | {4-[5-(2-Fluor-4-methylphenoxy)-7-propoxy- oxazolo[5,4-d]pyrimidin-2-yl]-2,6-dimethylphenoxy}- essigsäure | LC1 | 482,2 | 1,41 |
| 45 | {4-[5-(2-Fluor-5-methylphenoxy)-7-propoxy- oxazolo[5,4-d]pyrimidin-2-yl]-2,6-dimethylphenoxy}- essigsäure | LC1 | 482,2 | 1,41 |
| 46 | {4-[5-(4-Chlorphenoxy)-7-propoxy-oxazolo[5,4- d]pyrimidin-2-yl]-2,6-dimethylphenoxy}-essigsäure | LC2 | 484,14 | 1,42 |
| 47 | {4-[5-(2-Chlorphenoxy)-7-propoxy-oxazolo[5,4- d]pyrimidin-2-yl]-2,6-dimethylphenoxy}-essigsäure | LC1 | 484,17 | 1,4 |
| 48 | {4-[5-(5-Chlorpyridin-3-yloxy)-7-propoxy-oxazolo[5,4- d]pyrimidin-2-yl]-2,6-dimethyl-phenoxy}-essigsäure | LC2 | 485,11 | 1,36 |
| 49 | {4-[5-(2,3-Difluorphenoxy)-7-propoxy-oxazolo[5,4- d]pyrimidin-2-yl]-2,6-dimethylphenoxy}-essigsäure | LC2 | 486,14 | 1,39 |
| 50 | {4-[5-(3,5-Difluorphenoxy)-7-propoxy-oxazolo[5,4- d]pyrimidin-2-yl]-2,6-dimethyl-phenoxy}-essigsäure | LC2 | 486,15 | 1,4 |
| 51 | {4-[5-(3,4-Difluorphenoxy)-7-propoxy-oxazolo[5,4- d]pyrimidin-2-yl]-2,6-dimethylphenoxy}-essigsäure | LC2 | 486,16 | 1,39 |
| 52 | {4-[5-(Indan-4-yloxy)-7-propoxy-oxazolo[5,4- d]pyrimidin-2-yl]-2,6-dimethylphenoxy}-essigsäure | LC2 | 490,2 | 1,45 |
| 53 | {4-[5-(Indan-5-yloxy)-7-propoxy-oxazolo[5,4- d]pyrimidin-2-yl]-2,6-dimethylphenoxy}-essigsäure | LC1 | 490,26 | 1,45 |
| 54 | {4-[5-(3-Chlor-2-methylphenoxy)-7-propoxy- oxazolo[5,4-d]pyrimidin-2-yl]-2,6-dimethyl-phenoxy}- essigsäure | LC2 | 498,13 | 1,45 |
| 55 | {4-[5-(3-Chlor-4-methylphenoxy)-7-propoxy- oxazolo[5,4-d]pyrimidin-2-yl]-2,6-dimethylphenoxy}- essigsäure | LC2 | 498,15 | 1,46 |
| 56 | {4-[5-(5-Chlor-2-methylphenoxy)-7-propoxy- oxazolo[5,4-d]pyrimidin-2-yl]-2,6-dimethyl-phenoxy}- essigsäure | LC2 | 498,16 | 1,45 |
| 57 | {2,6-Dimethyl-4-[5-(naphthalen-2-yloxy)-7-propoxy- oxazolo[5,4-d]pyrimidin-2-yl]-phenoxy}-essigsäure | LC2 | 500,18 | 1,43 |
| 58 | {2,6-Dimethyl-4-[7-propoxy-5-(quinolin-3-yloxy)- oxazolo[5,4-d]pyrimidin-2-yl]-phenoxy}-essigsäure | LC2 | 501,19 | 1,35 |
| 59 | {4-[5-(4-Chlor-2-fluorphenoxy)-7-propoxy- oxazolo[5,4-d]pyrimidin-2-yl]-2,6-dimethyl-phenoxy}- essigsäure | LC2 | 502,11 | 1,42 |
| 60 | {4-[5-(3-Chlor-5-fluorphenoxy)-7-propoxy- oxazolo[5,4-d]pyrimidin-2-yl]-2,6-dimethyl-phenoxy}- essigsäure | LC2 | 502,12 | 1,44 |
| 61 | {4-[5-(2-Chlor-4-fluorphenoxy)-7-propoxy- oxazolo[5,4-d]pyrimidin-2-yl]-2,6-dimethyl-phenoxy}- essigsäure | LC2 | 502,13 | 1,41 |
| 62 | {4-[5-(3-Chlor-4-fluorphenoxy)-7-propoxy- oxazolo[5,4-d]pyrimidin-2-yl]-2,6-dimethylphenoxy}- essigsäure | LC2 | 502,13 | 1,42 |
| 63 | {4-[5-(3-Chlor-2-fluorphenoxy)-7-propoxy- oxazolo[5,4-d]pyrimidin-2-yl]-2,6-dimethyl-phenoxy}- essigsäure | LC2 | 502,14 | 1,42 |
| 64 | {4-[5-(4-Chlor-3-fluorphenoxy)-7-propoxy- oxazolo[5,4-d]pyrimidin-2-yl]-2,6-dimethylphenoxy}- essigsäure | LC2 | 502,14 | 1,43 |
| 65 | {4-[5-(2-Chlor-5-fluorphenoxy)-7-propoxy- oxazolo[5,4-d]pyrimidin-2-yl]-2,6-dimethylphenoxy}- essigsäure | LC2 | 502,14 | 1,41 |
| 66 | {4-[5-(Benzothiazol-6-yloxy)-7-propoxy-oxazolo[5,4- d]pyrimidin-2-yl]-2,6-dimethylphenoxy}-essigsäure | LC2 | 507,12 | 1,33 |
| 67 | {2,6-Dimethyl-4-[7-propoxy-5-(3- trifluormethylphenoxy)-oxazolo[5,4-d]pyrimidin-2-yl]- phenoxy}-essigsäure | LC2 | 518,18 | 1,42 |
| 68 | {2,6-Dimethyl-4-[7-propoxy-5-(4-trifluormethylphenoxy)-oxazolo[5,4-d]pyrimid in-2-yl]-phenoxy}-essigsäure | LC2 | 518,18 | 1,42 |
| 69 | {2,6-Dimethyl-4-[7-propoxy-5-(2- trifluormethylphenoxy)-oxazolo[5,4-d]pyrimidin-2-yl]-phenoxy}-essigsäure | LC1 | 518,22 | 1,4 |
| 70 | {2,6-Dimethyl-4-[5-(2-methylbenzothiazol-5-yloxy)-7- propoxy-oxazolo[5,4-d]pyrimidin-2-yl]-phenoxy)- essigsäure | LC2 | 521,15 | 1,37 |
| 71 | {2,6-Dimethyl-4-[7-propoxy-5-(2- trifluormethoxyphenoxy)-oxazolo[5,4-d]pyrimidin-2- yl]-phenoxy}-essigsäure | LC2 | 534,16 | 1,41 |
| 72 | {4-[5-(2-Fluor-3-trifluormethylphenoxy)-7-propoxy- oxazolo[5,4-d]pyrimidin-2-yl]-2,6-dimethyl-phenoxy}- essigsäure | LC2 | 536,13 | 1,42 |
| 73 | {4-[5-(2-Fluor-5-trifluormethylphenoxy)-7-propoxy- oxazolo[5,4-d]pyrimidin-2-yl]-2,6-dimethylphenoxy}- essigsäure | LC2 | 536,14 | 1,42 |
| 74 | {4-[5-(4-Fluor-3-trifluormethylphenoxy)-7-propoxy- oxazolo[5,4-d]pyrimidin-2-yl]-2,6-dimethyl-phenoxy}- essigsäure | LC2 | 536,15 | 1,42 |
| 75 | {4-[5-(3-Fluor-5-trifluormethylphenoxy)-7-propoxy- oxazolo[5,4-d]pyrimidin-2-yl]-2,6-dimethyl-phenoxy}- essigsäure | LC2 | 536,16 | 1,44 |
| 76 | {2,6-Dimethyl-4-[7-propoxy-5-(3- trifluormethylsulfanyl-phenoxy)-oxazolo[5,4- d]pyrimidin-2-yl]-phenoxy}-essigsäure | LC2 | 550,11 | 1,46 |
| 77 | {4-[5-(2-Chlor-5-trifluormethylphenoxy)-7-propoxyoxazolo[5,4-d]pyrimidin-2-yl]-2,6-dimethyl-phenoxy}-essigsäure | LC2 | 552,11 | 1,44 |
| 78 | {4-[5-(4-Chlor-3-trifluormethylphenoxy)-7-propoxy-oxazolo[5,4-d]pyrimidin-2-yl]-2,6-dimethylphenoxy}-essigsäure | LC2 | 552,11 | 1,45 |
| 79 | {4-[5-(2-Chlor-3-trifluormethylphenoxy)-7-propoxyoxazolo[5,4-d]pyrimidin-2-yl]-2,6-dimethylphenoxy}-essigsäure | LC2 | 552,11 | 1,43 |

Bestimmung der pharmakologischen Wirkung

### A) GTP-γ-S-Assay mit humanen Edg-1-Rezeptoren

Zur Bestimmung der Edg-1-Rezeptor-Aktivierung durch die erfindungsgemäßen Verbindungen wurde ein GTP-γ-S-Assay (GTP-γ-S = Guanosin-5'-[thio]triphosphat) auf die Bindung an G-Protein gekoppeltem Rezeptor auf Basis des Szintillationsproximitätsassay-Prinzips verwendet, wobei ein Zellmembranpräparat einer CHO-Flp-In-Zelllinie, die den humanen Edg-1-Rezeptor konstitutiv überexprimiert, eingesetzt wurde.

### (a) Erzeugung der Zelllinie

Das Flp-In™-Expressionssystem (Invitrogen, Kat.-Nr. K6010-01) erlaubt die Erzeugung von stabilen Säugetierzelllinien, in die das interessierende Gen durch homologe Rekombination an einem spezifischen Genomort, der als FRT-Ort (FRT = Flp Recombination Target) bezeichnet wird, mit Hilfe einer durch das pOG44-Expressionsplasmid codierten Flp-Rekombinase integriert worden ist. Die Integration des pcDNA5/FRT-Expressionskonstrukts in das Flp-In-Wirtszellliniengenom führt zur Transkription des interessierenden Gens. Die stabil transfizierten Zellen werden hygromycinresistent.

Einen Tag vor der Transfektion wurden 200 000 Flp-In-CHO-Zellen in Ham-F-12-Medium (Invitrogen, Kat.-Nr. 31765) mit 10% fötalem Kälberserum (FCS; Perbio Science, Kat.-Nr. SH30068.03) in einer Platte mit 6 Vertiefungen ausgesät und über Nacht bei 37°C/5 % CO₂ inkubiert. Unter Verwendung von FuGENE^{®}-6-Transfektionsreagens (Roche, Kat.-Nr. 11988387001) wurden Zellen mit dem Flp-Rekombinase-Expressionsplasmid pOG44 und einem modifizierten Plasmid, das zusätzlich das edg-1-Gen (Zugangsnummer NM_001400) enthält und als pcDNA5-FRT-TO_nFLAG_DEST-EDG-1 bezeichnet wird, mit einem Verhältnis von 9:1 kotransfiziert. Zum Erhalt des modifizierten pcDNA5-FRT-TO_nFLAG_DEST-Plasmids wurde das Invitrogen-Plasmid pcDNA5/FRT/TO (Invitrogen, Kat.-Nr. V6520-20) durch Insertierung einer Gateway-Kassette mit ein ccdB-Gen und ein Chloramphenicolresistenzgen flankierenden attR-Rekombinationsstellen (Gateway Conversion System, Invitrogen, Kat.-Nr. 11828-029) auf das Gateway^{®}-Kloniersystem (Invitrogen) angepasst. Außerdem wurde vor der 5'-att-Rekombinationsstelle ein FLAG-Tag-Epitop hinzugefügt, um eine rekombinante Expression von Proteinen mit N-terminalem FLAG-Tag zu ermöglichen.

Für die Transfektion einer Vertiefung wurden 1,08 µg pOG44 und 0,12 µg pcDNA5-FRT-TO_nFLAG_DEST-EDG-1 mit 100 µl serumfreiem Ham-F-12-Medium mit 6 µl FuGENE^{®}-6-Transfektionsreagens gemischt. Nach 20 min Inkubation wurde der Transfektionsreagens/DNA-Komplex tropfenweise auf den Zellen verteilt. Die Zellen wurden 24 h bei 37°C inkubiert. Dann wurden die Zellen aus drei Vertiefungen in eine T75-Flasche (Greiner Cellstar^{®}, Kat.-Nr. 658175) mit Ham-F-12-Medium mit 10% FCS, aber ohne Antibiotikum, überführt und noch 24 h inkubiert. 48 h nach der Transfektion wurde das Medium durch Selektionsmedium (Ham F-12 mit 10 % FCS und 300 µg/ml Hygromycin B (Invitrogen, Kat.-Nr. 10687-010)) ersetzt. Das Medium wurde alle 2 bis 3 Tage ausgetauscht, bis eine resistente Population von Zellen herangewachsen war. Zellen wurden mehrmals aufgeteilt und in eine neue Flasche ausgesät, so daß die Zellen nicht mehr als 25% Konfluenz erreichten. Nach 2 Wochen Selektion wurden die Zellen in T175-Flaschen (Greiner Cellstar^{®}, Kat.-Nr. 660175) überführt und für die Batchproduktion kultiviert. Die Zellen wurden durch kurze Behandlung (2 bis 5 min) mit Accutase (PAA, Kat.-Nr. L11-007) aus den Kulturflaschen geerntet, in Selektionsmedium (siehe oben) resuspendiert und 5 min bei 200 x g zentrifugiert. Die Zellen wurden in einer Mischung von 90% FCS und 10% Dimethylsulfoxid resuspendiert und in flüssigem Stickstoff gefroren gelagert.

### (b) Membranpräparat

Aus der obenbeschriebenen CHO-Flp-In-Zelllinie, die den humanen Edg-1-Rezeptor konstitutiv überexprimiert, wurde nach Standardmethoden ein Membranpräparat erhalten. Kurz gesagt, wurden die kryokonservierten Zellen in Kultur genommen und in T175-Zellkulturflaschen (Becton Dickinson, Kat.-Nr. 35 5001) bis zur Konfluenz angezogen. Die Zellkultur wurde durch Waschen mit calciumfreier phosphatgepufferter Kochsalzlösung (PBS; Gibco, Kat.-Nr. 14190) gestoppt, und die Zellen wurden mit einem Gummischaber in 4°C kaltem und calciumfreiem PBS mit einem Proteaseinhibitorcocktail (Complete Protease Inhibitor; Roche, Kat.-Nr. 1697498; 1 Tablette pro 50 ml) geerntet und danach bei 4°C 15 min bei 1100 x g zentrifugiert (Heraeus Minifuge T). Zur Zelllyse wurde das Pellett in 4°C kaltem hypotonischem Puffer aus 5 mM HEPES (Sigma-Aldrich, Kat.-Nr. H-0981), 1 mM EDTA (Dinatriumsalz; Merck, Kat.-Nr. 8418) mit Proteaseinhibitorcocktail (wie oben) resuspendiert, in dem die Zellen noch 15 min auf Eis gelagert wurden. Nach der Lyse wurden die Zellen bei 4°C 10 min bei 400 x g zentrifugiert (Heraeus Minifuge T). Das Pellet wurde in einem Dounce-Homogenisator auseinandergebrochen, mit dem Überstand der vorhergehenden Zentrifugation verdünnt und danach bei 4°C 10 min bei 500 x g zentrifugiert (Heraeus Minifuge T), um Nuklei und noch intakte Zellen von den hauptsächlich im Überstand vorliegenden Membranen abzutrennen. Der Überstand wurde dann in hypotonischem Puffer verdünnt und bei 4°C bei ungefähr 18600 x g 2 h zentrifugiert (Beckmann, Avanti J251). Danach wurde das Membranpellet in einem Lagerpuffer aus 20 mM HEPES; 150 mM NaCl (Merck, Kat.-Nr. 6400), 1 mM EDTA (wie oben) mit Proteaseinhibitorcocktail (wie oben) resuspendiert. Das Membranpräparat wurde aliquotiert und bei -80°C gelagert. Die Proteinkonzentration des Membranpräparats wurde in einer Probe mit Hilfe eines kommerziellen Proteinassays (Bio-Rad, DC Protein Assay, Kat.-Nr. 500-0113, 500-0114, 500-0115) bestimmt.

### (c) GTP-γ-S-Assay

Das in (b) erhaltene Edg-1-Membranpräparat wurde in einem kommerziell erhältlichen Szintillationsproximitätsassay-Kit (SPA-Kit) auf die Bindung am G-Protein-gekoppelten Rezeptor von Amersham Biosciences/GE Healthcare (Code RPNQ0210) eingesetzt, indem die ligandeninduzierte Bindung von ³⁵S-radiomarkiertem GTP-γ-S an die rezeptorhaltige Membran, die an Szintillationsperlen gebunden ist, die Emission von Licht induziert und die Quantifizierung der in-vitro-Wirkung der Edg-1-agonistischen Verbindung erlaubt. Der Assay wurde auf einer Platte mit 96 Vertiefungen weitgehend nach den Anweisungen des Herstellers durchgeführt. Vor dem Beginn der Experimente wurden Szintillationsperlen in einem Rekonstitutionspuffer aus Tris-HCl (pH 7,4) mit 0,1% (w/v) Natriumazid suspendiert und dann auf Eis mit Assaypuffer (aus 20 mM HEPES, 100 mM NaCl, 1 mM EDTA (wie oben), 1 mM Dithiothreitol (DTT), auf pH 7,4 eingestellt) auf eine Perlenendkonzentration von 30 mg/ml verdünnt.

Die Vertiefungen wurden mit 10 µl des angegebenen Assaypuffers, 10 µl 100 µM Guanosindiphosphat-Lösung (GDP-Lösung) und 10 µl einer Lösung der Testverbindung in Assaypuffer/Dimethylsulfoxid versetzt, was eine Endkonzentration der Testverbindung von 10 µM ergibt. Anstelle der Lösung der Testverbindung wurden für die hohen Kontrollen 10 µl einer Lösung von Sphingosin-1-phosphat (S1P; Sigma, Kat.-Nr. S-9666), was eine S1P-Endkonzentration von 10 µM ergab, und für die niedrigen Kontrollen 10 µl Assaypuffer in die jeweiligen Vertiefungen gegeben. Alle Vertiefungen enthielten äquivalente Mengen von Dimethylsulfoxid. Dann wurden in jede Vertiefung 10 µl einer [³⁵S]GTP-γ-S-Lösung (4 nM) und das in (b) erhaltene Edg-1-Membranpräparat (15 µg Membranprotein in 100 µl Assaypuffer) gegeben. Nach Inkubation der Platten bei Raumtemperatur über einen Zeitraum von 5 min wurden 50 µl der angegebenen Szintillationsperlensuspension (30 mg/ml) zugegeben. Nach einem weiteren Inkubationszeitraum von 45 min bei Raumtemperatur wurden die Platten 10 min bei 500 x g zentrifugiert. Die Quantifizierung der [³⁵S]GTP-γ-S-Bindung und somit der Rezeptoraktivierung wurde mit Hilfe eines Beta-Zählers (MicroBeta, Wallac) über einen Zeitraum von 1 min gemessen. Die Werte wurden durch Subtraktion der jeweiligen niedrigen Kontrolle hintergrundkorrigiert. Alle Messungen wurden dreifach durchgeführt. Die Rezeptoraktivierung durch die Testverbindung wird in % der jeweiligen hohen Kontrolle (10 µM S1P; wird als 100% Aktivierung erachtet) ausgedrückt. Die mit Beispielverbindungen bei 10 µM beobachteten Aktivierungen sind in Tabelle 2 aufgeführt.

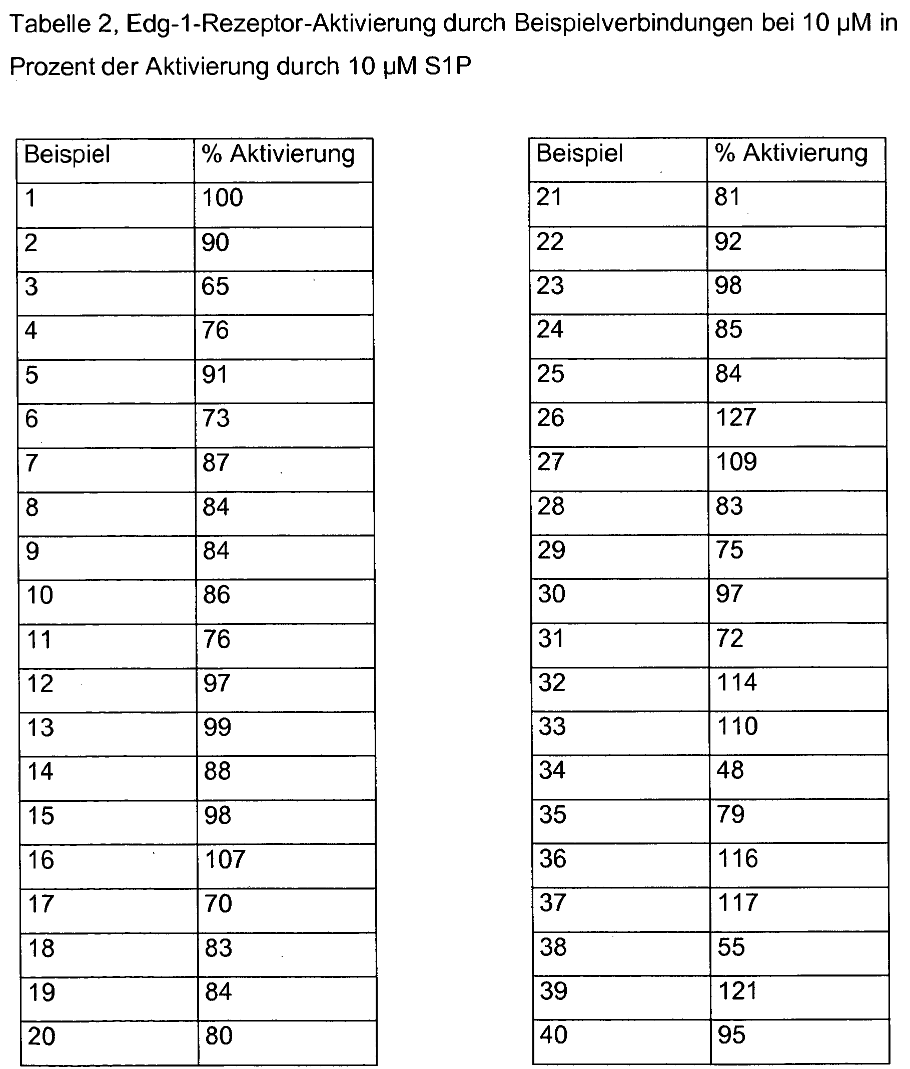

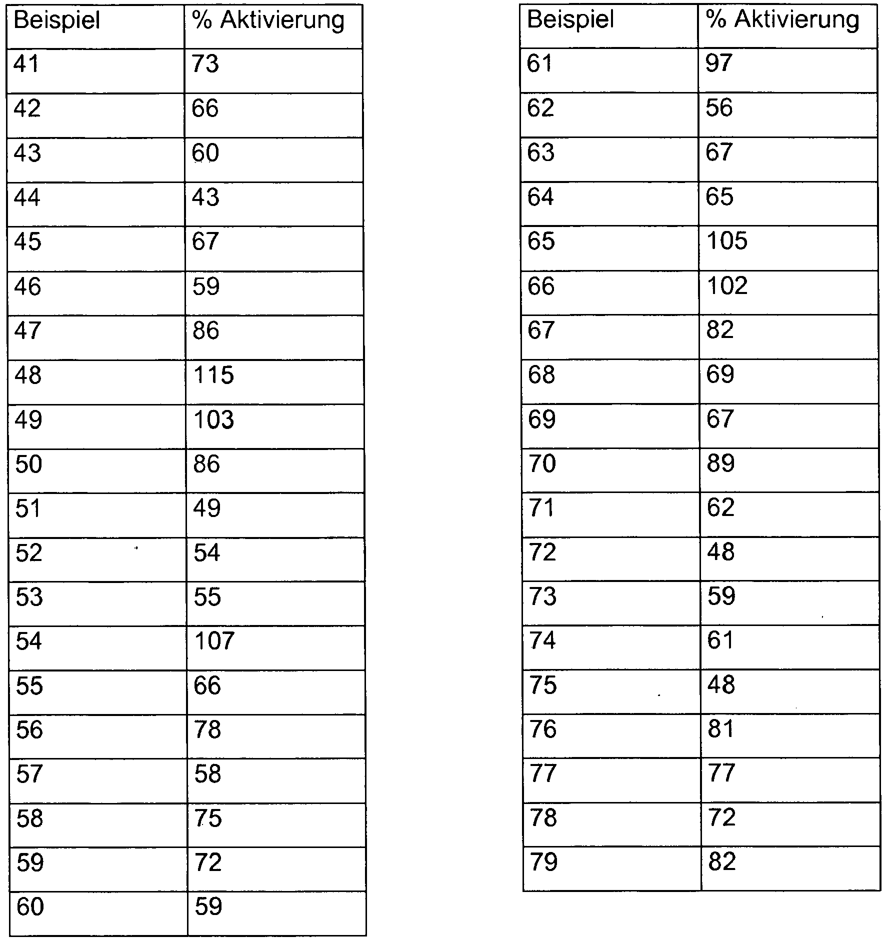

Aus den Messdaten ist ersichtlich, dass die Verbindungen gut zur Wundheilung und insbesondere zur Behandlung von Wundheilungsstörungen von Diabetes Patienten geeignet sind.

## Patentansprüche

1. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes, 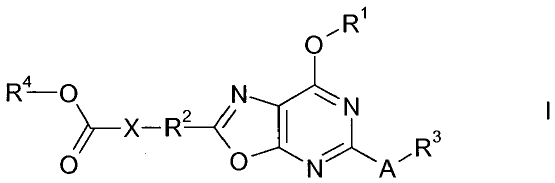 worin
A ausgewählt ist aus NH, O und S;
X ausgewählt ist aus (C₁-C₆)-Alkandiyl, (C₂-C₆)-Alkendiyl, (C₂-C₆)-Alkindiyl, (C₃-C₇)-Cycloalkandiyl und (C₁-C₆)-Alkandiyloxy, die alle gegebenenfalls durch einen oder mehrere gleiche oder verschiedene Substituenten substituiert sind, die aus Fluor und Hydroxy ausgewählt sind, wobei das Sauerstoffatom der (C₁-C₆)-Alkandiyloxygruppe an die Gruppe R² gebunden ist;
R¹ ausgewählt ist aus (C₁-C₆)-Alkyl, (C₂-C₆)-Alkenyl, (C₂-C₆)-Alkinyl, (C₃-C₇)-Cycloalkyl-CₜH₂ₜ- und Het-CₜH₂ₜ-, worin t aus 0, 1, 2 und 3 ausgewählt ist;
R² ausgewählt ist aus Phenylen und einem zweiwertigen Rest eines aromatischen, 5-gliedrigen bis 6-gliedrigen monocyclischen Heterocyclus, der 1, 2 oder 3 gleiche oder verschiedene Ringheteroatome enthält, die aus N, O und S ausgewählt sind, wobei eines der Ringstickstoffatome ein Wasserstoffatom oder einen Substituenten R²¹ tragen kann und wobei das Phenylen und der zweiwertige Rest eines aromatischen Heterocyclus gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₂-C₆)-Alkenyl, (C₂-C₆)-Alkinyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1, 2, 3 oder 4 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und ein oder zwei der Ringschwefelatome eine oder zwei Oxogruppen tragen können und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
R⁴ ausgewählt ist aus Wasserstoff, (C₁-C₄)-Alkyl und (C₃-C₇)-Cycloalkyl-C_{z}H_{2z}-, worin z aus 0, 1 und 2 ausgewählt ist;
R²¹ ausgewählt ist aus (C₁-C₄)-Alkyl, (C₃-C₇)-Cycloalkyl-C_{w}H_{2w}- und Oxy, wobei w aus 0, 1 und 2 ausgewählt ist;
R²² ausgewählt ist aus Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl;
R³¹ ausgewählt ist aus Halogen, (C₁-C₄)-Alkyl, (C₃-C₇)-Cycloalkyl, Hydroxy, (C₁-C₄)-Alkyloxy, Oxo, (C₁-C₄)-Alkyl-S(O)ₘ-, Amino, (C₁-C₄)-Alkylamino, Di((C₁-C₄)-alkyl)amino, (C₁-C₄)-Alkylcarbonylamino, (C₁-C₄)-Alkylsulfonylamino, Nitro, Cyano, (C₁-C₄)-Alkylcarbonyl, Aminosulfonyl, (C₁-C₄)-Alkylaminosulfonyl und Di((C₁-C₄)-alkyl)aminosulfonyl;
Het für einen Rest eines gesättigten, 4-gliedrigen bis 7-gliedrigen, monocyclischen Heterocyclus, der 1 oder 2 gleiche oder verschiedene Ringheteroatome enthält, die unter N, O und S ausgewählt sind, und der über ein Ringkohlenstoffatom gebunden ist, steht, wobei der Rest eines Heterocyclus gegebenenfalls durch einen oder mehrere gleiche oder verschiedene Substituenten substituiert ist, die unter Fluor und (C₁-C₄)-Alkyl ausgewählt sind;
m aus 0, 1 und 2 ausgewählt ist, wobei alle Zahlen m voneinander unabhängig sind; wobei alle Cycloalkyl- und Cycloalkandiylgruppen unabhängig voneinander und unabhängig von anderen Substituenten gegebenenfalls durch einen oder mehrere gleiche oder verschiedene Substituenten substituiert sind, die unter Fluor und (C₁-C₄)-Alkyl ausgewählt sind;
wobei alle Alkyl-, Alkandiyl-, CtH2t-, CᵤH₂ᵤ-, CᵥH₂ᵥ-, C_{w}H_{2w}-, C_{z}H_{2z}-, Alkenyl-, Alkendiyl-, Alkinyl- und Alkindiylgruppen unabhängig voneinander und unabhängig von anderen Substituenten gegebenenfalls durch einen oder mehrere Fluorsubstituenten substituiert sind.

2. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach Anspruch 1, worin A ausgewählt ist aus O und S.

3. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 und 2, wobei X aus (C₁-C₆)-Alkandiyl, (C₂-C₆)-Alkandiyl und (C₁-C₆)-Alkandiyloxy ausgewählt ist.

4. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 3, worin
R¹ ausgewählt ist aus (C₁-C₆)-Alkyl, (C₃- C₇)-Cycloalkyl-CₜH₂ₜ- und Het-CₜH₂ₜ-, worin t aus 0, 1 und 2 ausgewählt ist.

5. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 4, worin
R² ausgewählt ist aus Phenylen und Pyridindiyl, worin das Phenylen und das Pyridindiyl gegebenenfalls an einem oder mehreren Ringkohlenstoffatomen durch gleiche oder verschiedene Substituenten R²² substituiert sind.

6. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 5, worin
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 10-gliedrigen, monocyclischen oder bicyclischen Rings, der 0, 1 oder 2 gleiche oder verschiedene Ringheteroatome enthält, die aus N, O und S ausgewählt sind, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind.

7. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 6, worin
A aus O und S ausgewählt ist;
X ausgewählt ist aus (C₁-C₆)-Alkandiyl, (C₂-C₆)-Alkendiyl und (C₁-C₆)-Alkandiyloxy; R¹ ausgewählt ist aus (C₁-C₆)-Alkyl, (C₃-C₇)-Cycloalkyl-CₜH₂ₜ- und Het-CₜH₂ₜ-, worin t aus 0, 1 und 2 ausgewählt ist;
R² ausgewählt ist aus Phenylen und Pyridindiyl, worin das Phenylen und das Pyridindiyl gegebenenfalls an einem oder mehreren Ringkohlenstoffatomen durch gleiche oder verschiedene Substituenten R²² substituiert sind;
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 1 0-gliedrigen, monocyclischen oder bicyclischen Rings, der 0, 1 oder 2 gleiche oder verschiedene Ringheteroatome, die aus N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
Het für einen Rest eines gesättigten, 4-gliedrigen bis 6-gliedrigen, monocyclischen Heterocyclus, der 1 Ringheteroatom, das aus N, O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind.

8. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 7, worin A für O steht.

9. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes nach einem oder mehreren der Ansprüche 1 bis 8, worin
A für O steht;
X aus (C₁-C₆)-Alkandiyl und (C₁-C₆)-Alkandiyloxy ausgewählt ist;
R¹ aus (C₁-C₆)-Alkyl und (C₃-C₆)-Cycloalkyl-CₜH₂ₜ-, worin t aus 0 und 1 ausgewählt ist, ausgewählt ist;
R² für Phenylen steht, das gegebenenfalls an einem oder mehreren Ringkohlenstoffatomen durch gleiche oder verschiedene Substituenten R²² substituiert ist;
R³ ausgewählt ist aus (C₁-C₆)-Alkyl, (C₃-C₇)-Cycloalkyl-CᵤH₂ᵤ- und Het-CᵥH₂ᵥ-, worin u und v aus 1 und 2 ausgewählt sind, oder R³ für einen Rest eines gesättigten oder ungesättigten, 3-gliedrigen bis 7-gliedrigen, monocyclischen oder bicyclischen Rings, der 0, 1 oder 2 gleiche oder verschiedene Ringheteroatome, die unter N, O und S ausgewählt sind, enthält, steht, wobei ein oder zwei der Ringstickstoffatome ein Wasserstoffatom oder einen (C₁-C₄)-Alkylsubstituenten tragen können und eines der Ringschwefelatome eine oder zwei Oxogruppen tragen kann und wobei der Rest eines Rings gegebenenfalls an einem oder mehreren Ringkohlenstoffatomen durch gleiche oder verschiedene Substituenten R³¹ substituiert ist, mit der Maßgabe, daß R³ nicht für (C₁-C₆)-Alkyl stehen kann, wenn A für S steht;
R⁴ aus Wasserstoff und (C₁-C₄)-Alkyl ausgewählt ist;
R²² ausgewählt ist aus Halogen, Hydroxy, (C₁-C₄)-Alkyl- und (C₁-C₄)-Alkyloxy;
R³¹ ausgewählt ist aus Halogen, (C₁-C₄)-Alkyl, (C₃-C₇)-Cycloalkyl, Hydroxy und (C₁-C₄)-Alkyloxy;
Het für einen Rest eines gesättigten, 4-gliedrigen bis 6-gliedrigen, monocyclischen Heterocyclus, der 1 Ringheteroatom, das aus O und S ausgewählt ist, enthält und über ein Ringkohlenstoffatom gebunden ist, steht, wobei der Rest eines Heterocyclus gegebenenfalls durch einen oder mehrere gleiche oder verschiedene Substituenten substituiert ist, die aus Fluor und (C₁-C₄)-Alkyl ausgewählt sind;
wobei alle Cycloalkylgruppen unabhängig voneinander und unabhängig von anderen Substituenten gegebenenfalls durch einen oder mehrere gleiche oder verschiedene Substituenten substituiert sind, die aus Fluor und (C₁-C₄)-Alkyl ausgewählt sind;
wobei alle Alkyl-, Alkandiyl-, CₜH₂ₜ-, CᵤH₂ᵤ- und CᵥH₂ᵥ-Gruppen unabhängig voneinander und unabhängig von anderen Substituenten gegebenenfalls durch einen oder mehrere Fluorsubstituenten substituiert sind.

10. Verbindung der Formel I oder ein physiologisch akzeptables Salz davon, oder ein physiologisch akzeptables Solvat einer derartigen Verbindung oder eines derartigen Salzes, nach einem oder mehreren der Ansprüche 1 bis 9, die ausgewählt ist aus
{4-[5-(2,5-Difluorphenoxy)-7-propoxyoxazolo[5,4-d]pyrimidin-2-yl]-2,6-dimethylphenoxy}essigsäure,
[4-(5-Cyclopentyloxy-7-propoxyoxazolo[5,4-d]pyrimidin-2-yl)-2,6-dimethylphenoxy]-essigsäure,
{4-[5-(trans-2-Fluorcyclohexyloxy)-7-propoxyoxazolo[5,4-d]pyrimidin-2-yl]-2,6-dimethylphenoxy}essigsäure,
{4-[5-(2-Fluorphenoxy)-7-propoxyoxazolo[5,4-d]pyrimidin-2-yl]-2,6-dimethylphenoxy}-essigsäure,
{4-[5-(5-Fluor-2-methylphenoxy)-7-propoxyoxazolo[5,4-d]pyrimidin-2-yl]-2,6-dimethylphenoxy}essigsäure,
{4-[5-(3-Fluor-4-methylphenoxy)-7-propoxyoxazolo[5,4-d]pyrimidin-2-yl]-2,6-dimethyl-phenoxy}essigsäure,
{2,6-Dimethyl-4-[7-propoxy-5-(pyridin-3-yloxy)-oxazolo[5,4-d]pyrimidin-2-yl]phenoxy}-essigsäure,
{4-[5-(2,4-Difluorphenoxy)-7-propoxyoxazolo[5,4-d]pyrimidin-2-yl]-2,6-dimethylphenoxy}essigsäure,
[2,6-Dimethyl-4-(5-phenoxy-7-propoxyoxazolo[5,4-d]pyrimidin-2-yl)-phenoxy]-essigsäure,
{4-[5-(3-Chlorphenoxy)-7-propoxyoxazolo[5,4-d]pyrimidin-2-yl]-2,6-dimethylphenoxy}-essigsäure,
[4-(5-Cyclohexylmethoxy-7-propoxyoxazolo[5,4-d]pyrimidin-2-yl)-2,6-dimethylphenoxy]essigsäure,
[4-(5-Isobutoxy-7-propoxyoxazolo[5,4-d]pyrimidin-2-yl)-2,6-dimethylphenoxy]-essigsäure,
[4-(5-Cyclobutylmethoxy-7-propoxyoxazolo[5,4-d]pyrimidin-2-yl)-2,6-dimethylphenoxy]essigsäure,
[4-(5-Cyclobutoxy-7-propoxyoxazolo[5,4-d]pyrimidin-2-yl)-2,6-dimethylphenoxy]-essigsäure,
[4-(5,7-Dipropoxyoxazolo[5,4-d]pyrimidin-2-yl)-2,6-dimethylphenoxy]essigsäure,
{2,6-Dimethyl-4-[7-propoxy-5-(3,3,3-trifluorpropoxy)oxazolo[5,4-d]pyrimidin-2-yl]-phenoxy}essigsäure,
[4-(5-Ethoxy-7-propoxyoxazolo[5,4-d]pyrimidin-2-yl)-2,6-dimethylphenoxy]essigsäure,
[4-(5-Cyclopentylmethoxy-7-propoxyoxazolo[5,4-d]pyrimidin-2-yl)-2,6-dimethylphenoxy]essigsäure,
{2,6-Dimethyl-4-[7-propoxy-5-(tetrahydrofuran-2-ylmethoxy)oxazolo[5,4-d]pyrimidin-2-yl]phenoxy}essigsäure,
[4-(5-sek.-Butoxy-7-propoxyoxazolo[5,4-d]pyrimidin-2-yl)-2,6-dimethylphenoxy]-essigsäure,
{2,6-Dimethyl-4-[7-propoxy-5-(3,3,3-trifluor-1-methylpropoxy)oxazolo[5,4-d]pyrimidin-2-yl]phenoxy}essigsäure,
{2,6-Dimethyl-4-[5-(3-methylbutoxy)-7-propoxyoxazolo[5,4-d]pyrimidin-2-yl]phenoxy}-essigsäure,
{4-[5-(2-Cyclopropylethoxy)-7-propoxyoxazolo[5,4-d]pyrimidin-2-yl]-2,6-dimethylphenoxy}essigsäure,
{2,6-Dimethyl-4-[7-propoxy-5-(2,2,2-trifluor-1-methylethoxy)oxazolo[5,4-d]pyrimidin-2-yl]phenoxy}essigsäure.

11. Verfahren zur Herstellung einer Verbindung der Formel I nach einem der Ansprüche 1 bis 10, bei dem man eine Verbindung der Formel II mit einer Verbindung der Formel III umsetzt, 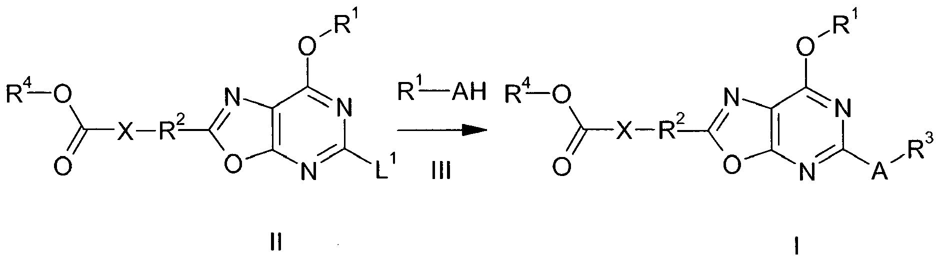 wobei die Gruppen A, X, R¹, R², R³ und R⁴ in den Verbindungen der Formeln II und III wie in den Verbindungen der Formel I definiert sind und außerdem funktionelle Gruppen in geschützter Form oder in Form einer Vorläufergruppe vorliegen können und die Gruppe L¹ für ein Halogenatom oder eine Gruppe der Formel -S(O)-Alk oder -S(O)₂-Alk steht, worin Alk für (C₁-C₄)-Alkyl steht.

12. Pharmazeutische Zusammensetzung, enthaltend mindestens eine Verbindung der Formel I nach einem der Ansprüche 1 bis 10 oder ein physiologisch akzeptables Salz davon oder ein physiologisch akzeptables Solvat einer derartigen Verbindung oder eines derartigen Salzes und einen pharmazeutisch akzeptablen Träger.

13. Verbindung der Formel I nach einem der Ansprüche 1 bis 10 oder physiologisch akzeptables Salz davon oder physiologisch akzeptables Solvat einer derartigen Verbindung oder eines derartigen Salzes zur Verwendung als Arzneimittel.

14. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Behandlung von Wundheilungsstörungen.

15. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Wundheilung.

16. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Wundheilung bei Diabetikern.

17. Verbindung der Formel I nach einem der Ansprüche 1 bis 10 oder eines physiologisch akzeptablen Salzes davon oder eines physiologisch akzeptablen Solvats einer derartigen Verbindung oder eines derartigen Salzes zur Behandlung des Diabetischen Fußsyndroms.

## Claims

1. A compound of the formula I, in any of its stereoisomeric forms, or a mixture of stereoisomeric forms in any ratio, or a physiologically acceptable salt thereof, or a physiologically acceptable solvate of any of them, 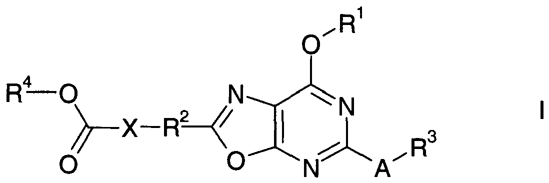 wherein
A is chosen from NH, O and S;
X is chosen from (C₁-C₆)-alkanediyl, (C₂-C₆)-alkenediyl, (C₂-C₆)-alkynediyl, (C₃-C₇)-cycloalkanediyl and (C₁-C₆)-alkanediyl-oxy, which all are optionally substituted by one or more identical or different substituents chosen from fluorine and hydroxy, wherein the oxygen atom of the (C₁-C₆)-alkanediyl-oxy group is bonded to the group R²;
R¹ is chosen from (C₁-C₆)-alkyl, (C₂-C₆)-alkenyl, (C₂-C₆)-alkynyl, (C₃-C₇)-cycloalkyl-CₜH₂ₜ- and Het-CₜH₂ₜ-, wherein t is chosen from 0, 1, 2 and 3;
R² is chosen from phenylene and a divalent residue of an aromatic, 5-membered to 6-membered monocyclic heterocycle which comprises 1, 2 or 3 identical or different ring heteroatoms chosen from N, O and S, wherein one of the ring nitrogen atoms can carry a hydrogen atom or a substituent R²¹, and wherein the phenylene and divalent residue of an aromatic heterocycle are optionally substituted on one or more ring carbon atoms by identical or different substituents R²²;
R³ is chosen from (C₁-C₆)-alkyl, (C₂-C₆)-alkenyl, (C₂-C₆)-alkynyl, (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1, 2, 3 or 4 identical or different ring heteroatoms chosen from N, O and S, wherein one or two of the ring nitrogen atoms can carry a hydrogen atom or a (C₁-C₄)-alkyl substituent and one or two of the ring sulfur atoms can carry one or two oxo groups, and wherein the residue of a ring is optionally substituted on one or more ring carbon atoms by identical or different substituents R³¹, provided that R³ cannot be (C₁-C₆)-alkyl if A is S;
R⁴ is chosen from hydrogen, (C₁-C₄)-alkyl and (C₃-C₇)-cycloalkyl-C_{z}H_{2z}-, wherein z is chosen from 0, 1 and 2;
R²¹ is chosen from (C₁-C₄)-alkyl, (C₃-C₇)-cycloalkyl-C_{w}H_{2w}- and oxy, wherein w is chosen from 0, 1 and 2;
R²² is chosen from halogen, hydroxy, (C₁-C₄)-alkyl-, (C₁-C₄)-alkyloxy, (C₁-C₄)-alkyl-S(O)ₘ-, amino, nitro, cyano, hydroxycarbonyl, (C₁-C₄)-alkyloxycarbonyl, aminocarbonyl and aminosulfonyl;
R³¹ is chosen from halogen, (C₁-C₄)-alkyl, (C₃-C₇)-cycloalkyl, hydroxy, (C₁-C₄)-alkyloxy, oxo, (C₁-C₄)-alkyl-S(O)ₘ-, amino, (C₁-C₄)-alkylamino, di((C₁-C₄)-alkyl)amino, (C₁-C₄)-alkylcarbonylamino, (C₁-C₄)-alkylsulfonylamino, nitro, cyano, (C₁-C₄)-alkylcarbonyl, aminosulfonyl, (C₁-C₄)-alkylaminosulfonyl and di((C₁-C₄)-alkyl)aminosulfonyl;
Het is a residue of a saturated, 4-membered to 7-membered, monocyclic heterocycle which comprises 1 or 2 identical or different ring heteroatoms chosen from N, O and S and which is bonded via a ring carbon atom, wherein the residue of a heterocycle is optionally substituted by one or more identical or different substituents chosen from fluorine and (C₁-C₄)-alkyl;
m is chosen from 0, 1 and 2, wherein all numbers m are independent of each other;
wherein all cycloalkyl and cycloalkanediyl groups, independently of each other and independently of any other substituents, are optionally substituted by one or more identical or different substituents chosen from fluorine and (C₁-C₄)-alkyl;
wherein all alkyl, alkanediyl, CₜH₂ₜ, CᵤH₂ᵤ, CᵥH₂ᵥ, C_{w}H_{2w}, C_{z}H_{2z}, alkenyl, alkenediyl, alkynyl and alkynediyl groups, independently of each other and independently of any other substituents, are optionally substituted by one or more fluorine substituents.

2. A compound of the formula I, in any of its stereoisomeric forms, or a mixture of stereoisomeric forms in any ratio, or a physiologically acceptable salt thereof, or a physiologically acceptable solvate of any of them, as claimed in claim 1, wherein A is chosen from O and S.

3. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 2, wherein X is chosen from (C₁-C₆)-alkanediyl, (C₂-C₆)-alkenediyl and (C₁-C₆)-alkanediyl-oxy.

4. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 3, wherein
R¹ is chosen from (C₁-C₆)-alkyl, (C₃-C₇)-cycloalkyl-CₜH₂ₜ- and Het-CₜH₂ₜ-, wherein t is chosen from 0, 1 and 2.

5. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 4, wherein
R² is chosen from phenylene and pyridinediyl, wherein the phenylene and the pyridinediyl are optionally substituted on one or more ring carbon atoms by identical or different substituents R²².

6. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 5, wherein
R³ is chosen from (C₁-C₆)-alkyl, (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R³¹, provided that R³ cannot be (C₁-C₆)-alkyl if A is S; Het is a residue of a saturated, 4-membered to 6-membered, monocyclic heterocycle which comprises 1 ring heteroatom chosen from N, O and S and which is bonded via a ring carbon atom, wherein the residue of a heterocycle is optionally substituted by one or more identical or different substituents chosen from fluorine and (C₁-C₄)-alkyl.

7. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 6, wherein
A is chosen from O and S;
X is chosen from (C₁-C₆)-alkanediyl, (C₂-C₆)-alkenediyl and (C₁-C₆)-alkanediyl-oxy;
R¹ is chosen from (C₁-C₆)-alkyl, (C₃-C₇)-cycloalkyl-CₜH₂ₜ- and Het-CₜH₂ₜ-, wherein t is chosen from 0, 1 and 2;
R² is chosen from phenylene and pyridinediyl, wherein the phenylene and the pyridinediyl are optionally substituted on one or more ring carbon atoms by identical or different substituents R²²;
R³ is chosen from (C₁-C₆)-alkyl, (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 10-membered, monocyclic or bicyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R³¹, provided that R³ cannot be (C₁-C₆)-alkyl if A is S;
Het is a residue of a saturated, 4-membered to 6-membered, monocyclic heterocycle which comprises 1 ring heteroatom chosen from N, O and S and which is bonded via a ring carbon atom, wherein the residue of a heterocycle is optionally substituted by one or more identical or different substituents chosen from fluorine and (C₁-C₄)-alkyl.

8. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 7, wherein A is O.

9. A compound of the formula I, in any of its stereoisomeric forms, or a mixture of stereoisomeric forms in any ratio, or a physiologically acceptable salt thereof, or a physiologically acceptable solvate of any of them, as claimed in one or more of claims 1 to 8, wherein
A is O;
X is chosen from (C₁-C₆)-alkanediyl and (C₁-C₆)-alkanediyl-oxy;
R¹ is chosen from (C₁-C₆)-alkyl and (C₃-C₆)-cycloalkyl-CₜH₂ₜ-, wherein t is chosen from 0 and 1;
R² is phenylene which is optionally substituted on one or more ring carbon atoms by identical or different substituents R²²;
R³ is chosen from (C₁-C₆)-alkyl, (C₃-C₇)-cycloalkyl-CᵤH₂ᵤ- and Het-CᵥH₂ᵥ-, wherein u and v are chosen from 1 and 2, or R³ is a residue of a saturated or unsaturated, 3-membered to 7-membered, monocyclic or bicyclic ring which comprises 0, 1 or 2 identical or different ring heteroatoms chosen from N, O and S, wherein one or two of the ring nitrogen atoms can carry a hydrogen atom or a (C₁-C₄)-alkyl substituent and one of the ring sulfur atoms can carry one or two oxo groups, and wherein the residue of a ring is optionally substituted on one or more ring carbon atoms by identical or different substituents R³¹, provided that R³ cannot be (C₁-C₆)-alkyl if A is S; R⁴ is chosen from hydrogen and (C₁-C₄)-alkyl;
R²² is chosen from halogen, hydroxy, (C₁-C₄)-alkyl- and (C₁-C₄)-alkyloxy; R³¹ is chosen from halogen, (C₁-C₄)-alkyl, (C₃-C₇)-cycloalkyl, hydroxy and (C₁-C₄)-alkyloxy;
Het is a residue of a saturated, 4-membered to 6-membered, monocyclic heterocycle which comprises 1 ring heteroatom chosen from O and S and which is bonded via a ring carbon atom, wherein the residue of a heterocycle is optionally substituted by one or more identical or different substituents chosen from fluorine and (C₁-C₄)-alkyl;
wherein all cycloalkyl groups, independently of each other and independently of any other substituents, are optionally substituted by one or more identical or different substituents chosen from fluorine and (C₁-C₄)-alkyl;
wherein all alkyl, alkanediyl, CₜH₂ₜ, CᵤH₂ᵤ and CᵥH₂ᵥ groups, independently of each other and independently of any other substituents, are optionally substituted by one or more fluorine substituents.

10. A compound of the formula I, or a physiologically acceptable salt thereof, or a physiologically acceptable solvate of any of them, as claimed in one or more of claims 1 to 9, chosen from
{4-[5-(2,5-difluoro-phenoxy)-7-propoxy-oxazolo[5,4-d]pyrimidin-2-yl]-2,6-dimethyl-phenoxy}-acetic acid,
[4-(5-cyclopentyloxy-7-propoxy-oxazolo[5,4-d]pyrimidin-2-yl)-2,6-dimethylphenoxy]-acetic acid,
{4-[5-(trans-2-fluoro-cyclohexyloxy)-7-propoxy-oxazolo[5,4-d]pyrimidin-2-yl]-2,6-dimethyl-phenoxy}-acetic acid,
{4-[5-(2-fluoro-phenoxy)-7-propoxy-oxazolo[5,4-d]pyrimidin-2-yl]-2,6-dimethyl-phenoxy}-acetic acid,
{4-[5-(5-fluoro-2-methyl-phenoxy)-7-propoxy-oxazolo[5,4-d]pyrimidin-2-yl]-2,6-dimethyl-phenoxy}-acetic acid,
{4-[5-(3-fluoro-4-methyl-phenoxy)-7-propoxy-oxazolo[5,4-d]pyrimidin-2-yl]-2,6-dimethyl-phenoxy}-acetic acid,
{2,6-dimethyl-4-[7-propoxy-5-(pyridin-3-yloxy)-oxazolo[5,4-d]pyrimidin-2-yl]-phenoxy}-acetic acid,
{4-[5-(2,4-difluoro-phenoxy)-7-propoxy-oxazolo[5,4-d]pyrimidin-2-yl]-2,6-dimethyl-phenoxy}-acetic acid,
[2,6-dimethyl-4-(5-phenoxy-7-propoxy-oxazolo[5,4-d]pyrimidin-2-yl)-phenoxy]-acetic acid,
{4-[5-(3-chloro-phenoxy)-7-propoxy-oxazolo[5,4-d]pyrimidin-2-yl]-2,6-dimethyl-phenoxy}-acetic acid,
[4-(5-cyclohexylmethoxy-7-propoxy-oxazolo[5,4-d]pyrimidin-2-yl)-2,6-dimethyl-phenoxy]-acetic acid,
[4-(5-isobutoxy-7-propoxy-oxazolo[5,4-d]pyrimidin-2-yl)-2,6-dimethylphenoxy]-acetic acid,
[4-(5-cyclobutylmethoxy-7-propoxy-oxazolo[5,4-d]pyrimidin-2-yl)-2,6-dimethyl-phenoxy]-acetic acid,
[4-(5-cyclobutoxy-7-propoxy-oxazolo[5,4-d]pyrimidin-2-yl)-2,6-dimethyl-phenoxy]-acetic acid,
[4-(5,7-dipropoxy-oxazolo[5,4-d]pyrimidin-2-yl)-2,6-dimethyl-phenoxy]-acetic acid,
{2,6-dimethyl-4-[7-propoxy-5-(3,3,3-trifluoro-propoxy)-oxazolo[5,4-d]pyrimidin-2-yl]-phenoxy}-acetic acid,
[4-(5-ethoxy-7-propoxy-oxazolo[5,4-d]pyrimidin-2-yl)-2,6-dimethyl-phenoxy]-acetic acid,
[4-(5-cyclopentylmethoxy-7-propoxy-oxazolo[5,4-d]pyrimidin-2-yl)-2,6-dimethyl-phenoxy]-acetic acid,
{2,6-dimethyl-4-[7-propoxy-5-(tetrahydrofuran-2-ylmethoxy)-oxazolo[5,4-d]pyrimidin-2-yl]-phenoxy}-acetic acid,
[4-(5-sec-butoxy-7-propoxy-oxazolo[5,4-d]pyrimidin-2-yl)-2,6-dimethylphenoxy]-acetic acid,
{2,6-dimethyl-4-[7-propoxy-5-(3,3,3-trifluoro-1-methyl-propoxy)-oxazolo[5,4-d]pyrimidin-2-yl]-phenoxy}-acetic acid,
{2,6-dimethyl-4-[5-(3-methyl-butoxy)-7-propoxy-oxazolo[5,4-d]pyrimidin-2-yl]-phenoxy}-acetic acid,
{4-[5-(2-cyclopropyl-ethoxy)-7-propoxy-oxazolo[5,4-d]pyrimidin-2-yl]-2,6-dimethyl-phenoxy}-acetic acid, and
{2,6-dimethyl-4-[7-propoxy-5-(2,2,2-trifluoro-1-methyl-ethoxy)-oxazolo[5,4-d]pyrimidin-2-yl]-phenoxy}-acetic acid.

11. A process for the preparation of a compound of the formula I as claimed in any of claims 1 to 10, comprising reacting a compound of the formula II with a compound of the formula III, 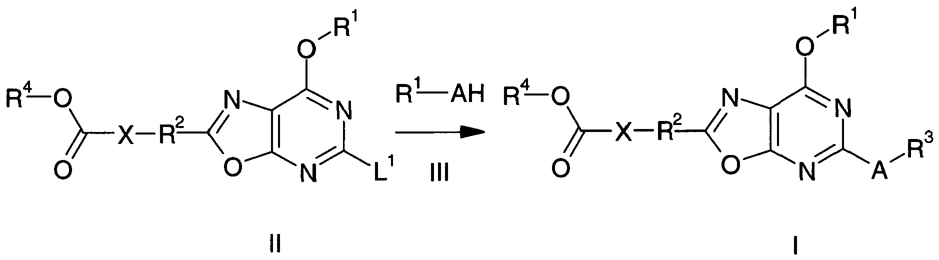 wherein the groups A, X, R¹, R², R³ and R⁴ in the compounds of the formulae II and III are defined as in the compounds of the formula I and additionally functional groups can be present in protected form or in the form of a precursor group, and the group L¹ is a halogen atom or a group of the formula -S(O)-Alk or -S(O)₂-Alk wherein Alk is (C₁-C₄)-alkyl.

12. A pharmaceutical composition, comprising at least one compound of the formula I as claimed in any of claims 1 to 10 or a physiologically acceptable salt thereof, or a physiologically acceptable solvate of any of them, and a pharmaceutically acceptable carrier.

13. A compound of the formula I as claimed in any of claims 1 to 10 or a physiologically acceptable salt thereof, or a physiologically acceptable solvate of any of them, for use as a pharmaceutical.

14. A compound of the formula I as claimed in any of claims 1 to 10 or a physiologically acceptable salt thereof, or a physiologically acceptable solvate of any of them, for the treatment of wound healing disorders.

15. A compound of the formula I as claimed in any of claims 1 to 10 or a physiologically acceptable salt thereof, or a physiologically acceptable solvate of any of them, for wound healing.

16. A compound of the formula I as claimed in any of claims 1 to 10 or a physiologically acceptable salt thereof, or a physiologically acceptable solvate of any of them for wound healing in diabetics.

17. A compound of the formula I as claimed in any of claims 1 to 10 or a physiologically acceptable salt thereof, or a physiologically acceptable solvate of any of them, for the treatment of diabetic foot syndrome.

## Revendications

1. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel, 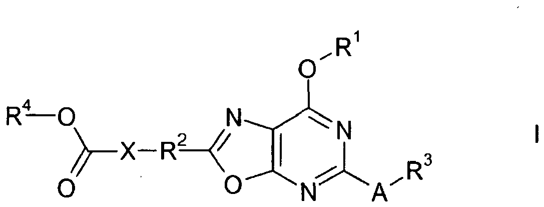 dans lequel
A est choisi parmi NH, O et S ;
X est choisi parmi alcanediyle en (C₁-C₆), alcènediyle en (C₂-C₆), alcynediyle en (C₂-C₆), cycloalcanediyle en (C₃-C₇) et alcanediyloxy en (C₁-C₆), qui sont tous éventuellement substitués par un ou plusieurs substituants identiques ou différents choisis parmi fluor et hydroxy, l'atome d'oxygène du groupe alcanediyloxy en (C₁-C₆) étant relié au groupe R² ;
R¹ est choisi parmi alkyle en (C₁-C₆), alcényle en (C₂-C₆), alcynyle en (C₂-C₆), cycloalkyle en (C₃-C₇)-CₜH₂ₜ- et Het-CₜH₂ₜ-, t étant choisi parmi 0, 1, 2 et 3 ;
R² est choisi parmi phénylène et un radical bivalent d'un hétérocycle monocyclique aromatique de 5 éléments à 6 éléments, qui contient 1, 2 ou 3 hétéroatomes de cycle identiques ou différents choisis parmi N, O et S, un des atomes d'azote de cycle pouvant porter un atome d'hydrogène ou un substituant R²¹, et le phénylène et le radical bivalent d'un hétérocycle aromatique étant éventuellement substitués sur un ou plusieurs atomes de carbone de cycle par des substituants R²² identiques ou différents ;
R³ est choisi parmi alkyle en (C₁-C₆), alcényle en (C₂-C₆), alcynyle en (C₂-C₆), cycloalkyle en (C₃-C₇)-CᵤH₂ᵤ- et Het-CᵥH₂ᵥ-, u et v étant choisis parmi 1 et 2, ou R³ représente un radical d'un cycle monocyclique ou bicyclique, saturé ou insaturé, de 3 éléments à 10 éléments, qui contient 0, 1, 2, 3 ou 4 hétéroatomes de cycle identiques ou différents choisis parmi N, O et S, un ou deux des atomes d'azote de cycle pouvant porter un atome d'hydrogène ou un substituant alkyle en (C₁-C₄), et un ou deux des atomes de soufre de cycle pouvant porter un ou deux groupes oxo, et le radical d'un cycle étant éventuellement substitué sur un ou plusieurs atomes de carbone de cycle par des substituants R³¹ identiques ou différents à condition que R³ ne puisse pas représenter alkyle en (C₁-C₆) lorsque A représente S ;
R⁴ est choisi parmi hydrogène, alkyle en (C₁-C₄) et cycloalkyle en (C₃-C₇)-C_{z}H_{2z}-, z étant choisi parmi 0, 1 et 2 ;
R²¹ est choisi parmi alkyle en (C₁-C₄), cycloalkyle en (C₃-C₇)-C_{w}H_{2w}- et oxy, w étant choisi parmi 0, 1 et 2 ;
R²² est choisi parmi halogène, hydroxy, alkyle en (C₁-C₄), alkyloxy en (C₁-C₄), alkyl-S(O)ₘ en (C₁-C₄), amino, nitro, cyano, hydroxycarbonyle, alkyloxycarbonyle en (C₁-C₄), aminocarbonyle et aminosulfonyle ;
R³¹ est choisi parmi halogène, alkyle en (C₁-C₄), cycloalkyle en (C₃-C₇), hydroxy, alkyloxy en (C₁-C₄), oxo, alkyl-S(O)ₘ en (C₁-C₄), amino, alkylamino en (C₁-C₄), di(alkyle en (C₁-C₄))-amino, alkylcarbonylamino en (C₁-C₄), alkylsulfonylamino en (C₁-C₄), nitro, cyano, alkylcarbonyle en (C₁-C₄), aminosulfonyle, alkylaminosulfonyle en (C₁-C₄) et di(alkyle en (C₁-C₄))-aminosulfonyle ;
Het représente un radical d'un hétérocycle monocyclique saturé de 4 éléments à 7 éléments, qui contient 1 ou 2 hétéroatomes de cycle identiques ou différents choisis parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄) ;
m est choisi parmi 0, 1 et 2, tous les nombres m étant indépendants les uns des autres ;
tous les groupes cycloalkyle et cycloalcanediyle étant éventuellement substitués indépendamment les uns des autres et indépendamment des autres substituants par un ou plusieurs substituants identiques ou différents choisis parmi fluor et alkyle en (C₁-C₄) ;
tous les groupes alkyle, alcanediyle, CₜH₂ₜ, CᵤH₂ᵤ, CᵥH₂ᵥ, C_{w}H_{2w}, C_{z}H_{2z}, alcényle, alcènediyle, alcynyle et alcynediyle étant éventuellement substitués indépendamment les uns des autres et indépendamment des autres substituants par un ou plusieurs substituants fluor.

2. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon la revendication 1, dans lequel A est choisi parmi O et S.

3. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 et 2, dans lequel X est choisi parmi alcanediyle en (C₁-C₆), alcènediyle en (C₂-C₆) et alcanediyloxy en (C₁-C₆).

4. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 3, dans lequel
R¹ est choisi parmi alkyle en (C₁-C₆), cycloalkyle en (C₃-C₇)-CₜH₂ₜ- et Het-CₜH₂ₜ-, t étant choisi parmi 0, 1 et 2.

5. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 4, dans lequel
R² est choisi parmi phénylène et pyridinediyle, dans lequel le phénylène et le pyridinediyle sont éventuellement substitués sur un ou plusieurs atomes de carbone de cycle par des substituants R²² identiques ou différents.

6. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 5, dans lequel
R³ est choisi parmi alkyle en (C₁-C₆), cycloalkyle en (C₃-C₇)-CᵤH₂ᵤ- et Het-CᵥH₂ᵥ-, u et v étant choisis parmi 1 et 2, ou R³ représente un radical d'un cycle monocyclique ou bicyclique, saturé ou insaturé, de 3 éléments à 10 éléments, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄), et un des atomes de soufre de cycle pouvant porter un ou deux groupes oxo, et le radical d'un cycle étant éventuellement substitué sur un ou plusieurs atomes de carbone de cycle par des substituants R³¹ identiques ou différents à condition que R³ ne puisse pas représenter alkyle en (C₁-C₆) lorsque A représente S ;
Het représente un radical d'un hétérocycle monocyclique saturé de 4 éléments à 6 éléments, qui contient 1 hétéroatome de cycle choisi parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄).

7. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 6, dans lequel
A est choisi parmi O et S ;
X est choisi parmi alcanediyle en (C₁-C₆), alcènediyle en (C₂-C₆) et alcanediyloxy en (C₁-C₆) ;
R¹ est choisi parmi alkyle en (C₁-C₆), cycloalkyle en (C₃-C₇)-CₜH₂ₜ- et Het-CₜH₂ₜ-, t étant choisi parmi 0, 1 et 2 ;
R² est choisi parmi phénylène et pyridinediyle, le phénylène et le pyridinediyle étant éventuellement substitués sur un ou plusieurs atomes de carbone de cycle par des substituants R²² identiques ou différents ;
R³ est choisi parmi alkyle en (C₁-C₆), cycloalkyle en (C₃-C₇)-CᵤH₂ᵤ- et Het-CᵥH₂ᵥ-, u et v étant choisis parmi 1 et 2, ou R³ représente un radical d'un cycle monocyclique ou bicyclique, saturé ou insaturé, de 3 éléments à 10 éléments, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄), et un des atomes de soufre de cycle pouvant porter un ou deux groupes oxo, et le radical d'un cycle étant éventuellement substitué sur un ou plusieurs atomes de carbone de cycle par des substituants R³¹ identiques ou différents à condition que R³ ne puisse pas représenter alkyle en (C₁-C₆) lorsque A représente S ;
Het représente un radical d'un hétérocycle monocyclique saturé de 4 éléments à 6 éléments, qui contient 1 hétéroatome de cycle choisi parmi N, O et S, et qui est relié par un atome de carbone de cycle, le radical d'un hétérocycle étant éventuellement substitué par un ou plusieurs substituants identiques ou différents choisis parmi fluor et alkyle en (C₁-C₄).

8. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 7, dans lequel A représente O.

9. Composé de formule I sous l'une quelconque de ses formes stéréoisomères ou en un mélange de formes stéréoisomères en un rapport quelconque ou sel physiologiquement acceptable de celui-ci ou solvate physiologiquement acceptable d'un tel composé ou d'un tel sel selon une ou plusieurs des revendications 1 à 8, dans lequel
A représente O ;
X est choisi parmi alcanediyle en (C₁-C₆) et alcanediyloxy en (C₁-C₆) ; R¹ est choisi parmi alkyle en (C₁-C₆) et cycloalkyle en (C₃-C₆)-CₜH₂ₜ-, t étant choisi parmi 0 et 1 ;
R² représente phénylène, qui est éventuellement substitué sur un ou plusieurs atomes de carbone de cycle par des substituants R²² identiques ou différents ;
R³ est choisi parmi alkyle en (C₁-C₆), cycloalkyle en (C₃-C₇)-CᵤH₂ᵤ- et Het-CᵥH₂ᵥ-, u et v étant choisis parmi 1 et 2, ou R³ représente un radical d'un cycle monocyclique ou bicyclique, saturé ou insaturé, de 3 éléments à 7 éléments, qui contient 0, 1 ou 2 hétéroatomes de cycle identiques ou différents choisis parmi N, O et S, un ou deux des atomes d'azote de cycle pouvant porter un atome d'hydrogène ou un substituant alkyle en (C₁-C₄), et un des atomes de soufre de cycle pouvant porter un ou deux groupes oxo, et le radical d'un cycle étant éventuellement substitué sur un ou plusieurs atomes de carbone de cycle par des substituants R³¹ identiques ou différents à condition que R³ ne puisse pas représenter alkyle en (C₁-C₆) lorsque A représente S ;
R⁴ est choisi parmi hydrogène et alkyle en (C₁-C₄) ;
R²² est choisi parmi halogène, hydroxy, alkyle en (C₁-C₄) et alkyloxy en (C₁-C₄) ;
R³¹ est choisi parmi halogène, alkyle en (C₁-C₄), cycloalkyle en (C₃-C₇), hydroxy et alkyloxy en (C₁-C₄) ;
Het représente un radical d'un hétérocycle monocyclique saturé de 4 éléments à 6 éléments, qui contient 1 hétéroatome de cycle choisi parmi O et S, et qui est relié par un atome de carbone de cycle, le radical d'un hétérocycle étant éventuellement substitué par un ou plusieurs substituants identiques ou différents choisis parmi fluor et alkyle en (C₁-C₄) ;
tous les groupes cycloalkyle étant éventuellement substitués indépendamment les uns des autres et indépendamment des autres substituants par un ou plusieurs substituants identiques ou différents choisis parmi fluor et alkyle en (C₁-C₄) ;
tous les groupes alkyle, alcanediyle, CₜH₂ₜ, CᵤH₂ᵤ et CᵥH₂ᵥ étant éventuellement substitués indépendamment les uns des autres et indépendamment des autres substituants par un ou plusieurs substituants fluor.

10. Composé de formule I ou sel physiologiquement acceptable de celui-ci ou solvate physiologiquement acceptable d'un tel composé ou d'un tel sel, selon une ou plusieurs des revendications 1 à 9, choisi parmi :
l'acide {4-[5-(2,5-difluorophénoxy)-7-propoxyoxazolo[5,4-d]pyrimidin-2-yl]-2,6-diméthylphénoxy}acétique,
l'acide [4-(5-cyclopentyloxy-7-propoxyoxazolo[5,4-d]pyrimidin-2-yl)-2,6-diméthylphénoxy]-acétique,
l'acide {4-[5-(trans-2-fluorocyclohexyloxy)-7-propoxyoxazolo[5,4-d]pyrimidin-2-yl]-2,6-diméthylphénoxy}acétique,
l'acide {4-[5-(2-fluorophénoxy)-7-propoxyoxazolo[5,4-d]pyrimidin-2-yl]-2,6-diméthylphénoxy}-acétique,
l'acide {4-[5-(5-fluoro-2-méthylphénoxy)-7-propoxyoxazolo[5,4-d]pyrimidin-2-yl]-2,6-diméthyl-phénoxy]acétique,
l'acide {4-[5-(3-fluoro-4-méthylphénoxy)-7-propoxyoxazolo[5,4-d]pyrimidin-2-yl]-2,6-diméthyl-phénoxy}acétique,
l'acide {2,6-diméthyl-4-[7-propoxy-5-(pyridin-3-yloxy)-oxazolo[5,4-d]pyrimidin-2-yl]phénoxy]-acétique,
l'acide {4-[5-(2,4-difluorophénoxy)-7-propoxyoxazolo[5,4-d]pyrimidin-2-yl]-2,6-diméthyl-phénoxy}acétique,
l'acide [2,6-diméthyl-4-(5-phénoxy-7-propoxyoxazolo[5,4-d]pyrimidin-2-yl)-phénoxy]-acétique,
l'acide {4-[5-(3-chlorophénoxy)-7-propoxyoxazolo[5,4-d]pyrimidin-2-yl]-2,6-diméthylphénoxy}-acétique,
l'acide [4-(5-cyclohexylméthoxy-7-propoxyoxazolo[5,4-d]pyrimidin-2-yl)-2,6-diméthyl-phénoxy]acétique,
l'acide [4-(5-isobutoxy-7-propoxyoxazolo[5,4-d]pyrimidin-2-yl)-2,6-diméthylphénoxy]-acétique,
l'acide [4-(5-cyclobutylméthoxy-7-propoxyoxazolo[5,4-d]pyrimidin-2-yl)-2,6-diméthyl-phénoxy]acétique,
l'acide [4-(5-cyclobutoxy-7-propoxyoxazolo[5,4-d]pyrimidin-2-yl)-2,6-diméthylphénoxy]-acétique,
l'acide [4-(5,7-dipropoxyoxazolo[5,4-d]pyrimidin-2-yl)-2,6-diméthylphénoxy]acétique,
l'acide {2,6-diméthyl-4-[7-propoxy-5-(3,3,3-trifluoropropoxy)oxazolo[5,4-d]pyrimidin-2-yl]-phénoxy}acétique,
l'acide [4-(5-éthoxy-7-propoxyoxazolo[5,4-d]pyrimidin-2-yl)-2,6-diméthylphénoxy]acétique,
l'acide [4-(5-cyclopentylméthoxy-7-propoxyoxazolo[5,4-d]pyrimidin-2-yl)-2,6-diméthyl-phénoxy]acétique,
l'acide {2,6-diméthyl-4-[7-propoxy-5-(tétrahydrofuran-2-ylméthoxy)oxazolo[5,4-d]pyrimidin-2-yl]phénoxy}acétique,
l'acide [4-(5-sec.-butoxy-7-propoxyoxazolo[5,4-d]pyrimidin-2-yl)-2,6-diméthylphénoxy]-acétique,
l'acide {2,6-diméthyl-4-[7-propoxy-5-(3,3,3-trifluoro-1-méthylpropoxy)oxazolo[5,4-d]pyrimidin-2-yl]phénoxy}acétique,
l'acide {2,6-diméthyl-4-[5-(3-méthylbutoxy)-7-propoxyoxazolo[5,4-d]pyrimidin-2-yl]phénoxy}-acétique,
l'acide {4-[5-(2-cyclopropyléthoxy)-7-propoxyoxazolo[5,4-d]pyrimidin-2-yl]-2,6-diméthyl-phénoxy}acétique,
l'acide {2,6-diméthyl-4-[7-propoxy-5-(2,2,2-trifluoro-1-méthyléthoxy)oxazolo[5,4-d]pyrimidin-2-yl]phénoxy}acétique.

11. Procédé de fabrication d'un composé de formule I selon l'une quelconque des revendications 1 à 10, selon lequel un composé de formule II est mis en réaction avec un composé de formule III 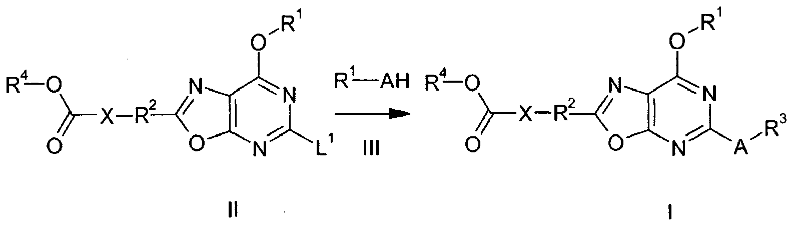 les groupes A, X, R¹, R², R³ et R⁴ dans les composés de formules II et III étant tels que définis dans les composés de formule I et des groupes fonctionnels pouvant également être présents sous forme protégée ou sous la forme d'un groupe précurseur, et le groupe L¹ représentant un atome d'halogène ou un groupe de formule -S(O)-Alk ou -S(O)₂-Alk, Alk représentant alkyle en (C₁-C₄).

12. Composition pharmaceutique, contenant au moins un composé de formule I selon l'une quelconque des revendications 1 à 10 ou un sel physiologiquement acceptable de celui-ci ou un solvate physiologiquement acceptable d'un tel composé ou d'un tel sel, et un véhicule pharmaceutiquement acceptable.

13. Composé de formule I selon l'une quelconque des revendications 1 à 10 ou sel physiologiquement acceptable de celui-ci ou solvate physiologiquement acceptable d'un tel composé ou d'un tel sel destiné à être utilisé en tant que médicament.

14. Composé de formule I selon l'une quelconque des revendications 1 à 10 ou sel physiologiquement acceptable de celui-ci ou solvate physiologiquement acceptable d'un tel composé ou d'un tel sel pour le traitement de troubles de la cicatrisation.

15. Composé de formule I selon l'une quelconque des revendications 1 à 10 ou sel physiologiquement acceptable de celui-ci ou solvate physiologiquement acceptable d'un tel composé ou d'un tel sel pour la cicatrisation.

16. Composé de formule I selon l'une quelconque des revendications 1 à 10 ou sel physiologiquement acceptable de celui-ci ou solvate physiologiquement acceptable d'un tel composé ou d'un tel sel pour la cicatrisation chez les diabétiques.

17. Composé de formule I selon l'une quelconque des revendications 1 à 10 ou sel physiologiquement acceptable de celui-ci ou solvate physiologiquement acceptable d'un tel composé ou d'un tel sel pour le traitement du syndrome du pied diabétique.
